# EUROPEAN PATENT APPLICATION

(11) **EP 4 696 692 A1**
(43) Date of publication of application: **18.02.2026**
(21) Application number: 24825892.3
(22) Date of filing: 18.06.2024
(51) Int. Cl.: C07D 498/08, A61K 31/5386, A61P 11/00, A61P 13/12, A61P 25/00, A61P 43/00, C07D 519/00

(54) **CRYSTAL OF NITROGEN-CONTAINING HETEROCYCLIC COMPOUND HAVING NRF2 ACTIVATION EFFECT**

(30) Priority: 19.06.2023 JP 2023100075
(71) Applicant: CHUGAI SEIYAKU KABUSHIKI KAISHA, Tokyo 115-8543 (JP)
(72) Inventor: IWATA Tomoya, Yokohama City, Kanagawa 244-8602 (JP); TAKATA Noriyuki, Tokyo 115-8543 (JP); KONO Masato, Tokyo 115-8543 (JP)
(74) Representative: Vossius & Partner Patentanwälte Rechtsanwälte mbB
(86) International application number: PCT/JP2024/022040
(87) International publication number: WO 2024/262491

(57) **Abstract**

Provided is a low-molecular compound or a salt thereof, or a solvate thereof which has a Nrf2-activating effect. The present invention provides a crystal of a compound represented by any of the following formulas (1) to (5) or a salt thereof, or a solvate thereof:

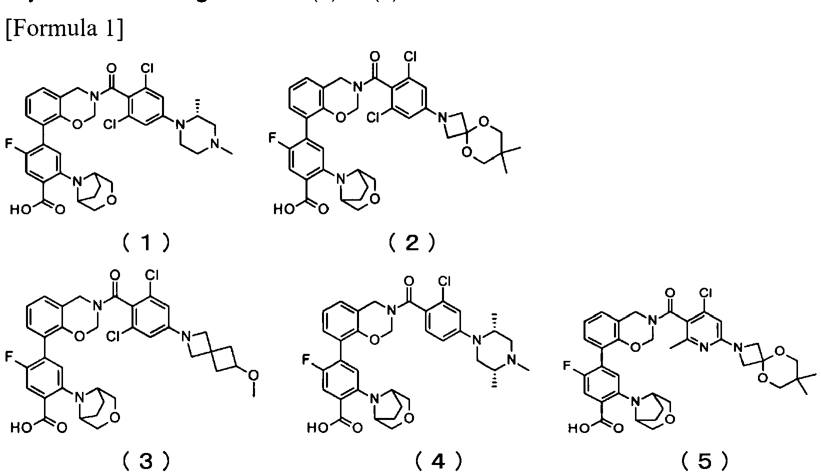

## Description

### Technical Field

The present invention relates to crystals of a nitrogen-containing heterocyclic compound or a salt thereof, or a solvate thereof which has a Nrf2-activating effect and use thereof. The present invention also relates to a medicament and a pharmaceutical composition containing the same as an active ingredient.

### Background Art

Nrf2 is a control factor for gene groups involved in various body's defenses and is a transcriptional factor that is activated in response to oxidative stress. In a normal state, Nrf2 binds to Keap1 and undergoes ubiquitination and degradation through the proteasome pathway. Under stress conditions, Nrf2 circumvents degradation by dissociation from Keap1 and translocates into the nucleus. Then, Nrf2 forms a heterodimer with a small Maf group factor, and this heterodimer binds to antioxidant response elements (ARE) to induce the transcription of downstream molecules.

The activation of Nrf2 exhibits an antioxidative effect as well as a wide range of pharmacological effects such as an anti-inflammatory effect, an anti fibrotic effect, and an anti-apoptotic effect, and is considered to defensively act on various diseases. Specific examples of such a disease include neurodegenerative disease including Alzheimer's disease, Parkinson's disease, Huntington's disease, Friedreich's ataxia, and amyotrophic lateral sclerosis, lung disease including idiopathic pulmonary fibrosis, acute respiratory distress syndrome, chronic obstructive pulmonary disease, pulmonary arterial hypertension, and asthma, kidney disease including chronic kidney disease and acute kidney injury, ophthalmic disease including uveitis, glaucoma, and age-related macular degeneration, liver disease including nonalcoholic steatohepatitis, and immunological or inflammatory disease including multiple sclerosis, rheumatoid arthritis, and ulcerative colitis (Non Patent Literatures 3 to 7). In actuality, bardoxolone methyl (CDDO-Me; Patent Literature 1) which targets various chronic kidney diseases is under clinical trial as a compound having a Nrf2 activation-inducing effect. RTA-408 (omaveloxolone; Patent Literature 2) which targets Friedreich's ataxia or the like has been approved by FDA. Dimethyl fumarate (Patent Literature 3) is used as a therapeutic drug for multiple sclerosis. However, these compounds are compounds that covalently bind to Keap1. A fear of the risk of heart failure ascribable to the interaction of the covalent binding site of bardoxolone methyl with a non-target protein has been reported (Non Patent Literature 8).

For example, compounds described in Patent Literature 4 have been reported so far as Nrf2 activators. However, these compounds have not yet been clinically developed. Thus, there is a demand for a non-covalently binding type of Nrf2 activator which can be expected to reduce interaction with a non-target protein (Non Patent Literatures 1 to 3).

### Citation List

### Patent Literature

Patent Literature 1: International Publication No. WO 1999/065478
Patent Literature 2: International Publication No. WO 2014/176415
Patent Literature 3: International Publication No. WO 2016/090154
Patent Literature 4: International Publication No. WO 2020/165776

### Non Patent Literature

Non Patent Literature 1: Med Chem Commun, 2017, 8, 286-294.
Non Patent Literature 2: Medicinal Chemistry Research, 2020, 29, 846-867.
Non Patent Literature 3: Nature Reviews Drug Discovery, 2019, 18, 295-317.
Non Patent Literature 4: Oxydative Medicine and Cellular Longevity, 2019, Article ID 9372182, 1-20.
Non Patent Literature 5: Neurodegenr Dis Manag, 2017, 7, 97-100.
Non Patent Literature 6: Oxydative Medicine and Cellular Longevity, 2019, Article ID 7090534, 1-17.
Non Patent Literature 7: Oxydative Medicine and Cellular Longevity, 2020, Article ID 9410952, 1-22.
Non Patent Literature 8: Am J Nephrol, 2014, 39, 499-508

### Summary of Invention

### Technical Problem

An object of the present invention is to provide crystals of a low-molecular compound or a salt thereof, or a solvate thereof which has the ability to activate Nrf2 and reduces interaction with a non-target protein attributed to covalent binding. Another object of the present invention is to provide a prophylactic agent or a therapeutic agent for various diseases, for example, neurodegenerative disease, lung disease, and kidney disease, containing the same as an active ingredient.

### Solution to Problem

The present inventors have conducted diligent studies to attain the objects and consequently completed the present invention by finding that a compound represented by any of the formulas (1) to (5) which largely differ in chemical structure from Nrf2 activators known in the art, or a salt thereof, or a solvate thereof has an excellent Nrf2-activating effect, and also finding crystals of a compound represented by any of the formulas (1) to (5) or a salt thereof, or a solvate thereof.

Specifically, one aspect of the present invention provides the following invention.
[A1] A crystal of a compound represented by the following formula (1) or a salt thereof, or a solvate thereof: 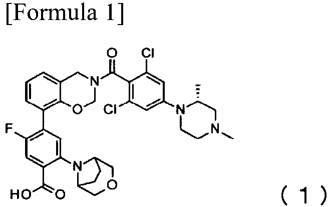
[A2] The crystal according to [A1], wherein the crystal is a solvate crystal of the compound represented by the formula (1).
[A3] The crystal according to [A1] or [A2], wherein the solvate crystal is a hydrate crystal.
[A4] The crystal according to [A3], wherein the hydrate crystal is a 1A-type crystal comprising at least one peak selected from the group consisting of 6.0°, 9.3°, 9.7°, 10.4°, 11.9°, 12.9°, 15.3°, 15.9°, 16.4°, and 16.8° (±0.2°) as diffraction angles (2θ values) in powder X-ray diffraction.
[A4-1] The crystal according to [A3], wherein the hydrate crystal is a 1A-type crystal comprising at least three peaks selected from the group consisting of 6.0°, 9.3°, 9.7°, 10.4°, 11.9°, 12.9°, 15.3°, 15.9°, 16.4°, and 16.8° (±0.2°) as diffraction angles (2θ values) in powder X-ray diffraction.
[A4-2] The crystal according to [A3], wherein the hydrate crystal is a 1A-type crystal comprising at least five peaks selected from the group consisting of 6.0°, 9.3°, 9.7°, 10.4°, 11.9°, 12.9°, 15.3°, 15.9°, 16.4°, and 16.8° (±0.2°) as diffraction angles (2θ values) in powder X-ray diffraction.
[A4-3] The crystal according to [A3], wherein the hydrate crystal is a 1A-type crystal comprising at least seven peaks selected from the group consisting of 6.0°, 9.3°, 9.7°, 10.4°, 11.9°, 12.9°, 15.3°, 15.9°, 16.4°, and 16.8° (±0.2°) as diffraction angles (2θ values) in powder X-ray diffraction.
[A4-4] The crystal according to [A3], wherein the hydrate crystal is a 1A-type crystal comprising peaks of 6.0°, 9.3°, 9.7°, 10.4°, 11.9°, 12.9°, 15.3°, 15.9°, 16.4°, and 16.8° (±0.2°) as diffraction angles (2θ values) in powder X-ray diffraction.
[A5] The crystal according to [A3], wherein the hydrate crystal is a 1G-type crystal comprising at least one peak selected from the group consisting of 8.8°, 9.2°, 10.4°, 12.7°, 13.4°, 15.8°, 16.9°, 17.3°, 18.3°, and 18.9° (±0.2°) as diffraction angles (2θ values) in powder X-ray diffraction.
[A5-1] The crystal according to [A3], wherein the hydrate crystal is a 1G-type crystal comprising at least three peaks selected from the group consisting of 8.8°, 9.2°, 10.4°, 12.7°, 13.4°, 15.8°, 16.9°, 17.3°, 18.3°, and 18.9° (±0.2°) as diffraction angles (2θ values) in powder X-ray diffraction.
[A5-2] The crystal according to [A3], wherein the hydrate crystal is a 1G-type crystal comprising at least five peaks selected from the group consisting of 8.8°, 9.2°, 10.4°, 12.7°, 13.4°, 15.8°, 16.9°, 17.3°, 18.3°, and 18.9° (±0.2°) as diffraction angles (2θ values) in powder X-ray diffraction.
[A5-3] The crystal according to [A3], wherein the hydrate crystal is a 1G-type crystal comprising at least seven peaks selected from the group consisting of 8.8°, 9.2°, 10.4°, 12.7°, 13.4°, 15.8°, 16.9°, 17.3°, 18.3°, and 18.9° (±0.2°) as diffraction angles (2θ values) in powder X-ray diffraction.
[A5-4] The crystal according to [A3], wherein the hydrate crystal is a 1G-type crystal comprising peaks of 8.8°, 9.2°, 10.4°, 12.7°, 13.4°, 15.8°, 16.9°, 17.3°, 18.3°, and 18.9° (±0.2°) as diffraction angles (2θ values) in powder X-ray diffraction.
[A6] The crystal according to [A3], wherein the hydrate crystal is a 1G-type crystal comprising at least one peak selected from the group consisting of 8.9°, 9.3°, 10.5°, 12.8°, 13.5°, 15.9°, 17.0°, 17.4°, 18.4°, and 19.0° (±0.2°) as diffraction angles (2θ values) in powder X-ray diffraction.
[A6-1] The crystal according to [A3], wherein the hydrate crystal is a 1G-type crystal comprising at least three peaks selected from the group consisting of 8.9°, 9.3°, 10.5°, 12.8°, 13.5°, 15.9°, 17.0°, 17.4°, 18.4°, and 19.0° (±0.2°) as diffraction angles (2θ values) in powder X-ray diffraction.
[A6-2] The crystal according to [A3], wherein the hydrate crystal is a 1G-type crystal comprising at least five peaks selected from the group consisting of 8.9°, 9.3°, 10.5°, 12.8°, 13.5°, 15.9°, 17.0°, 17.4°, 18.4°, and 19.0° (±0.2°) as diffraction angles (2θ values) in powder X-ray diffraction.
[A6-3] The crystal according to [A3], wherein the hydrate crystal is a 1G-type crystal comprising at least seven peaks selected from the group consisting of 8.9°, 9.3°, 10.5°, 12.8°, 13.5°, 15.9°, 17.0°, 17.4°, 18.4°, and 19.0° (±0.2°) as diffraction angles (2θ values) in powder X-ray diffraction.
[A6-4] The crystal according to [A3], wherein the hydrate crystal is a 1G-type crystal comprising peaks of 8.9°, 9.3°, 10.5°, 12.8°, 13.5°, 15.9°, 17.0°, 17.4°, 18.4°, and 19.0° (±0.2°) as diffraction angles (2θ values) in powder X-ray diffraction.
[A7] The crystal according to any of [A6] to [A6-4], wherein the diffraction angles (2θ values) are diffraction angles (2θ values) of the hydrate crystal preserved at a temperature of 34°C and a relative humidity of 60% for 5 minutes.
[A8] The crystal according to [A3], wherein the hydrate crystal is a 1J-type crystal comprising at least one peak selected from the group consisting of 9.7°, 10.5°, 13.1°, 15.0°, 16.2°, 16.6°, 17.6°, 18.9°, 19.3°, and 20.8° (±0.2°) as diffraction angles (2θ values) in powder X-ray diffraction.
[A8-1] The crystal according to [A3], wherein the hydrate crystal is a 1J-type crystal comprising at least three peaks selected from the group consisting of 9.7°, 10.5°, 13.1°, 15.0°, 16.2°, 16.6°, 17.6°, 18.9°, 19.3°, and 20.8° (±0.2°) as diffraction angles (2θ values) in powder X-ray diffraction.
[A8-2] The crystal according to [A3], wherein the hydrate crystal is a 1J-type crystal comprising at least five peaks selected from the group consisting of 9.7°, 10.5°, 13.1°, 15.0°, 16.2°, 16.6°, 17.6°, 18.9°, 19.3°, and 20.8° (±0.2°) as diffraction angles (2θ values) in powder X-ray diffraction.
[A8-3] The crystal according to [A3], wherein the hydrate crystal is a 1J-type crystal comprising at least seven peaks selected from the group consisting of 9.7°, 10.5°, 13.1°, 15.0°, 16.2°, 16.6°, 17.6°, 18.9°, 19.3°, and 20.8° (±0.2°) as diffraction angles (2θ values) in powder X-ray diffraction.
[A8-4] The crystal according to [A3], wherein the hydrate crystal is a 1J-type crystal comprising peaks of 9.7°, 10.5°, 13.1°, 15.0°, 16.2°, 16.6°, 17.6°, 18.9°, 19.3°, and 20.8° (±0.2°) as diffraction angles (2θ values) in powder X-ray diffraction.
[A9] The crystal according to any of [A8] to [A8-4], wherein the diffraction angles (2θ values) are diffraction angles (2θ values) of the hydrate crystal preserved at a temperature of 34°C and a relative humidity of 30% for 15 minutes.
[A10] The crystal according to [A1], wherein the crystal is a non-solvate crystal of the compound represented by the formula (1).
[A11] The crystal according to [A1] or [A10], wherein the non-solvate crystal is a 1H-type crystal comprising at least one peak selected from the group consisting of 12.3°, 15.1°, 15.9°, 16.2°, 16.5°, 16.9°, 17.5°, 17.8°, 18.5°, and 19.7° (±0.2°) as diffraction angles (2θ values) in powder X-ray diffraction.
[A11-1] The crystal according to [A1] or [A10], wherein the non-solvate crystal is a 1H-type crystal comprising at least three peaks selected from the group consisting of 12.3°, 15.1°, 15.9°, 16.2°, 16.5°, 16.9°, 17.5°, 17.8°, 18.5°, and 19.7° (±0.2°) as diffraction angles (2θ values) in powder X-ray diffraction.
[A11-2] The crystal according to [A1] or [A10], wherein the non-solvate crystal is a 1H-type crystal comprising at least five peaks selected from the group consisting of 12.3°, 15.1°, 15.9°, 16.2°, 16.5°, 16.9°, 17.5°, 17.8°, 18.5°, and 19.7° (±0.2°) as diffraction angles (2θ values) in powder X-ray diffraction.
[A11-3] The crystal according to [A1] or [A10], wherein the non-solvate crystal is a 1H-type crystal comprising at least seven peaks selected from the group consisting of 12.3°, 15.1°, 15.9°, 16.2°, 16.5°, 16.9°, 17.5°, 17.8°, 18.5°, and 19.7° (±0.2°) as diffraction angles (2θ values) in powder X-ray diffraction.
[A11-4] The crystal according to [A1] or [A10], wherein the non-solvate crystal is a 1H-type crystal comprising peaks of 12.3°, 15.1°, 15.9°, 16.2°, 16.5°, 16.9°, 17.5°, 17.8°, 18.5°, and 19.7° (±0.2°) as diffraction angles (2θ values) in powder X-ray diffraction.
[A12] The crystal according to any of [A11] to [A11-4], wherein the diffraction angles (2θ values) are diffraction angles (2θ values) of the non-solvate crystal preserved at a temperature of 34°C and a relative humidity of 0% for 60 minutes.
[A13] The crystal according to [A1] or [A2], wherein the crystal is a 1K-type crystal comprising at least one peak selected from the group consisting of 9.5°, 11.3°, 16.3°, 16.6°, 17.6°, 18.4°, 19.1°, 19.9°, 21.0°, and 21.9° (±0.2°) as diffraction angles (2θ values) in powder X-ray diffraction.
[A13-1] The crystal according to [A1] or [A2], wherein the crystal is a 1K-type crystal comprising at least three peaks selected from the group consisting of 9.5°, 11.3°, 16.3°, 16.6°, 17.6°, 18.4°, 19.1°, 19.9°, 21.0°, and 21.9° (±0.2°) as diffraction angles (2θ values) in powder X-ray diffraction.
[A13-2] The crystal according to [A1] or [A2], wherein the crystal is a 1K-type crystal comprising at least five peaks selected from the group consisting of 9.5°, 11.3°, 16.3°, 16.6°, 17.6°, 18.4°, 19.1°, 19.9°, 21.0°, and 21.9° (±0.2°) as diffraction angles (2θ values) in powder X-ray diffraction.
[A13-3] The crystal according to [A1] or [A2], wherein the crystal is a 1K-type crystal comprising at least seven peaks selected from the group consisting of 9.5°, 11.3°, 16.3°, 16.6°, 17.6°, 18.4°, 19.1°, 19.9°, 21.0°, and 21.9° (±0.2°) as diffraction angles (2θ values) in powder X-ray diffraction.
[A13-4] The crystal according to [A1] or [A2], wherein the crystal is a 1K-type crystal comprising peaks of 9.5°, 11.3°, 16.3°, 16.6°, 17.6°, 18.4°, 19.1°, 19.9°, 21.0°, and 21.9° (±0.2°) as diffraction angles (2θ values) in powder X-ray diffraction.
[B1] A crystal of a compound represented by the following formula (2) or a salt thereof, or a solvate thereof: 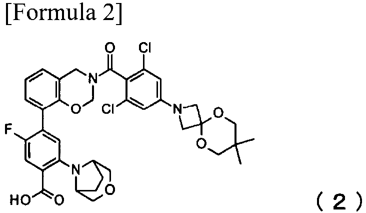
[B2] The crystal according to [B1], wherein the crystal is a non-solvate crystal of the compound represented by the formula (2).
[B3] The crystal according to [B1] or [B2], wherein the non-solvate crystal is a 3A-type crystal comprising at least one peak selected from the group consisting of 10.4°, 12.6°, 15.1°, 15.9°, 16.6°, 17.1°, 18.4°, 19.6°, 19.9°, and 20.6° (±0.2°) as diffraction angles (2θ values) in powder X-ray diffraction.
[B3-1] The crystal according to [B1] or [B2], wherein the non-solvate crystal is a 3A-type crystal comprising at least three peaks selected from the group consisting of 10.4°, 12.6°, 15.1°, 15.9°, 16.6°, 17.1°, 18.4°, 19.6°, 19.9°, and 20.6° (±0.2°) as diffraction angles (2θ values) in powder X-ray diffraction.
[B3-2] The crystal according to [B1] or [B2], wherein the non-solvate crystal is a 3A-type crystal comprising at least five peaks selected from the group consisting of 10.4°, 12.6°, 15.1°, 15.9°, 16.6°, 17.1°, 18.4°, 19.6°, 19.9°, and 20.6° (±0.2°) as diffraction angles (2θ values) in powder X-ray diffraction.
[B3-3] The crystal according to [B1] or [B2], wherein the non-solvate crystal is a 3A-type crystal comprising at least seven peaks selected from the group consisting of 10.4°, 12.6°, 15.1°, 15.9°, 16.6°, 17.1°, 18.4°, 19.6°, 19.9°, and 20.6° (±0.2°) as diffraction angles (2θ values) in powder X-ray diffraction.
[B3-4] The crystal according to [B1] or [B2], wherein the non-solvate crystal is a 3A-type crystal comprising peaks of 10.4°, 12.6°, 15.1°, 15.9°, 16.6°, 17.1°, 18.4°, 19.6°, 19.9°, and 20.6° (±0.2°) as diffraction angles (2θ values) in powder X-ray diffraction.
[B4] The crystal according to [B1] or [B2], wherein the non-solvate crystal is a 3C-type crystal comprising at least one peak selected from the group consisting of 7.9°, 8.5°, 8.8°, 9.2°, 10.4', 11.4°, 12.0°, 13.1°, 15.2°, and 15.7° (±0.2°) as diffraction angles (2θ values) in powder X-ray diffraction.
[B4-1] The crystal according to [B1] or [B2], wherein the non-solvate crystal is a 3C-type crystal comprising at least three peaks selected from the group consisting of 7.9°, 8.5°, 8.8°, 9.2°, 10.4°, 11.4°, 12.0°, 13.1°, 15.2°, and 15.7° (±0.2°) as diffraction angles (2θ values) in powder X-ray diffraction.
[B4-2] The crystal according to [B1] or [B2], wherein the non-solvate crystal is a 3C-type crystal comprising at least five peaks selected from the group consisting of 7.9°, 8.5°, 8.8°, 9.2°, 10.4°, 11.4°, 12.0°, 13.1°, 15.2°, and 15.7° (±0.2°) as diffraction angles (2θ values) in powder X-ray diffraction.
[B4-3] The crystal according to [B1] or [B2], wherein the non-solvate crystal is a 3C-type crystal comprising at least seven peaks selected from the group consisting of 7.9°, 8.5°, 8.8°, 9.2°, 10.4°, 11.4°, 12.0°, 13.1°, 15.2°, and 15.7° (±0.2°) as diffraction angles (2θ values) in powder X-ray diffraction.
[B4-4] The crystal according to [B1] or [B2], wherein the non-solvate crystal is a 3C-type crystal comprising peaks of 7.9°, 8.5°, 8.8°, 9.2°, 10.4°, 11.4°, 12.0°, 13.1°, 15.2°, and 15.7° (±0.2°) as diffraction angles (2θ values) in powder X-ray diffraction.
[B5] The crystal according to [B1], wherein the crystal is a solvate crystal of the compound represented by the formula (2).
[B6] The crystal according to [B1] or [B5], wherein the crystal is a 3D-type crystal comprising at least one peak selected from the group consisting of 6.6°, 7.3°, 10.9°, 11.8°, 13.9°, 17.1°, 17.5°, 18.3°, 19.3°, and 22.0° (±0.2°) as diffraction angles (2θ values) in powder X-ray diffraction.
[B6-1] The crystal according to [B1] or [B5], wherein the crystal is a 3D-type crystal comprising at least three peaks selected from the group consisting of 6.6°, 7.3°, 10.9°, 11.8°, 13.9°, 17.1°, 17.5°, 18.3°, 19.3°, and 22.0° (±0.2°) as diffraction angles (2θ values) in powder X-ray diffraction.
[B6-2] The crystal according to [B1] or [B5], wherein the crystal is a 3D-type crystal comprising at least five peaks selected from the group consisting of 6.6°, 7.3°, 10.9°, 11.8°, 13.9°, 17.1°, 17.5°, 18.3°, 19.3°, and 22.0° (±0.2°) as diffraction angles (2θ values) in powder X-ray diffraction.
[B6-3] The crystal according to [B1] or [B5], wherein the crystal is a 3D-type crystal comprising at least seven peaks selected from the group consisting of 6.6°, 7.3°, 10.9°, 11.8°, 13.9°, 17.1°, 17.5°, 18.3°, 19.3°, and 22.0° (±0.2°) as diffraction angles (2θ values) in powder X-ray diffraction.
[B6-4] The crystal according to [B1] or [B5], wherein the crystal is a 3D-type crystal comprising peaks of 6.6°, 7.3°, 10.9°, 11.8°, 13.9°, 17.1°, 17.5°, 18.3°, 19.3°, and 22.0° (±0.2°) as diffraction angles (2θ values) in powder X-ray diffraction.
[B7] The crystal according to [B1] or [B5], wherein the crystal is a 3B-type crystal comprising at least one peak selected from the group consisting of 7.4°, 14.0°, 14.6°, 15.5°, 17.1°, 18.1°, 19.8°, 21.8°, 22.4°, and 24.1° (±0.2°) as diffraction angles (2θ values) in powder X-ray diffraction.
[B7-1] The crystal according to [B1] or [B5], wherein the crystal is a 3B-type crystal comprising at least three peaks selected from the group consisting of 7.4°, 14.0°, 14.6°, 15.5°, 17.1°, 18.1°, 19.8°, 21.8°, 22.4°, and 24.1° (±0.2°) as diffraction angles (2θ values) in powder X-ray diffraction.
[B7-2] The crystal according to [B1] or [B5], wherein the crystal is a 3B-type crystal comprising at least five peaks selected from the group consisting of 7.4°, 14.0°, 14.6°, 15.5°, 17.1°, 18.1°, 19.8°, 21.8°, 22.4°, and 24.1° (±0.2°) as diffraction angles (2θ values) in powder X-ray diffraction.
[B7-3] The crystal according to [B1] or [B5], wherein the crystal is a 3B-type crystal comprising at least seven peaks selected from the group consisting of 7.4°, 14.0°, 14.6°, 15.5°, 17.1°, 18.1°, 19.8°, 21.8°, 22.4°, and 24.1° (±0.2°) as diffraction angles (2θ values) in powder X-ray diffraction.
[B7-4] The crystal according to [B1] or [B5], wherein the crystal is a 3B-type crystal comprising peaks of 7.4°, 14.0°, 14.6°, 15.5°, 17.1°, 18.1°, 19.8°, 21.8°, 22.4°, and 24.1° (±0.2°) as diffraction angles (2θ values) in powder X-ray diffraction.
[C1] A crystal of a compound represented by the following formula (3) or a salt thereof, or a solvate thereof: 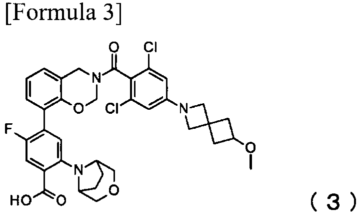
[C2] The crystal according to [C1], wherein the crystal is a non-solvate crystal of the compound represented by the formula (3).
[C3] The crystal according to [C1] or [C2], wherein the non-solvate crystal is a 2B-type crystal comprising at least one peak selected from the group consisting of 7.2°, 7.6°, 13.4°, 14.5°, 16.0°, 16.6°, 17.3°, 19.9°, 20.6°, and 22.4° (±0.2°) as diffraction angles (2θ values) in powder X-ray diffraction.
[C3-1] The crystal according to [C1] or [C2], wherein the non-solvate crystal is a 2B-type crystal comprising at least three peaks selected from the group consisting of 7.2°, 7.6°, 13.4°, 14.5°, 16.0°, 16.6°, 17.3°, 19.9°, 20.6°, and 22.4° (±0.2°) as diffraction angles (2θ values) in powder X-ray diffraction.
[C3-2] The crystal according to [C1] or [C2], wherein the non-solvate crystal is a 2B-type crystal comprising at least five peaks selected from the group consisting of 7.2°, 7.6°, 13.4°, 14.5°, 16.0°, 16.6°, 17.3°, 19.9°, 20.6°, and 22.4° (±0.2°) as diffraction angles (2θ values) in powder X-ray diffraction.
[C3-3] The crystal according to [C1] or [C2], wherein the non-solvate crystal is a 2B-type crystal comprising at least seven peaks selected from the group consisting of 7.2°, 7.6°, 13.4°, 14.5°, 16.0°, 16.6°, 17.3°, 19.9°, 20.6°, and 22.4° (±0.2°) as diffraction angles (2θ values) in powder X-ray diffraction.
[C3-4] The crystal according to [C1] or [C2], wherein the non-solvate crystal is a 2B-type crystal comprising peaks of 7.2°, 7.6°, 13.4°, 14.5°, 16.0°, 16.6°, 17.3°, 19.9°, 20.6°, and 22.4° (±0.2°) as diffraction angles (2θ values) in powder X-ray diffraction.
[C4] The crystal according to [C1] or [C2], wherein the non-solvate crystal is a 2A-type crystal comprising at least one peak selected from the group consisting of 7.5°, 10.0°, 10.7°, 13.7°, 14.6°, 15.1°, 16.8°, 17.4°, 18.0°, and 18.5° (±0.2°) as diffraction angles (2θ values) in powder X-ray diffraction.
[C4-1] The crystal according to [C1] or [C2], wherein the non-solvate crystal is a 2A-type crystal comprising at least three peaks selected from the group consisting of 7.5°, 10.0°, 10.7°, 13.7°, 14.6°, 15.1°, 16.8°, 17.4°, 18.0°, and 18.5° (±0.2°) as diffraction angles (2θ values) in powder X-ray diffraction.
[C4-2] The crystal according to [C1] or [C2], wherein the non-solvate crystal is a 2A-type crystal comprising at least five peaks selected from the group consisting of 7.5°, 10.0°, 10.7°, 13.7°, 14.6°, 15.1°, 16.8°, 17.4°, 18.0°, and 18.5° (±0.2°) as diffraction angles (2θ values) in powder X-ray diffraction.
[C4-3] The crystal according to [C1] or [C2], wherein the non-solvate crystal is a 2A-type crystal comprising at least seven peaks selected from the group consisting of 7.5°, 10.0°, 10.7°, 13.7°, 14.6°, 15.1°, 16.8°, 17.4°, 18.0°, and 18.5° (±0.2°) as diffraction angles (2θ values) in powder X-ray diffraction.
[C4-4] The crystal according to [C1] or [C2], wherein the non-solvate crystal is a 2A-type crystal comprising peaks of 7.5°, 10.0°, 10.7°, 13.7°, 14.6°, 15.1°, 16.8°, 17.4°, 18.0°, and 18.5° (±0.2°) as diffraction angles (2θ values) in powder X-ray diffraction.
[C5] The crystal according to [C1] or [C2], wherein the non-solvate crystal is a 2C-type crystal comprising at least one peak selected from the group consisting of 7.2°, 9.9°, 14.5°, 15.3°, 15.7°, 17.2°, 17.6°, 18.0°, 19.1°, and 19.8° (±0.2°) as diffraction angles (2θ values) in powder X-ray diffraction.
[C5-1] The crystal according to [C1] or [C2], wherein the non-solvate crystal is a 2C-type crystal comprising at least three peaks selected from the group consisting of 7.2°, 9.9°, 14.5°, 15.3°, 15.7°, 17.2°, 17.6°, 18.0°, 19.1°, and 19.8° (±0.2°) as diffraction angles (2θ values) in powder X-ray diffraction.
[C5-2] The crystal according to [C1] or [C2], wherein the non-solvate crystal is a 2C-type crystal comprising at least five peaks selected from the group consisting of 7.2°, 9.9°, 14.5°, 15.3°, 15.7°, 17.2°, 17.6°, 18.0°, 19.1°, and 19.8° (±0.2°) as diffraction angles (2θ values) in powder X-ray diffraction.
[C5-3] The crystal according to [C1] or [C2], wherein the non-solvate crystal is a 2C-type crystal comprising at least seven peaks selected from the group consisting of 7.2°, 9.9°, 14.5°, 15.3°, 15.7°, 17.2°, 17.6°, 18.0°, 19.1°, and 19.8° (±0.2°) as diffraction angles (2θ values) in powder X-ray diffraction.
[C5-4] The crystal according to [C1] or [C2], wherein the non-solvate crystal is a 2C-type crystal comprising peaks of 7.2°, 9.9°, 14.5°, 15.3°, 15.7°, 17.2°, 17.6°, 18.0°, 19.1°, and 19.8° (±0.2°) as diffraction angles (2θ values) in powder X-ray diffraction.
[C6] The crystal according to [C1] or [C2], wherein the non-solvate crystal is a 2F-type crystal comprising at least one peak selected from the group consisting of 7.3°, 9.9°, 11.4°, 14.5°, 15.3°, 16.4°, 16.8°, 17.2°, 17.7°, and 18.1° (±0.2°) as diffraction angles (2θ values) in powder X-ray diffraction.
[C6-1] The crystal according to [C1] or [C2], wherein the non-solvate crystal is a 2F-type crystal comprising at least three peaks selected from the group consisting of 7.3°, 9.9°, 11.4°, 14.5°, 15.3°, 16.4°, 16.8°, 17.2°, 17.7°, and 18.1° (±0.2°) as diffraction angles (2θ values) in powder X-ray diffraction.
[C6-2] The crystal according to [C1] or [C2], wherein the non-solvate crystal is a 2F-type crystal comprising at least five peaks selected from the group consisting of 7.3°, 9.9°, 11.4°, 14.5°, 15.3°, 16.4°, 16.8°, 17.2°, 17.7°, and 18.1° (±0.2°) as diffraction angles (2θ values) in powder X-ray diffraction.
[C6-3] The crystal according to [C1] or [C2], wherein the non-solvate crystal is a 2F-type crystal comprising at least seven peaks selected from the group consisting of 7.3°, 9.9°, 11.4°, 14.5°, 15.3°, 16.4°, 16.8°, 17.2°, 17.7°, and 18.1° (±0.2°) as diffraction angles (2θ values) in powder X-ray diffraction.
[C6-4] The crystal according to [C1] or [C2], wherein the non-solvate crystal is a 2F-type crystal comprising peaks of 7.3°, 9.9°, 11.4°, 14.5°, 15.3°, 16.4°, 16.8°, 17.2°, 17.7°, and 18.1° (±0.2°) as diffraction angles (2θ values) in powder X-ray diffraction.
[D1] A crystal of a compound represented by the following formula (4) or a salt thereof, or a solvate thereof: 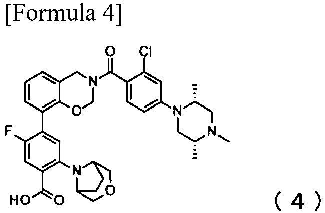
[D2] The crystal according to [D1], wherein the crystal is a solvate crystal of the compound represented by the formula (4).
[D3] The crystal according to [D1] or [D2], wherein the solvate crystal is a hydrate crystal.
[D4] The crystal according to [D3], wherein the hydrate crystal is a 5A-type crystal comprising at least one peak selected from the group consisting of 7.4°, 8.8°, 9.5°, 10.4°, 12.2°, 15.5°, 16.6°, 17.1°, 17.5°, and 18.1° (±0.2°) as diffraction angles (2θ values) in powder X-ray diffraction.
[D4-1] The crystal according to [D3], wherein the hydrate crystal is a 5A-type crystal comprising at least three peaks selected from the group consisting of 7.4°, 8.8°, 9.5°, 10.4°, 12.2°, 15.5°, 16.6°, 17.1°, 17.5°, and 18.1° (±0.2°) as diffraction angles (2θ values) in powder X-ray diffraction.
[D4-2] The crystal according to [D3], wherein the hydrate crystal is a 5A-type crystal comprising at least five peaks selected from the group consisting of 7.4°, 8.8°, 9.5°, 10.4°, 12.2°, 15.5°, 16.6°, 17.1°, 17.5°, and 18.1° (±0.2°) as diffraction angles (2θ values) in powder X-ray diffraction.
[D4-3] The crystal according to [D3], wherein the hydrate crystal is a 5A-type crystal comprising at least seven peaks selected from the group consisting of 7.4°, 8.8°, 9.5°, 10.4°, 12.2°, 15.5°, 16.6°, 17.1°, 17.5°, and 18.1° (±0.2°) as diffraction angles (2θ values) in powder X-ray diffraction.
[D4-4] The crystal according to [D3], wherein the hydrate crystal is a 5A-type crystal comprising peaks of 7.4°, 8.8°, 9.5°, 10.4°, 12.2°, 15.5°, 16.6°, 17.1°, 17.5°, and 18.1° (±0.2°) as diffraction angles (2θ values) in powder X-ray diffraction.
[D5] The crystal according to [D3], wherein the hydrate crystal is a 5A-type crystal comprising peaks of 7.4°, 8.8°, and 9.5° (±0.2°) as diffraction angles (2θ values) in powder X-ray diffraction.
[D6] The crystal according to [D3], wherein the hydrate crystal is a 5A-type crystal comprising at least one peak selected from the group consisting of 7.4°, 8.8°, 9.5°, 10.8°, 12.7°, 16.0°, 16.8°, 17.3°, 17.9°, and 19.2° (±0.2°) as diffraction angles (2θ values) in powder X-ray diffraction.
[D6-1] The crystal according to [D3], wherein the hydrate crystal is a 5A-type crystal comprising at least three peaks selected from the group consisting of 7.4°, 8.8°, 9.5°, 10.8°, 12.7°, 16.0°, 16.8°, 17.3°, 17.9°, and 19.2° (±0.2°) as diffraction angles (2θ values) in powder X-ray diffraction.
[D6-2] The crystal according to [D3], wherein the hydrate crystal is a 5A-type crystal comprising at least five peaks selected from the group consisting of 7.4°, 8.8°, 9.5°, 10.8°, 12.7°, 16.0°, 16.8°, 17.3°, 17.9°, and 19.2° (±0.2°) as diffraction angles (2θ values) in powder X-ray diffraction.
[D6-3] The crystal according to [D3], wherein the hydrate crystal is a 5A-type crystal comprising at least seven peaks selected from the group consisting of 7.4°, 8.8°, 9.5°, 10.8°, 12.7°, 16.0°, 16.8°, 17.3°, 17.9°, and 19.2° (±0.2°) as diffraction angles (2θ values) in powder X-ray diffraction.
[D6-4] The crystal according to [D3], wherein the hydrate crystal is a 5A-type crystal comprising peaks of 7.4°, 8.8°, 9.5°, 10.8°, 12.7°, 16.0°, 16.8°, 17.3°, 17.9°, and 19.2° (±0.2°) as diffraction angles (2θ values) in powder X-ray diffraction.
[D7] The crystal according to any of [D6] to [D6-4], wherein the diffraction angles (2θ values) are diffraction angles (2θ values) of the hydrate crystal preserved at a temperature of 25°C and a relative humidity of 10% for 1 hour.
[D8] The crystal according to [D3], wherein the hydrate crystal is a 5A-type crystal comprising at least one peak selected from the group consisting of 7.4°, 8.8°, 9.5°, 10.5°, 12.2°, 15.6°, 16.6°, 17.1°, 17.3°, and 17.5° (±0.2°) as diffraction angles (2θ values) in powder X-ray diffraction.
[D8-1] The crystal according to [D3], wherein the hydrate crystal is a 5A-type crystal comprising at least three peaks selected from the group consisting of 7.4°, 8.8°, 9.5°, 10.5°, 12.2°, 15.6°, 16.6°, 17.1°, 17.3°, and 17.5° (±0.2°) as diffraction angles (2θ values) in powder X-ray diffraction.
[D8-2] The crystal according to [D3], wherein the hydrate crystal is a 5A-type crystal comprising at least five peaks selected from the group consisting of 7.4°, 8.8°, 9.5°, 10.5°, 12.2°, 15.6°, 16.6°, 17.1°, 17.3°, and 17.5° (±0.2°) as diffraction angles (2θ values) in powder X-ray diffraction.
[D8-3] The crystal according to [D3], wherein the hydrate crystal is a 5A-type crystal comprising at least seven peaks selected from the group consisting of 7.4°, 8.8°, 9.5°, 10.5°, 12.2°, 15.6°, 16.6°, 17.1°, 17.3°, and 17.5° (±0.2°) as diffraction angles (2θ values) in powder X-ray diffraction.
[D8-4] The crystal according to [D3], wherein the hydrate crystal is a 5A-type crystal comprising peaks of 7.4°, 8.8°, 9.5°, 10.5°, 12.2°, 15.6°, 16.6°, 17.1°, 17.3°, and 17.5° (±0.2°) as diffraction angles (2θ values) in powder X-ray diffraction.
[D9] The crystal according to any of [D8] to [D8-4], wherein the diffraction angles (2θ values) are diffraction angles (2θ values) of the hydrate crystal preserved at a temperature of 25°C and a relative humidity of 20% for 1 hour.
[D10] The crystal according to [D3], wherein the hydrate crystal is a 5A-type crystal comprising at least one peak selected from the group consisting of 7.4°, 8.8°, 9.5°, 10.5°, 12.2°, 15.5°, 16.4°, 16.6°, 17.1°, and 17.6° (±0.2°) as diffraction angles (2θ values) in powder X-ray diffraction.
[D10-1] The crystal according to [D3], wherein the hydrate crystal is a 5A-type crystal comprising at least three peaks selected from the group consisting of 7.4°, 8.8°, 9.5°, 10.5°, 12.2°, 15.5°, 16.4°, 16.6°, 17.1°, and 17.6° (±0.2°) as diffraction angles (2θ values) in powder X-ray diffraction.
[D10-2] The crystal according to [D3], wherein the hydrate crystal is a 5A-type crystal comprising at least five peaks selected from the group consisting of 7.4°, 8.8°, 9.5°, 10.5°, 12.2°, 15.5°, 16.4°, 16.6°, 17.1°, and 17.6° (±0.2°) as diffraction angles (2θ values) in powder X-ray diffraction.
[D10-3] The crystal according to [D3], wherein the hydrate crystal is a 5A-type crystal comprising at least seven peaks selected from the group consisting of 7.4°, 8.8°, 9.5°, 10.5°, 12.2°, 15.5°, 16.4°, 16.6°, 17.1°, and 17.6° (±0.2°) as diffraction angles (2θ values) in powder X-ray diffraction.
[D10-4] The crystal according to [D3], wherein the hydrate crystal is a 5A-type crystal comprising peaks of 7.4°, 8.8°, 9.5°, 10.5°, 12.2°, 15.5°, 16.4°, 16.6°, 17.1°, and 17.6° (±0.2°) as diffraction angles (2θ values) in powder X-ray diffraction.
[D11] The crystal according to any of [D 10] to [D10-4], wherein the diffraction angles (2θ values) are diffraction angles (2θ values) of the hydrate crystal preserved at a temperature of 25°C and a relative humidity of 30% for 1 hour.
[D12] The crystal according to [D3], wherein the hydrate crystal is a 5A-type crystal comprising at least one peak selected from the group consisting of 7.4°, 8.8°, 9.5°, 10.3°, 12.2°, 15.4°, 16.3°, 16.6°, 17.0°, and 17.6° (±0.2°) as diffraction angles (2θ values) in powder X-ray diffraction.
[D12-1] The crystal according to [D3], wherein the hydrate crystal is a 5A-type crystal comprising at least three peaks selected from the group consisting of 7.4°, 8.8°, 9.5°, 10.3°, 12.2°, 15.4°, 16.3°, 16.6°, 17.0°, and 17.6° (±0.2°) as diffraction angles (2θ values) in powder X-ray diffraction.
[D12-2] The crystal according to [D3], wherein the hydrate crystal is a 5A-type crystal comprising at least five peaks selected from the group consisting of 7.4°, 8.8°, 9.5°, 10.3°, 12.2°, 15.4°, 16.3°, 16.6°, 17.0°, and 17.6° (±0.2°) as diffraction angles (2θ values) in powder X-ray diffraction.
[D12-3] The crystal according to [D3], wherein the hydrate crystal is a 5A-type crystal comprising at least seven peaks selected from the group consisting of 7.4°, 8.8°, 9.5°, 10.3°, 12.2°, 15.4°, 16.3°, 16.6°, 17.0°, and 17.6° (±0.2°) as diffraction angles (2θ values) in powder X-ray diffraction.
[D12-4] The crystal according to [D3], wherein the hydrate crystal is a 5A-type crystal comprising peaks of 7.4°, 8.8°, 9.5°, 10.3°, 12.2°, 15.4°, 16.3°, 16.6°, 17.0°, and 17.6° (±0.2°) as diffraction angles (2θ values) in powder X-ray diffraction.
[D13] The crystal according to any of [D12] to [D12-4], wherein the diffraction angles (2θ values) are diffraction angles (2θ values) of the hydrate crystal preserved at a temperature of 25°C and a relative humidity of 60% for 1 hour.
[D14] The crystal according to [D3], wherein the hydrate crystal is a 5E-type crystal comprising at least one peak selected from the group consisting of 9.2°, 10.6°, 11.9°, 13.7°, 15.0°, 15.8°, 16.6°, 17.1°, 18.5°, and 19.2° (±0.2°) as diffraction angles (2θ values) in powder X-ray diffraction.
[D14-1] The crystal according to [D3], wherein the hydrate crystal is a 5E-type crystal comprising at least three peaks selected from the group consisting of 9.2°, 10.6°, 11.9°, 13.7°, 15.0°, 15.8°, 16.6°, 17.1°, 18.5°, and 19.2° (±0.2°) as diffraction angles (2θ values) in powder X-ray diffraction.
[D14-2] The crystal according to [D3], wherein the hydrate crystal is a 5E-type crystal comprising at least five peaks selected from the group consisting of 9.2°, 10.6°, 11.9°, 13.7°, 15.0°, 15.8°, 16.6°, 17.1°, 18.5°, and 19.2° (10.2°) as diffraction angles (2θ values) in powder X-ray diffraction.
[D14-3] The crystal according to [D3], wherein the hydrate crystal is a 5E-type crystal comprising at least seven peaks selected from the group consisting of 9.2°, 10.6°, 11.9°, 13.7°, 15.0°, 15.8°, 16.6°, 17.1°, 18.5°, and 19.2° (±0.2°) as diffraction angles (2θ values) in powder X-ray diffraction.
[D14-4] The crystal according to [D3], wherein the hydrate crystal is a 5E-type crystal comprising peaks of 9.2°, 10.6°, 11.9°, 13.7°, 15.0°, 15.8°, 16.6°, 17.1°, 18.5°, and 19.2° (±0.2°) as diffraction angles (2θ values) in powder X-ray diffraction.
[D15] The crystal according to [D3], wherein the hydrate crystal is a 5J-type crystal comprising peaks of 7.3°, 9.0°, 9.6°, 10.8°, 12.2°, 15.7°, 16.6°, and 17.3° (±0.2°) as diffraction angles (2θ values) in powder X-ray diffraction.
[D16] The crystal according to [D3], wherein the hydrate crystal is a 5J-type crystal comprising at least one peak selected from the group consisting of 7.3°, 9.0°, 9.6°, 10.9°, 12.3°, 15.9°, 16.6°, 17.1°, and 17.5° (±0.2°) as diffraction angles (2θ values) in powder X-ray diffraction.
[D16-1] The crystal according to [D3], wherein the hydrate crystal is a 5J-type crystal comprising at least three peaks selected from the group consisting of 7.3°, 9.0°, 9.6°, 10.9°, 12.3°, 15.9°, 16.6°, 17.1°, and 17.5° (±0.2°) as diffraction angles (2θ values) in powder X-ray diffraction.
[D16-2] The crystal according to [D3], wherein the hydrate crystal is a 5J-type crystal comprising at least five peaks selected from the group consisting of 7.3°, 9.0°, 9.6°, 10.9°, 12.3°, 15.9°, 16.6°, 17.1°, and 17.5° (±0.2°) as diffraction angles (2θ values) in powder X-ray diffraction.
[D16-3] The crystal according to [D3], wherein the hydrate crystal is a 5J-type crystal comprising at least seven peaks selected from the group consisting of 7.3°, 9.0°, 9.6°, 10.9°, 12.3°, 15.9°, 16.6°, 17.1°, and 17.5° (±0.2°) as diffraction angles (2θ values) in powder X-ray diffraction.
[D16-4] The crystal according to [D3], wherein the hydrate crystal is a 5J-type crystal comprising peaks of 7.3°, 9.0°, 9.6°, 10.9°, 12.3°, 15.9°, 16.6°, 17.1°, and 17.5° (±0.2°) as diffraction angles (2θ values) in powder X-ray diffraction.
[D17] The crystal according to any of [D16] to [D16-4], wherein the diffraction angles (2θ values) are diffraction angles (2θ values) of the hydrate crystal preserved at a temperature of 25°C and a relative humidity of 30% for 1 hour.
[D18] The crystal according to [D3], wherein the hydrate crystal is a 5J-type crystal comprising at least one peak selected from the group consisting of 7.3°, 8.9°, 9.6°, 10.8°, 12.2°, 15.7°, 16.3°, 16.6°, 17.0°, and 17.3° (±0.2°) as diffraction angles (2θ values) in powder X-ray diffraction.
[D18-1] The crystal according to [D3], wherein the hydrate crystal is a 5J-type crystal comprising at least three peaks selected from the group consisting of 7.3°, 8.9°, 9.6°, 10.8°, 12.2°, 15.7°, 16.3°, 16.6°, 17.0°, and 17.3° (±0.2°) as diffraction angles (2θ values) in powder X-ray diffraction.
[D18-2] The crystal according to [D3], wherein the hydrate crystal is a 5J-type crystal comprising at least five peaks selected from the group consisting of 7.3°, 8.9°, 9.6°, 10.8°, 12.2°, 15.7°, 16.3°, 16.6°, 17.0°, and 17.3° (±0.2°) as diffraction angles (2θ values) in powder X-ray diffraction.
[D18-3] The crystal according to [D3], wherein the hydrate crystal is a 5J-type crystal comprising at least seven peaks selected from the group consisting of 7.3°, 8.9°, 9.6°, 10.8°, 12.2°, 15.7°, 16.3°, 16.6°, 17.0°, and 17.3° (±0.2°) as diffraction angles (2θ values) in powder X-ray diffraction.
[D18-4] The crystal according to [D3], wherein the hydrate crystal is a 5J-type crystal comprising peaks of 7.3°, 8.9°, 9.6°, 10.8°, 12.2°, 15.7°, 16.3°, 16.6°, 17.0°, and 17.3° (±0.2°) as diffraction angles (2θ values) in powder X-ray diffraction.
[D19] The crystal according to any of [D18] to [D18-4], wherein the diffraction angles (2θ values) are diffraction angles (2θ values) of the hydrate crystal preserved at a temperature of 25°C and a relative humidity of 40% for 1 hour.
[D20] The crystal according to [D3], wherein the hydrate crystal is a 5J-type crystal comprising at least one peak selected from the group consisting of 7.3°, 8.9°, 9.6°, 10.7°, 12.1°, 15.6°, 16.2°, 16.6°, 16.9°, and 17.2° (±0.2°) as diffraction angles (2θ values) in powder X-ray diffraction.
[D20-1] The crystal according to [D3], wherein the hydrate crystal is a 5J-type crystal comprising at least three peaks selected from the group consisting of 7.3°, 8.9°, 9.6°, 10.7°, 12.1°, 15.6°, 16.2°, 16.6°, 16.9°, and 17.2° (±0.2°) as diffraction angles (2θ values) in powder X-ray diffraction.
[D20-2] The crystal according to [D3], wherein the hydrate crystal is a 5J-type crystal comprising at least five peaks selected from the group consisting of 7.3°, 8.9°, 9.6°, 10.7°, 12.1°, 15.6°, 16.2°, 16.6°, 16.9°, and 17.2° (±0.2°) as diffraction angles (2θ values) in powder X-ray diffraction.
[D20-3] The crystal according to [D3], wherein the hydrate crystal is a 5J-type crystal comprising at least seven peaks selected from the group consisting of 7.3°, 8.9°, 9.6°, 10.7°, 12.1°, 15.6°, 16.2°, 16.6°, 16.9°, and 17.2° (±0.2°) as diffraction angles (2θ values) in powder X-ray diffraction.
[D20-4] The crystal according to [D3], wherein the hydrate crystal is a 5J-type crystal comprising peaks of 7.3°, 8.9°, 9.6°, 10.7°, 12.1°, 15.6°, 16.2°, 16.6°, 16.9°, and 17.2° (±0.2°) as diffraction angles (2θ values) in powder X-ray diffraction.
[D21] The crystal according to any of [D20] to [D20-4], wherein the diffraction angles (2θ values) are diffraction angles (2θ values) of the hydrate crystal preserved at a temperature of 25°C and a relative humidity of 50 to 60% for 1 hour.
[D22] The crystal according to [D3], wherein the hydrate crystal is a 5J-type crystal comprising at least one peak selected from the group consisting of 7.2°, 8.9°, 9.5°, 10.6°, 12.0°, 15.5°, 16.1°, 16.6°, and 17.1° (±0.2°) as diffraction angles (2θ values) in powder X-ray diffraction.
[D22-1] The crystal according to [D3], wherein the hydrate crystal is a 5J-type crystal comprising at least three peaks selected from the group consisting of 7.2°, 8.9°, 9.5°, 10.6°, 12.0°, 15.5°, 16.1°, 16.6°, and 17.1° (±0.2°) as diffraction angles (2θ values) in powder X-ray diffraction.
[D22-2] The crystal according to [D3], wherein the hydrate crystal is a 5J-type crystal comprising at least five peaks selected from the group consisting of 7.2°, 8.9°, 9.5°, 10.6°, 12.0°, 15.5°, 16.1°, 16.6°, and 17.1° (±0.2°) as diffraction angles (2θ values) in powder X-ray diffraction.
[D22-3] The crystal according to [D3], wherein the hydrate crystal is a 5J-type crystal comprising at least seven peaks selected from the group consisting of 7.2°, 8.9°, 9.5°, 10.6°, 12.0°, 15.5°, 16.1°, 16.6°, and 17.1° (±0.2°) as diffraction angles (2θ values) in powder X-ray diffraction.
[D22-4] The crystal according to [D3], wherein the hydrate crystal is a 5J-type crystal comprising peaks of 7.2°, 8.9°, 9.5°, 10.6°, 12.0°, 15.5°, 16.1°, 16.6°, and 17.1° (±0.2°) as diffraction angles (2θ values) in powder X-ray diffraction.
[D23] The crystal according to any of [D22] to [D22-4], wherein the diffraction angles (2θ values) are diffraction angles (2θ values) of the hydrate crystal preserved at a temperature of 25°C and a relative humidity of 70% or higher for 1 hour.
[D24] The crystal according to [D1], wherein the crystal is a non-solvate crystal of the compound represented by the formula (4).
[D25] The crystal according to [D1] or [D24], wherein the crystal is a 5C-type crystal comprising at least one peak selected from the group consisting of 7.3°, 9.7°, 12.1°, 14.8°, 15.2°, 15.7°, 16.6°, and 17.7° (±0.2°) as diffraction angles (2θ values) in powder X-ray diffraction.
[D25-1] The crystal according to [D1] or [D24], wherein the crystal is a 5C-type crystal comprising at least three peaks selected from the group consisting of 7.3°, 9.7°, 12.1°, 14.8°, 15.2°, 15.7°, 16.6°, and 17.7° (±0.2°) as diffraction angles (2θ values) in powder X-ray diffraction.
[D25-2] The crystal according to [D1] or [D24], wherein the crystal is a 5C-type crystal comprising at least five peaks selected from the group consisting of 7.3°, 9.7°, 12.1°, 14.8°, 15.2°, 15.7°, 16.6°, and 17.7° (±0.2°) as diffraction angles (2θ values) in powder X-ray diffraction.
[D25-3] The crystal according to [D1] or [D24], wherein the crystal is a 5C-type crystal comprising at least seven peaks selected from the group consisting of 7.3°, 9.7°, 12.1°, 14.8°, 15.2°, 15.7°, 16.6°, and 17.7° (±0.2°) as diffraction angles (2θ values) in powder X-ray diffraction.
[D25-4] The crystal according to [D1] or [D24], wherein the crystal is a 5C-type crystal comprising peaks of 7.3°, 9.7°, 12.1°, 14.8°, 15.2°, 15.7°, 16.6°, and 17.7° (±0.2°) as diffraction angles (2θ values) in powder X-ray diffraction.
[D26] The crystal according to [D1] or [D24], wherein the non-solvate crystal is a 5D-type crystal comprising at least one peak selected from the group consisting of 11.3°, 13.1°, 13.8°, 14.9°, 15.9°, 16.1°, 16.7°, 17.5°, 18.1°, and 18.5° (±0.2°) as diffraction angles (2θ values) in powder X-ray diffraction.
[D26-1] The crystal according to [D1] or [D24], wherein the non-solvate crystal is a 5D-type crystal comprising at least three peaks selected from the group consisting of 11.3°, 13.1°, 13.8°, 14.9°, 15.9°, 16.1°, 16.7°, 17.5°, 18.1°, and 18.5° (±0.2°) as diffraction angles (2θ values) in powder X-ray diffraction.
[D26-2] The crystal according to [D1] or [D24], wherein the non-solvate crystal is a 5D-type crystal comprising at least five peaks selected from the group consisting of 11.3°, 13.1°, 13.8°, 14.9°, 15.9°, 16.1°, 16.7°, 17.5°, 18.1°, and 18.5° (±0.2°) as diffraction angles (2θ values) in powder X-ray diffraction.
[D26-3] The crystal according to [D1] or [D24], wherein the non-solvate crystal is a 5D-type crystal comprising at least seven peaks selected from the group consisting of 11.3°, 13.1°, 13.8°, 14.9°, 15.9°, 16.1°, 16.7°, 17.5°, 18.1°, and 18.5° (±0.2°) as diffraction angles (2θ values) in powder X-ray diffraction.
[D26-4] The crystal according to [D1] or [D24], wherein the non-solvate crystal is a 5D-type crystal comprising peaks of 11.3°, 13.1°, 13.8°, 14.9°, 15.9°, 16.1°, 16.7°, 17.5°, 18.1°, and 18.5° (±0.2°) as diffraction angles (2θ values) in powder X-ray diffraction.
[D27] The crystal according to [D1] or [D2], wherein the crystal is a 5G-type crystal comprising at least one peak selected from the group consisting of 9.4°, 11.0°, 13.4°, 15.0°, 16.1°, 17.8°, 18.1°, 19.7°, 20.8°, and 22.0° (±0.2°) as diffraction angles (2θ values) in powder X-ray diffraction.
[D27-1] The crystal according to [D1] or [D2], wherein the crystal is a 5G-type crystal comprising at least three peaks selected from the group consisting of 9.4°, 11.0°, 13.4°, 15.0°, 16.1°, 17.8°, 18.1°, 19.7°, 20.8°, and 22.0° (±0.2°) as diffraction angles (2θ values) in powder X-ray diffraction.
[D27-2] The crystal according to [D1] or [D2], wherein the crystal is a 5G-type crystal comprising at least five peaks selected from the group consisting of 9.4°, 11.0°, 13.4°, 15.0°, 16.1°, 17.8°, 18.1°, 19.7°, 20.8°, and 22.0° (±0.2°) as diffraction angles (2θ values) in powder X-ray diffraction.
[D27-3] The crystal according to [D1] or [D2], wherein the crystal is a 5G-type crystal comprising at least seven peaks selected from the group consisting of 9.4°, 11.0°, 13.4°, 15.0°, 16.1°, 17.8°, 18.1°, 19.7°, 20.8°, and 22.0° (±0.2°) as diffraction angles (2θ values) in powder X-ray diffraction.
[D27-4] The crystal according to [D1] or [D2], wherein the crystal is a 5G-type crystal comprising peaks of 9.4°, 11.0°, 13.4°, 15.0°, 16.1°, 17.8°, 18.1°, 19.7°, 20.8°, and 22.0° (±0.2°) as diffraction angles (2θ values) in powder X-ray diffraction.
[E1] A crystal of a compound represented by the following formula (5) or a salt thereof, or a solvate thereof: 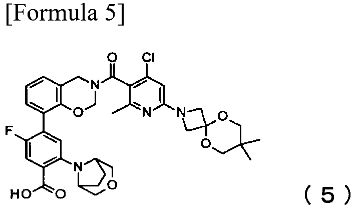
[E2] The crystal according to [E1], wherein the crystal is a solvate crystal of the compound represented by the formula (5).
[E3] The crystal according to [E1] or [E2], wherein the solvate crystal is a hydrate crystal.
[E4] The crystal according to [E3], wherein the hydrate crystal is a 4B-type crystal comprising at least one peak selected from the group consisting of 7.7°, 13.3°, 14.0°, 15.4°, 16.2°, 17.9°, 18.2°, 18.5°, 19.1°, and 20.6° (±0.2°) as diffraction angles (2θ values) in powder X-ray diffraction.
[E4-1] The crystal according to [E3], wherein the hydrate crystal is a 4B-type crystal comprising at least three peaks selected from the group consisting of 7.7°, 13.3°, 14.0°, 15.4°, 16.2°, 17.9°, 18.2°, 18.5°, 19.1°, and 20.6° (±0.2°) as diffraction angles (2θ values) in powder X-ray diffraction.
[E4-2] The crystal according to [E3], wherein the hydrate crystal is a 4B-type crystal comprising at least five peaks selected from the group consisting of 7.7°, 13.3°, 14.0°, 15.4°, 16.2°, 17.9°, 18.2°, 18.5°, 19.1°, and 20.6° (±0.2°) as diffraction angles (2θ values) in powder X-ray diffraction.
[E4-3] The crystal according to [E3], wherein the hydrate crystal is a 4B-type crystal comprising at least seven peaks selected from the group consisting of 7.7°, 13.3°, 14.0°, 15.4°, 16.2°, 17.9°, 18.2°, 18.5°, 19.1°, and 20.6° (±0.2°) as diffraction angles (2θ values) in powder X-ray diffraction.
[E4-4] The crystal according to [E3], wherein the hydrate crystal is a 4B-type crystal comprising peaks of 7.7°, 13.3°, 14.0°, 15.4°, 16.2°, 17.9°, 18.2°, 18.5°, 19.1°, and 20.6° (±0.2°) as diffraction angles (2θ values) in powder X-ray diffraction.
[E5] The crystal according to [E1] or [E2], wherein the crystal is a 4A-type crystal comprising at least one peak selected from the group consisting of 11.5°, 11.8°, 15.7°, 17.2°, 17.5°, 18.1°, 19.5°, 20.3°, 20.5°, and 21.2° (±0.2°) as diffraction angles (2θ values) in powder X-ray diffraction.
[E5-1] The crystal according to [E1] or [E2], wherein the crystal is a 4A-type crystal comprising at least three peaks selected from the group consisting of 11.5°, 11.8°, 15.7°, 17.2°, 17.5°, 18.1°, 19.5°, 20.3°, 20.5°, and 21.2° (±0.2°) as diffraction angles (2θ values) in powder X-ray diffraction.
[E5-2] The crystal according to [E1] or [E2], wherein the crystal is a 4A-type crystal comprising at least five peaks selected from the group consisting of 11.5°, 11.8°, 15.7°, 17.2°, 17.5°, 18.1°, 19.5°, 20.3°, 20.5°, and 21.2° (±0.2°) as diffraction angles (2θ values) in powder X-ray diffraction.
[E5-3] The crystal according to [E1] or [E2], wherein the crystal is a 4A-type crystal comprising at least seven peaks selected from the group consisting of 11.5°, 11.8°, 15.7°, 17.2°, 17.5°, 18.1°, 19.5°, 20.3°, 20.5°, and 21.2° (±0.2°) as diffraction angles (2θ values) in powder X-ray diffraction.
[E5-4] The crystal according to [E1] or [E2], wherein the crystal is a 4A-type crystal comprising peaks of 11.5°, 11.8°, 15.7°, 17.2°, 17.5°, 18.1°, 19.5°, 20.3°, 20.5°, and 21.2° (±0.2°) as diffraction angles (2θ values) in powder X-ray diffraction.
[F1] A crystal of a compound represented by any of the following formulas (1) to (5) or a salt thereof, or a solvate thereof: 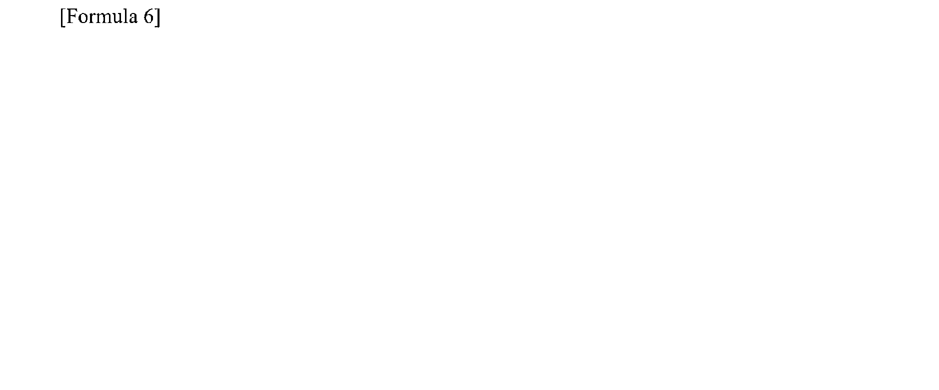 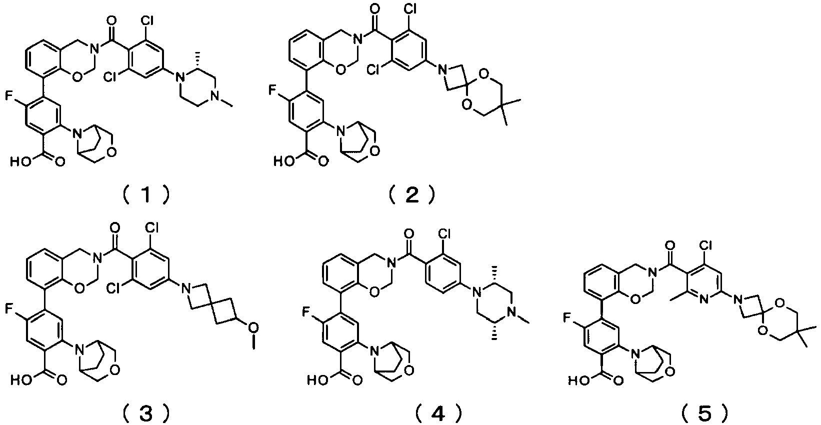
[F2] The crystal according to [F1], wherein the crystal is a crystal of a compound or a salt thereof, or a solvate thereof according to any of [A1] to [A13-4], [B1] to [B7-4], [C1] to [C6-4], [D1] to [D27-4], and [E1] to [E5-4].
[G1] A pharmaceutical composition comprising a crystal of a compound or a salt thereof, or a solvate thereof according to any of [A1] to [A13-4], [B1] to [B7-4], [C1] to [C6-4], [D1] to [D27-4], [E1] to [E5-4], and [F1] to [F2].
[G2] The pharmaceutical composition according to [G1] for the prevention and/or treatment of neurodegenerative disease, lung disease, or kidney disease.
[G3] A method for preventing and/or treating neurodegenerative disease, lung disease, or kidney disease, comprising administering an effective amount of a crystal of a compound or a salt thereof, or a solvate thereof according to any of [A1] to [A13-4], [B1] to [B7-4], [C1] to [C6-4], [D1] to [D27-4], [E1] to [E5-4], and [F1] to [F2] to a subject.
[G4] The crystal of a compound or a salt thereof, or a solvate thereof according to any of [A1] to [A13-4], [B1] to [B7-4], [C1] to [C6-4], [D1] to [D27-4], [E1] to [E5-4], and [F1] to [F2] for use in the prevention and/or treatment of neurodegenerative disease, lung disease, or kidney disease.
[G5] Use of a crystal of a compound or a salt thereof, or a solvate thereof according to any of [A1] to [A13-4], [B1] to [B7-4], [C 1] to [C6-4], [D1] to [D27-4], [E1] to [E5-4], and [F1] to [F2] for producing a pharmaceutical composition for the prevention and/or treatment of neurodegenerative disease, lung disease, or kidney disease.
[G6] A pharmaceutical composition comprising a crystal of a compound or a salt thereof, or a solvate thereof according to any of [A1] to [A13-4], [B1] to [B7-4], [C1] to [C6-4], [D1] to [D27-4], [E1] to [E5-4], and [F1] to [F2] mixed with a pharmacologically acceptable carrier or medium.
[G7] A pharmaceutical composition comprising a crystal of a compound or a salt thereof, or a solvate thereof according to any of [A1] to [A13-4], [B1] to [B7-4], [C1] to [C6-4], [D1] to [D27-4], [E1] to [E5-4], and [F1] to [F2] dissolved in a pharmacologically acceptable carrier or medium.
[H1] A method for producing a pharmaceutical composition containing a compound represented by any of the following formulas (1) to (5) or a salt thereof, or a solvate thereof as an active ingredient, comprising the step of
mixing a crystal of a compound or a salt thereof, or a solvate thereof according to any of [A1] to [A13-4], [Bl] to [B7-4], [C1] to [C6-4], [D1] to [D27-4], and [E1] to [E5-4] with a pharmacologically acceptable carrier or medium: 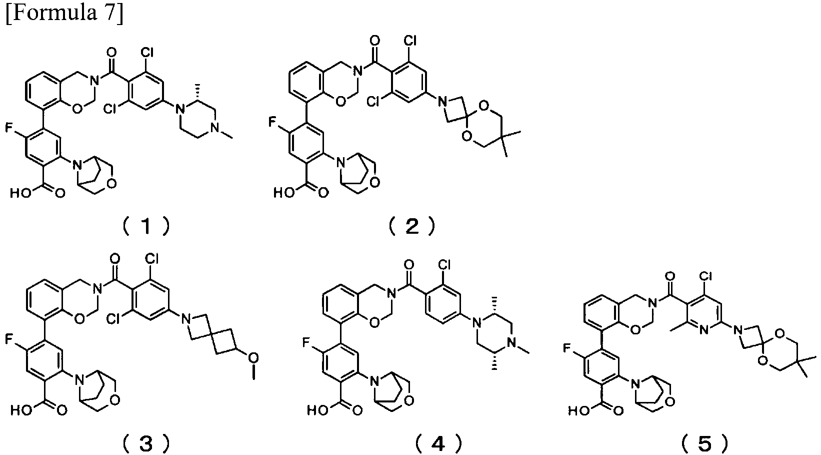
[H2] The method according to [H1], wherein the crystal of a compound or a salt thereof, or a solvate thereof is a solvate crystal of the compound.
[H3] The method according to [H2], wherein the solvate crystal is a hydrate crystal.
[H4] The method according to [H1], wherein the crystal of a compound or a salt thereof, or a solvate thereof is a non-solvate crystal of the compound.
[H5] The method according to any of [H1] to [H4], wherein the step of mixing the crystal of a compound or a salt thereof, or a solvate thereof with a pharmacologically acceptable carrier or medium is the step of dissolving the crystal of a compound or a salt thereof, or a solvate thereof in a pharmacologically acceptable carrier or medium.
[H6] A method for producing a pharmaceutical composition containing a compound represented by any of the following formulas (1) to (5) or a salt thereof, or a solvate thereof as an active ingredient, comprising the step of
mixing the compound or the salt thereof, or a solvate thereof with a pharmacologically acceptable carrier or medium: 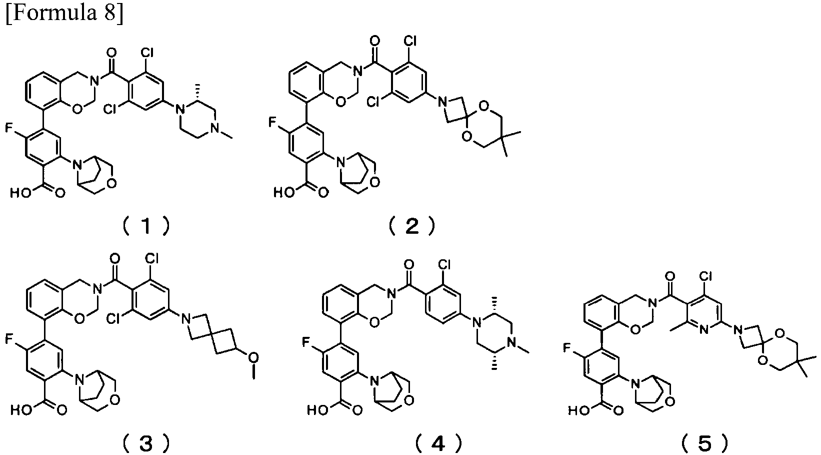
[H7] The method according to [H6], wherein the compound represented by any of the formulas (1) to (5) or the salt thereof, or a solvate thereof is a solvate of the compound represented by any of the formulas (1) to (5).
[H8] The method according to [H7], wherein the solvate is a hydrate.
[H9] The method according to [H6], wherein the compound represented by any of the formulas (1) to (5) or the salt thereof, or a solvate thereof is a non-solvate of the compound represented by any of the formulas (1) to (5).
[H10] The method according to any of [H6] to [H9], wherein the step of mixing the compound or the salt thereof, or a solvate thereof with a pharmacologically acceptable carrier or medium is the step of dissolving the compound or the salt thereof, or a solvate thereof in a pharmacologically acceptable carrier or medium.

In the numbering described above, each number cited in a dependent item includes a branch number of the number unless otherwise specified. For example, [A4] cited in a dependent item includes [A4] as well as its branch numbers [A4-1], [A4-2], [A4-3], and [A4-4]. The same holds true for the other numbers.

### Advantageous Effects of Invention

The present invention provides a crystal of a compound or a salt thereof, or a solvate thereof which has a Nrf2-activating effect, the crystals being capable of providing a prophylactic agent or a therapeutic agent for various diseases, for example, neurodegenerative disease, lung disease, and kidney disease.

### Brief Description of Drawings

[Figure 1] Figure 1 shows results of powder X-ray diffractometry of crystals obtained in Example 2-1-1. The ordinate depicts diffraction intensity, and the abscissa depicts a diffraction angle 2θ (°).
[Figure 2] Figure 2 shows results of TG-DTA measurement of crystals obtained in Example 2-1-1. The ordinate of Figure 2(A) depicts change in weight, and the abscissa depicts a temperature. The ordinate of Figure 2(B) depicts heat flow, and the abscissa depicts a temperature. The + mark with a temperature described on the graph of Figure 2(B) represents a melting point or other transition points.
[Figure 3] Figure 3 shows results of powder X-ray diffractometry of crystals obtained in Example 2-1-3. The ordinate depicts diffraction intensity, and the abscissa depicts a diffraction angle 2θ (°).
[Figure 4] Figure 4 shows results of TG-DTA measurement of crystals obtained in Example 2-1-3. The ordinate of Figure 4(A) depicts change in weight, and the abscissa depicts a temperature. The ordinate of Figure 4(B) depicts heat flow, and the abscissa depicts a temperature. The + mark with a temperature described on the graph of Figure 4(B) represents a melting point or other transition points.
[Figure 5] Figure 5 shows results of powder X-ray diffractometry under a humidity adjustment condition obtained in Example 2-1-4. The ordinate depicts diffraction intensity, and the abscissa depicts a diffraction angle 2θ (°). The relative humidity (RH) of each pattern is shown in the drawing. A broad peak around 6.4° is derived from a measurement apparatus.
[Figure 6] Figure 6 shows results of powder X-ray diffractometry of crystals obtained in Example 2-1-5. The ordinate depicts diffraction intensity, and the abscissa depicts a diffraction angle 2θ (°).
[Figure 7] Figure 7 shows the crystal structure of 1G-type crystals described in Example 2-1-6.
[Figure 8] Figure 8 shows results of powder X-ray diffractometry of crystals obtained in Example 2-2-2. The ordinate depicts diffraction intensity, and the abscissa depicts a diffraction angle 2θ (°).
[Figure 9] Figure 9 shows results of powder X-ray diffractometry of crystals obtained in Example 2-2-4. The ordinate depicts diffraction intensity, and the abscissa depicts a diffraction angle 2θ (°).
[Figure 10] Figure 10 shows results of TG-DTA measurement of crystals obtained in Example 2-2-4. The ordinate of Figure 10(A) depicts change in weight, and the abscissa depicts a temperature. The ordinate of Figure 10(B) depicts heat flow, and the abscissa depicts a temperature. The + mark with a temperature described on the graph of Figure 10(B) represents a melting point or other transition points.
[Figure 11] Figure 11 shows results of powder X-ray diffractometry of crystals obtained in Example 2-2-5. The ordinate depicts diffraction intensity, and the abscissa depicts a diffraction angle 20 (°).
[Figure 12] Figure 12 shows results of TG-DTA measurement of crystals obtained in Example 2-2-5. The ordinate of Figure 12(A) depicts change in weight, and the abscissa depicts a temperature. The ordinate of Figure 12(B) depicts heat flow, and the abscissa depicts a temperature. The + mark with a temperature described on the graph of Figure 12(B) represents a melting point or other transition points.
[Figure 13] Figure 13 shows results of powder X-ray diffractometry of crystals obtained in Example 2-2-6. The ordinate depicts diffraction intensity, and the abscissa depicts a diffraction angle 2θ (°).
[Figure 14] Figure 14 shows results of TG-DTA measurement of crystals obtained in Example 2-2-6. The ordinate of Figure 14(A) depicts change in weight, and the abscissa depicts a temperature. The ordinate of Figure 14(B) depicts heat flow, and the abscissa depicts a temperature. The + mark with a temperature described on the graph of Figure 14(B) represents a melting point or other transition points.
[Figure 15] Figure 15 shows results of powder X-ray diffractometry of crystals before vacuum drying obtained in Example 2-3-1. The ordinate depicts diffraction intensity, and the abscissa depicts a diffraction angle 2θ (°).
[Figure 16] Figure 16 shows results of powder X-ray diffractometry of crystals after vacuum drying obtained in Example 2-3-1. The ordinate depicts diffraction intensity, and the abscissa depicts a diffraction angle 2θ (°).
[Figure 17] Figure 17 shows results of TG-DTA measurement of crystals obtained in Example 2-3-1. The ordinate of Figure 17(A) depicts change in weight, and the abscissa depicts a temperature. The ordinate of Figure 17(B) depicts heat flow, and the abscissa depicts a temperature. The + mark with a temperature described on the graph of Figure 17(B) represents a melting point or other transition points.
[Figure 18] Figure 18 shows results of powder X-ray diffractometry of crystals obtained in Example 2-3-2. The ordinate depicts diffraction intensity, and the abscissa depicts a diffraction angle 2θ (°).
[Figure 19] Figure 19 shows results of powder X-ray diffractometry of crystals obtained in Example 2-3-3. The ordinate depicts diffraction intensity, and the abscissa depicts a diffraction angle 2θ (°).
[Figure 20] Figure 20 shows results of TG-DTA measurement of crystals obtained in Example 2-3-3. The ordinate of Figure 20(A) depicts change in weight, and the abscissa depicts a temperature. The ordinate of Figure 20(B) depicts heat flow, and the abscissa depicts a temperature. The + mark with a temperature described on the graph of Figure 20(B) represents a melting point or other transition points.
[Figure 21] Figure 21 shows results of powder X-ray diffractometry of crystals obtained in Example 2-4-1. The ordinate depicts diffraction intensity, and the abscissa depicts a diffraction angle 2θ (°).
[Figure 22] Figure 22 shows the crystal structure of 4B-type crystals described in Example 2-4-2.
[Figure 23] Figure 23 shows results of powder X-ray diffractometry of crystals obtained in Example 2-4-3. The ordinate depicts diffraction intensity, and the abscissa depicts a diffraction angle 2θ (°).
[Figure 24] Figure 24 shows results of TG-DTA measurement of crystals obtained in Example 2-4-3. The ordinate of Figure 24(A) depicts change in weight, and the abscissa depicts a temperature. The ordinate of Figure 24(B) depicts heat flow, and the abscissa depicts a temperature. The + mark with a temperature described on the graph of Figure 24(B) represents a melting point or other transition points.
[Figure 25] Figure 25 shows results of powder X-ray diffractometry of crystals obtained in Example 2-5-2. The ordinate depicts diffraction intensity, and the abscissa depicts a diffraction angle 2θ (°).
[Figure 26] Figure 26 shows results of TG-DTA measurement of crystals obtained in Example 2-5-2. The ordinate of Figure 26(A) depicts change in weight, and the abscissa depicts a temperature. The ordinate of Figure 26(B) depicts heat flow, and the abscissa depicts a temperature. The + mark with a temperature described on the graph of Figure 26(B) represents a melting point or other transition points.
[Figure 27] Figure 27 shows results of powder X-ray diffractometry of crystals before vacuum drying obtained in Example 2-5-3. The ordinate depicts diffraction intensity, and the abscissa depicts a diffraction angle 2θ (°).
[Figure 28] Figure 28 shows results of TG-DTA measurement of crystals before vacuum drying obtained in Example 2-5-3. The ordinate of Figure 28(A) depicts change in weight, and the abscissa depicts a temperature. The ordinate of Figure 28(B) depicts heat flow, and the abscissa depicts a temperature. The + mark with a temperature described on the graph of Figure 28(B) represents a melting point or other transition points.
[Figure 29] Figure 29 shows results of powder X-ray diffractometry of crystals after vacuum drying obtained in Example 2-5-3. The ordinate depicts diffraction intensity, and the abscissa depicts a diffraction angle 2θ (°).
[Figure 30] Figure 30 shows results of powder X-ray diffractometry under each humidity condition obtained in Example 2-5-4. The ordinate depicts diffraction intensity, and the abscissa depicts a diffraction angle 2θ (°). The relative humidity (RH) of each pattern is shown in the drawing.
[Figure 31] Figure 31 shows results (first and second runs) of powder X-ray diffractometry under a relative humidity (RH) condition of 60% obtained in Example 2-5-4. The ordinate depicts diffraction intensity, and the abscissa depicts a diffraction angle 2θ (°). The RH and measurement timing of each pattern are shown in the drawing. A broad peak around 6.4° is derived from a measurement apparatus.
[Figure 32] Figure 32 shows results of powder X-ray diffractometry of crystals obtained in Example 2-5-5. The ordinate depicts diffraction intensity, and the abscissa depicts a diffraction angle 2θ (°).
[Figure 33] Figure 33 shows results of powder X-ray diffractometry of crystals obtained in Example 2-5-6. The ordinate depicts diffraction intensity, and the abscissa depicts a diffraction angle 2θ (°).
[Figure 34] Figure 34 shows the crystal structure of 5A-type crystals described in Example 2-5-6.
[Figure 35] Figure 35 shows results of powder X-ray diffractometry under a relative humidity condition of 5% obtained in Example 2-5-8. The ordinate depicts diffraction intensity, and the abscissa depicts a diffraction angle 2θ (°). A broad peak around 6.4° is derived from a measurement apparatus.
[Figure 36] Figure 36 shows typical results of powder X-ray diffractometry under each humidity condition obtained in Example 2-5-9. The ordinate depicts diffraction intensity, and the abscissa depicts a diffraction angle 2θ (°). The relative humidity (RH) of each pattern is shown in the drawing. A broad peak around 6.4° is derived from a measurement apparatus.

### Description of Embodiments

Hereinafter, the present invention will be described in detail with reference to the definition of symbols, terms, and the like described in the present specification, the mode for carrying out the present invention, etc.

In an aspect, the present invention relates to crystals of a compound represented by the formulas (1) to (5) or a salt thereof, or a solvate thereof. Examples of the crystals of this compound specifically include non-solvate crystals and solvate crystals of this compound, and non-solvate crystals and solvate crystals of the salt of this compound. Examples of the solvate crystals specifically include hydrate crystals, acetone solvate crystals, dimethyl sulfoxide (DMSO) solvate crystals, dimethylformamide (DMF) solvate crystals, acetonitrile solvate crystals, tetrahydrofuran solvate crystals, methyl ethyl ketone solvate crystals, methyl isobutyl ketone solvate crystals, ethyl acetate solvate crystals, heptane solvate crystals, polyethylene glycol (PEG) solvate crystals, 2-propanol solvate crystals, methanol solvate crystals, and ethanol solvate crystals and preferably include hydrate crystals.

The compound described in the present specification can be a salt thereof, or a solvate thereof. In the present specification, the "compound or the salt thereof, or a solvate thereof " includes the compound, a salt of the compound, a solvate of the compound, and a solvate of the salt of the compound. Examples of the salt of the compound include: hydrochloride; hydrobromide; hydroiodide; phosphate; phosphonate; sulfate; sulfonate such as methanesulfonate and p-toluenesulfonate; carboxylate such as acetate, citrate, malate, tartrate, succinate, and salicylate; alkali metal salts such as sodium salt and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced, for example, by contacting the compound with an acid or a base. In the present specification, the solvate refers to one molecular population formed by the compound with a solvent and is not particularly limited as long as the solvate is formed from a solvent whose ingestion is accepted in association with the administration of a medicament. Examples thereof include not only hydrates, alcoholates (ethanolate, methanolate, 1-propanolate, 2-propanolate, etc.), and solvates with a single solvent such as dimethyl sulfoxide but a solvate formed from a plurality of solvents per molecule of the compound, and a solvate formed from plural types of solvents per molecule of the compound. The solvate is referred to as a hydrate when the solvent is water. The solvate of the compound of the present invention is preferably a hydrate. Specifically, such a hydrate is mono- to decahydrates, preferably mono- to pentahydrates, further preferably mono- to trihydrates.

Diffraction angles 2θ in powder X-ray diffraction are diffraction peaks measured using CuKα or CuKα1 radiation. Crystals further defined by diffraction angles 2θ in powder X-ray diffraction of these solvate crystals are also referred to as, for example, "1A-type crystals" of a hydrate given below and also simply referred to as "1A type".

In the notation of diffraction angles 2θ, the term "(±0.2°)" described at the end of listed diffraction angles 2θ means that the range of ±0.2° is acceptable for respective values described about all the listed diffraction angles 2θ.

In an aspect, the hydrate crystal of the compound of the formula (1) is a 1A-type crystal comprising at least one of the following peaks as diffraction angles 2θ in powder X-ray diffraction.
6.0°, 9.3°, 9.7°, 10.4°, 11.9°, 12.9°, 15.3°, 15.9°, 16.4°, and 16.8° (±0.2°)

In an aspect, the hydrate crystal of the compound of the formula (1) is a 1A-type crystal comprising at least three of the following peaks as diffraction angles 2θ in powder X-ray diffraction.
6.0°, 9.3°, 9.7°, 10.4°, 11.9°, 12.9°, 15.3°, 15.9°, 16.4°, and 16.8° (±0.2°)

In an aspect, the hydrate crystal of the compound of the formula (1) is a 1A-type crystal comprising at least five of the following peaks as diffraction angles 2θ in powder X-ray diffraction.
6.0°, 9.3°, 9.7°, 10.4°, 11.9°, 12.9°, 15.3°, 15.9°, 16.4°, and 16.8° (±0.2°)

In an aspect, the hydrate crystal of the compound of the formula (1) is a 1A-type crystal comprising at least seven of the following peaks as diffraction angles 2θ in powder X-ray diffraction.
6.0°, 9.3°, 9.7°, 10.4°, 11.9°, 12.9°, 15.3°, 15.9°, 16.4°, and 16.8° (±0.2°)

In an aspect, the hydrate crystal of the compound of the formula (1) is a 1A-type crystal comprising the following peaks as diffraction angles 2θ in powder X-ray diffraction. 6.0°, 9.3°, 9.7°, 10.4°, 11.9°, 12.9°, 15.3°, 15.9°, 16.4°, and 16.8° (±0.2°)

In an aspect, the hydrate crystal of the compound of the formula (1) is a 1G-type crystal comprising at least one of the following peaks as diffraction angles 2θ in powder X-ray diffraction.
8.8°, 9.2°, 10.4°, 12.7°, 13.4°, 15.8°, 16.9°, 17.3°, 18.3°, and 18.9° (±0.2°)

In an aspect, the hydrate crystal of the compound of the formula (1) is a 1G-type crystal comprising at least three of the following peaks as diffraction angles 2θ in powder X-ray diffraction.
8.8°, 9.2°, 10.4°, 12.7°, 13.4°, 15.8°, 16.9°, 17.3°, 18.3°, and 18.9° (±0.2°)

In an aspect, the hydrate crystal of the compound of the formula (1) is a 1G-type crystal comprising at least five of the following peaks as diffraction angles 2θ in powder X-ray diffraction.
8.8°, 9.2°, 10.4°, 12.7°, 13.4°, 15.8°, 16.9°, 17.3°, 18.3°, and 18.9° (±0.2°)

In an aspect, the hydrate crystal of the compound of the formula (1) is a 1G-type crystal comprising at least seven of the following peaks as diffraction angles 2θ in powder X-ray diffraction.
8.8°, 9.2°, 10.4°, 12.7°, 13.4°, 15.8°, 16.9°, 17.3°, 18.3°, and 18.9° (±0.2°)

In an aspect, the hydrate crystal of the compound of the formula (1) is a 1G-type crystal comprising the following peaks as diffraction angles 2θ in powder X-ray diffraction.
8.8°, 9.2°, 10.4°, 12.7°, 13.4°, 15.8°, 16.9°, 17.3°, 18.3°, and 18.9° (±0.2°)

In an aspect, the hydrate crystal of the compound of the formula (1) is a 1G-type crystal comprising at least one of the following peaks as diffraction angles 2θ in powder X-ray diffraction. The following diffraction angles (2θ values) are preferably diffraction angles (2θ values) of the hydrate crystal preserved at a temperature of 34°C and a relative humidity of 60% for 5 minutes.
8.9°, 9.3°, 10.5°, 12.8°, 13.5°, 15.9°, 17.0°, 17.4°, 18.4°, and 19.0° (±0.2°)

In an aspect, the hydrate crystal of the compound of the formula (1) is a 1G-type crystal comprising at least three of the following peaks as diffraction angles 2θ in powder X-ray diffraction. The following diffraction angles (2θ values) are preferably diffraction angles (2θ values) of the hydrate crystal preserved at a temperature of 34°C and a relative humidity of 60% for 5 minutes.
8.9°, 9.3°, 10.5°, 12.8°, 13.5°, 15.9°, 17.0°, 17.4°, 18.4°, and 19.0° (±0.2°)

In an aspect, the hydrate crystal of the compound of the formula (1) is a 1G-type crystal comprising at least five of the following peaks as diffraction angles 2θ in powder X-ray diffraction. The following diffraction angles (2θ values) are preferably diffraction angles (2θ values) of the hydrate crystal preserved at a temperature of 34°C and a relative humidity of 60% for 5 minutes.
8.9°, 9.3°, 10.5°, 12.8°, 13.5°, 15.9°, 17.0°, 17.4°, 18.4°, and 19.0° (±0.2°)

In an aspect, the hydrate crystal of the compound of the formula (1) is a 1G-type crystal comprising at least seven of the following peaks as diffraction angles 2θ in powder X-ray diffraction. The following diffraction angles (2θ values) are preferably diffraction angles (2θ values) of the hydrate crystal preserved at a temperature of 34°C and a relative humidity of 60% for 5 minutes.
8.9°, 9.3°, 10.5°, 12.8°, 13.5°, 15.9°, 17.0°, 17.4°, 18.4°, and 19.0° (±0.2°)

In an aspect, the hydrate crystal of the compound of the formula (1) is a 1G-type crystal comprising the following peaks as diffraction angles 2θ in powder X-ray diffraction. The following diffraction angles (2θ values) are preferably diffraction angles (2θ values) of the hydrate crystal preserved at a temperature of 34°C and a relative humidity of 60% for 5 minutes. 8.9°, 9.3°, 10.5°, 12.8°, 13.5°, 15.9°, 17.0°, 17.4°, 18.4°, and 19.0° (±0.2°)

In an aspect, the hydrate crystal of the compound of the formula (1) is a 1J-type crystal comprising at least one of the following peaks as diffraction angles 2θ in powder X-ray diffraction. The following diffraction angles (2θ values) may be diffraction angles (2θ values) of the hydrate crystal preserved at a temperature of 34°C and a relative humidity of 30% for 15 minutes.
9.7°, 10.5°, 13.1°, 15.0°, 16.2°, 16.6°, 17.6°, 18.9°, 19.3°, and 20.8° (±0.2°)

In an aspect, the hydrate crystal of the compound of the formula (1) is a 1J-type crystal comprising at least three of the following peaks as diffraction angles 2θ in powder X-ray diffraction. The following diffraction angles (2θ values) may be diffraction angles (2θ values) of the hydrate crystal preserved at a temperature of 34°C and a relative humidity of 30% for 15 minutes.
9.7°, 10.5°, 13.1°, 15.0°, 16.2°, 16.6°, 17.6°, 18.9°, 19.3°, and 20.8° (±0.2°)

In an aspect, the hydrate crystal of the compound of the formula (1) is a 1J-type crystal comprising at least five of the following peaks as diffraction angles 2θ in powder X-ray diffraction. The following diffraction angles (2θ values) may be diffraction angles (2θ values) of the hydrate crystal preserved at a temperature of 34°C and a relative humidity of 30% for 15 minutes.
9.7°, 10.5°, 13.1°, 15.0°, 16.2°, 16.6°, 17.6°, 18.9°, 19.3°, and 20.8° (±0.2°)

In an aspect, the hydrate crystal of the compound of the formula (1) is a 1J-type crystal comprising at least seven of the following peaks as diffraction angles 2θ in powder X-ray diffraction. The following diffraction angles (2θ values) may be diffraction angles (2θ values) of the hydrate crystal preserved at a temperature of 34°C and a relative humidity of 30% for 15 minutes.
9.7°, 10.5°, 13.1°, 15.0°, 16.2°, 16.6°, 17.6°, 18.9°, 19.3°, and 20.8° (±0.2°)

In an aspect, the hydrate crystal of the compound of the formula (1) is a 1J-type crystal comprising the following peaks as diffraction angles 2θ in powder X-ray diffraction. The following diffraction angles (2θ values) may be diffraction angles (2θ values) of the hydrate crystal preserved at a temperature of 34°C and a relative humidity of 30% for 15 minutes.
9.7°, 10.5°, 13.1°, 15.0°, 16.2°, 16.6°, 17.6°, 18.9°, 19.3°, and 20.8° (±0.2°)

In an aspect, the non-solvate crystal of the compound of the formula (1) is a 1H-type crystal comprising at least one of the following peaks as diffraction angles 20 in powder X-ray diffraction. The following diffraction angles (2θ values) may be diffraction angles (2θ values) of the non-hydrate crystal preserved at a temperature of 34°C and a relative humidity of 0% for 60 minutes.
12.3°, 15.1°, 15.9°, 16.2°, 16.5°, 16.9°, 17.5°, 17.8°, 18.5°, and 19.7° (±0.2°)

In an aspect, the non-solvate crystal of the compound of the formula (1) is a 1H-type crystal comprising at least three of the following peaks as diffraction angles 2θ in powder X-ray diffraction. The following diffraction angles (2θ values) may be diffraction angles (2θ values) of the non-hydrate crystal preserved at a temperature of 34°C and a relative humidity of 0% for 60 minutes.
12.3°, 15.1°, 15.9°, 16.2°, 16.5°, 16.9°, 17.5°, 17.8°, 18.5°, and 19.7° (±0.2°)

In an aspect, the non-solvate crystal of the compound of the formula (1) is a 1H-type crystal comprising at least five of the following peaks as diffraction angles 2θ in powder X-ray diffraction. The following diffraction angles (2θ values) may be diffraction angles (2θ values) of the non-hydrate crystal preserved at a temperature of 34°C and a relative humidity of 0% for 60 minutes.
12.3°, 15.1°, 15.9°, 16.2°, 16.5°, 16.9°, 17.5°, 17.8°, 18.5°, and 19.7° (±0.2°)

In an aspect, the non-solvate crystal of the compound of the formula (1) is a 1H-type crystal comprising at least seven of the following peaks as diffraction angles 2θ in powder X-ray diffraction. The following diffraction angles (2θ values) may be diffraction angles (2θ values) of the non-hydrate crystal preserved at a temperature of 34°C and a relative humidity of 0% for 60 minutes.
12.3°, 15.1°, 15.9°, 16.2°, 16.5°, 16.9°, 17.5°, 17.8°, 18.5°, and 19.7° (±0.2°)

In an aspect, the non-solvate crystal of the compound of the formula (1) is a 1H-type crystal comprising the following peaks as diffraction angles 2θ in powder X-ray diffraction. The following diffraction angles (2θ values) may be diffraction angles (2θ values) of the non-hydrate crystal preserved at a temperature of 34°C and a relative humidity of 0% for 60 minutes. 12.3°, 15.1°, 15.9°, 16.2°, 16.5°, 16.9°, 17.5°, 17.8°, 18.5°, and 19.7° (±0.2°)

In an aspect, the crystal of the compound of the formula (1) is a 1K-type crystal comprising at least one of the following peaks as diffraction angles 2θ in powder X-ray diffraction.
9.5°, 11.3°, 16.3°, 16.6°, 17.6°, 18.4°, 19.1°, 19.9°, 21.0°, and 21.9° (±0.2°)

In an aspect, the crystal of the compound of the formula (1) is a 1K-type crystal comprising at least three of the following peaks as diffraction angles 2θ in powder X-ray diffraction.
9.5°, 11.3°, 16.3°, 16.6°, 17.6°, 18.4°, 19.1°, 19.9°, 21.0°, and 21.9° (±0.2°)

In an aspect, the crystal of the compound of the formula (1) is a 1K-type crystal comprising at least five of the following peaks as diffraction angles 2θ in powder X-ray diffraction.
9.5°, 11.3°, 16.3°, 16.6°, 17.6°, 18.4°, 19.1°, 19.9°, 21.0°, and 21.9° (±0.2°)

In an aspect, the crystal of the compound of the formula (1) is a 1K-type crystal comprising at least seven of the following peaks as diffraction angles 2θ in powder X-ray diffraction.
9.5°, 11.3°, 16.3°, 16.6°, 17.6°, 18.4°, 19.1°, 19.9°, 21.0°, and 21.9° (±0.2°)

In an aspect, the crystal of the compound of the formula (1) is a lK-type crystal comprising the following peaks as diffraction angles 2θ in powder X-ray diffraction.
9.5°, 11.3°, 16.3°, 16.6°, 17.6°, 18.4°, 19.1°, 19.9°, 21.0°, and 21.9° (±0.2°)

In an aspect, the non-solvate crystal of the compound of the formula (2) is a 3A-type crystal comprising at least one of the following peaks as diffraction angles 2θ in powder X-ray diffraction.
10.4°, 12.6°, 15.1°, 15.9°, 16.6°, 17.1°, 18.4°, 19.6°, 19.9°, and 20.6° (±0.2°)

In an aspect, the non-solvate crystal of the compound of the formula (2) is a 3A-type crystal comprising at least three of the following peaks as diffraction angles 2θ in powder X-ray diffraction.
10.4°, 12.6°, 15.1°, 15.9°, 16.6°, 17.1°, 18.4°, 19.6°, 19.9°, and 20.6° (±0.2°)

In an aspect, the non-solvate crystal of the compound of the formula (2) is a 3A-type crystal comprising at least five of the following peaks as diffraction angles 2θ in powder X-ray diffraction.
10.4°, 12.6°, 15.1°, 15.9°, 16.6°, 17.1°, 18.4°, 19.6°, 19.9°, and 20.6° (±0.2°)

In an aspect, the non-solvate crystal of the compound of the formula (2) is a 3A-type crystal comprising at least seven of the following peaks as diffraction angles 2θ in powder X-ray diffraction.
10.4°, 12.6°, 15.1°, 15.9°, 16.6°, 17.1°, 18.4°, 19.6°, 19.9°, and 20.6° (±0.2°)

In an aspect, the non-solvate crystal of the compound of the formula (2) is a 3A-type crystal comprising the following peaks as diffraction angles 2θ in powder X-ray diffraction. 10.4°, 12.6°, 15.1°, 15.9°, 16.6°, 17.1°, 18.4°, 19.6°, 19.9°, and 20.6° (±0.2°)

In an aspect, the non-solvate crystal of the compound of the formula (2) is a 3C-type crystal comprising at least one of the following peaks as diffraction angles 2θ in powder X-ray diffraction.
7.9°, 8.5°, 8.8°, 9.2°, 10.4°, 11.4°, 12.0°, 13.1°, 15.2°, and 15.7° (±0.2°)

In an aspect, the non-solvate crystal of the compound of the formula (2) is a 3C-type crystal comprising at least three of the following peaks as diffraction angles 2θ in powder X-ray diffraction.
7.9°, 8.5°, 8.8°, 9.2°, 10.4°, 11.4°, 12.0°, 13.1°, 15.2°, and 15.7° (±0.2°)

In an aspect, the non-solvate crystal of the compound of the formula (2) is a 3C-type crystal comprising at least five of the following peaks as diffraction angles 2θ in powder X-ray diffraction.
7.9°, 8.5°, 8.8°, 9.2°, 10.4°, 11.4°, 12.0°, 13.1°, 15.2°, and 15.7° (±0.2°)

In an aspect, the non-solvate crystal of the compound of the formula (2) is a 3C-type crystal comprising at least seven of the following peaks as diffraction angles 2θ in powder X-ray diffraction.
7.9°, 8.5°, 8.8°, 9.2°, 10.4°, 11.4°, 12.0°, 13.1°, 15.2°, and 15.7° (±0.2°)

In an aspect, the non-solvate crystal of the compound of the formula (2) is a 3C-type crystal comprising the following peaks as diffraction angles 2θ in powder X-ray diffraction. 7.9°, 8.5°, 8.8°, 9.2°, 10.4°, 11.4°, 12.0°, 13.1°, 15.2°, and 15.7° (±0.2°)

In an aspect, the crystal of the compound of the formula (2) is a 3D-type crystal comprising at least one of the following peaks as diffraction angles 2θ in powder X-ray diffraction.
6.6°, 7.3°, 10.9°, 11.8°, 13.9°, 17.1°, 17.5°, 18.3°, 19.3°, and 22.0° (±0.2°)

In an aspect, the crystal of the compound of the formula (2) is a 3D-type crystal comprising at least three of the following peaks as diffraction angles 2θ in powder X-ray diffraction.
6.6°, 7.3°, 10.9°, 11.8°, 13.9°, 17.1°, 17.5°, 18.3°, 19.3°, and 22.0° (±0.2°)

In an aspect, the crystal of the compound of the formula (2) is a 3D-type crystal comprising at least five of the following peaks as diffraction angles 2θ in powder X-ray diffraction.
6.6°, 7.3°, 10.9°, 11.8°, 13.9°, 17.1°, 17.5°, 18.3°, 19.3°, and 22.0° (±0.2°)

In an aspect, the crystal of the compound of the formula (2) is a 3D-type crystal comprising at least seven of the following peaks as diffraction angles 2θ in powder X-ray diffraction.
6.6°, 7.3°, 10.9°, 11.8°, 13.9°, 17.1°, 17.5°, 18.3°, 19.3°, and 22.0° (±0.2°)

In an aspect, the crystal of the compound of the formula (2) is a 3D-type crystal comprising the following peaks as diffraction angles 2θ in powder X-ray diffraction.
6.6°, 7.3°, 10.9°, 11.8°, 13.9°, 17.1°, 17.5°, 18.3°, 19.3°, and 22.0° (±0.2°)

In an aspect, the crystal of the compound of the formula (2) is a 3B-type crystal comprising at least one of the following peaks as diffraction angles 2θ in powder X-ray diffraction.
7.4°, 14.0°, 14.6°, 15.5°, 17.1°, 18.1°, 19.8°, 21.8°, 22.4°, and 24.1° (±0.2°)

In an aspect, the crystal of the compound of the formula (2) is a 3B-type crystal comprising at least three of the following peaks as diffraction angles 2θ in powder X-ray diffraction.
7.4°, 14.0°, 14.6°, 15.5°, 17.1°, 18.1°, 19.8°, 21.8°, 22.4°, and 24.1° (±0.2°)

In an aspect, the crystal of the compound of the formula (2) is a 3B-type crystal comprising at least five of the following peaks as diffraction angles 2θ in powder X-ray diffraction.
7.4°, 14.0°, 14.6°, 15.5°, 17.1°, 18.1°, 19.8°, 21.8°, 22.4°, and 24.1° (±0.2°)

In an aspect, the crystal of the compound of the formula (2) is a 3B-type crystal comprising at least seven of the following peaks as diffraction angles 2θ in powder X-ray diffraction.
7.4°, 14.0°, 14.6°, 15.5°, 17.1°, 18.1°, 19.8°, 21.8°, 22.4°, and 24.1° (±0.2°)

In an aspect, the crystal of the compound of the formula (2) is a 3B-type crystal comprising the following peaks as diffraction angles 2θ in powder X-ray diffraction.
7.4°, 14.0°, 14.6°, 15.5°, 17.1°, 18.1°, 19.8°, 21.8°, 22.4°, and 24.1° (±0.2°)

In an aspect, the non-solvate crystal of the compound of the formula (3) is a 2B-type crystal comprising at least one of the following peaks as diffraction angles 2θ in powder X-ray diffraction.
7.2°, 7.6°, 13.4°, 14.5°, 16.0°, 16.6°, 17.3°, 19.9°, 20.6°, and 22.4° (±0.2°)

In an aspect, the non-solvate crystal of the compound of the formula (3) is a 2B-type crystal comprising at least three of the following peaks as diffraction angles 2θ in powder X-ray diffraction.
7.2°, 7.6°, 13.4°, 14.5°, 16.0°, 16.6°, 17.3°, 19.9°, 20.6°, and 22.4° (±0.2°)

In an aspect, the non-solvate crystal of the compound of the formula (3) is a 2B-type crystal comprising at least five of the following peaks as diffraction angles 2θ in powder X-ray diffraction.
7.2°, 7.6°, 13.4°, 14.5°, 16.0°, 16.6°, 17.3°, 19.9°, 20.6°, and 22.4° (±0.2°)

In an aspect, the non-solvate crystal of the compound of the formula (3) is a 2B-type crystal comprising at least seven of the following peaks as diffraction angles 2θ in powder X-ray diffraction.
7.2°, 7.6°, 13.4°, 14.5°, 16.0°, 16.6°, 17.3°, 19.9°, 20.6°, and 22.4° (±0.2°)

In an aspect, the non-solvate crystal of the compound of the formula (3) is a 2B-type crystal comprising the following peaks as diffraction angles 2θ in powder X-ray diffraction.
7.2°, 7.6°, 13.4°, 14.5°, 16.0°, 16.6°, 17.3°, 19.9°, 20.6°, and 22.4° (±0.2°)

In an aspect, the non-solvate crystal of the compound of the formula (3) is a 2A-type crystal comprising at least one of the following peaks as diffraction angles 20 in powder X-ray diffraction.
7.5°, 10.0°, 10.7°, 13.7°, 14.6°, 15.1°, 16.8°, 17.4°, 18.0°, and 18.5° (±0.2°)

In an aspect, the non-solvate crystal of the compound of the formula (3) is a 2A-type crystal comprising at least three of the following peaks as diffraction angles 2θ in powder X-ray diffraction.
7.5°, 10.0°, 10.7°, 13.7°, 14.6°, 15.1°, 16.8°, 17.4°, 18.0°, and 18.5° (±0.2°)

In an aspect, the non-solvate crystal of the compound of the formula (3) is a 2A-type crystal comprising at least five of the following peaks as diffraction angles 2θ in powder X-ray diffraction.
7.5°, 10.0°, 10.7°, 13.7°, 14.6°, 15.1°, 16.8°, 17.4°, 18.0°, and 18.5° (±0.2°)

In an aspect, the non-solvate crystal of the compound of the formula (3) is a 2A-type crystal comprising at least seven of the following peaks as diffraction angles 2θ in powder X-ray diffraction.
7.5°, 10.0°, 10.7°, 13.7°, 14.6°, 15.1°, 16.8°, 17.4°, 18.0°, and 18.5° (±0.2°)

In an aspect, the non-solvate crystal of the compound of the formula (3) is a 2A-type crystal comprising the following peaks as diffraction angles 2θ in powder X-ray diffraction.
7.5°, 10.0°, 10.7°, 13.7°, 14.6°, 15.1°, 16.8°, 17.4°, 18.0°, and 18.5° (±0.2°)

In an aspect, the non-solvate crystal of the compound of the formula (3) is a 2C-type crystal comprising at least one of the following peaks as diffraction angles 2θ in powder X-ray diffraction.
7.2°, 9.9°, 14.5°, 15.3°, 15.7°, 17.2°, 17.6°, 18.0°, 19.1°, and 19.8° (±0.2°)

In an aspect, the non-solvate crystal of the compound of the formula (3) is a 2C-type crystal comprising at least three of the following peaks as diffraction angles 2θ in powder X-ray diffraction.
7.2°, 9.9°, 14.5°, 15.3°, 15.7°, 17.2°, 17.6°, 18.0°, 19.1°, and 19.8° (±0.2°)

In an aspect, the non-solvate crystal of the compound of the formula (3) is a 2C-type crystal comprising at least five of the following peaks as diffraction angles 2θ in powder X-ray diffraction.
7.2°, 9.9°, 14.5°, 15.3°, 15.7°, 17.2°, 17.6°, 18.0°, 19.1°, and 19.8° (±0.2°)

In an aspect, the non-solvate crystal of the compound of the formula (3) is a 2C-type crystal comprising at least seven of the following peaks as diffraction angles 2θ in powder X-ray diffraction.
7.2°, 9.9°, 14.5°, 15.3°, 15.7°, 17.2°, 17.6°, 18.0°, 19.1°, and 19.8° (±0.2°)

In an aspect, the non-solvate crystal of the compound of the formula (3) is a 2C-type crystal comprising the following peaks as diffraction angles 2θ in powder X-ray diffraction.
7.2°, 9.9°, 14.5°, 15.3°, 15.7°, 17.2°, 17.6°, 18.0°, 19.1°, and 19.8° (±0.2°)

In an aspect, the non-solvate crystal of the compound of the formula (3) is a 2F-type crystal comprising at least one of the following peaks as diffraction angles 2θ in powder X-ray diffraction.
7.3°, 9.9°, 11.4°, 14.5°, 15.3°, 16.4°, 16.8°, 17.2°, 17.7°, and 18.1° (±0.2°)

In an aspect, the non-solvate crystal of the compound of the formula (3) is a 2F-type crystal comprising at least three of the following peaks as diffraction angles 2θ in powder X-ray diffraction.
7.3°, 9.9°, 11.4°, 14.5°, 15.3°, 16.4°, 16.8°, 17.2°, 17.7°, and 18.1° (±0.2°)

In an aspect, the non-solvate crystal of the compound of the formula (3) is a 2F-type crystal comprising at least five of the following peaks as diffraction angles 2θ in powder X-ray diffraction.
7.3°, 9.9°, 11.4°, 14.5°, 15.3°, 16.4°, 16.8°, 17.2°, 17.7°, and 18.1° (±0.2°)

In an aspect, the non-solvate crystal of the compound of the formula (3) is a 2F-type crystal comprising at least seven of the following peaks as diffraction angles 2θ in powder X-ray diffraction.
7.3°, 9.9°, 11.4°, 14.5°, 15.3°, 16.4°, 16.8°, 17.2°, 17.7°, and 18.1° (±0.2°)

In an aspect, the non-solvate crystal of the compound of the formula (3) is a 2F-type crystal comprising the following peaks as diffraction angles 2θ in powder X-ray diffraction.
7.3°, 9.9°, 11.4°, 14.5°, 15.3°, 16.4°, 16.8°, 17.2°, 17.7°, and 18.1° (±0.2°)

In an aspect, the hydrate crystal of the compound of the formula (4) is a 5A-type crystal comprising at least one of the following peaks as diffraction angles 2θ in powder X-ray diffraction.
7.4°, 8.8°, 9.5°, 10.4°, 12.2°, 15.5°, 16.6°, 17.1°, 17.5°, and 18.1° (±0.2°)

In an aspect, the hydrate crystal of the compound of the formula (4) is a 5A-type crystal comprising at least three of the following peaks as diffraction angles 2θ in powder X-ray diffraction.
7.4°, 8.8°, 9.5°, 10.4°, 12.2°, 15.5°, 16.6°, 17.1°, 17.5°, and 18.1° (±0.2°)

In an aspect, the hydrate crystal of the compound of the formula (4) is a 5A-type crystal comprising at least five of the following peaks as diffraction angles 2θ in powder X-ray diffraction.
7.4°, 8.8°, 9.5°, 10.4°, 12.2°, 15.5°, 16.6°, 17.1°, 17.5°, and 18.1° (±0.2°)

In an aspect, the hydrate crystal of the compound of the formula (4) is a 5A-type crystal comprising at least seven of the following peaks as diffraction angles 2θ in powder X-ray diffraction.
7.4°, 8.8°, 9.5°, 10.4°, 12.2°, 15.5°, 16.6°, 17.1°, 17.5°, and 18.1° (±0.2°)

In an aspect, the hydrate crystal of the compound of the formula (4) is a 5A-type crystal comprising the following peaks as diffraction angles 2θ in powder X-ray diffraction.
7.4°, 8.8°, 9.5°, 10.4°, 12.2°, 15.5°, 16.6°, 17.1°, 17.5°, and 18.1° (±0.2°)

In an aspect, the hydrate crystal of the compound of the formula (4) is a 5A-type crystal comprising the following peaks as diffraction angles 2θ in powder X-ray diffraction.
7.4°, 8.8°, and 9.5° (±0.2°)

In an aspect, the hydrate crystal of the compound of the formula (4) is a 5A-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 10% for 1 hour.
7.4°, 8.8°, 9.5°, 10.8°, 12.7°, 16.0°, 16.8°, 17.3°, 17.9°, and 19.2° (±0.2°)

In an aspect, the hydrate crystal of the compound of the formula (4) is a 5A-type crystal comprising at least three of the following peaks as diffraction angles 2θ in powder X-ray diffraction. The following diffraction angles (2θ values) may be diffraction angles (2θ values) of the hydrate crystal preserved at a temperature of 25°C and a relative humidity of 10% for 1 hour.
7.4°, 8.8°, 9.5°, 10.8°, 12.7°, 16.0°, 16.8°, 17.3°, 17.9°, and 19.2° (±0.2°)

In an aspect, the hydrate crystal of the compound of the formula (4) is a 5A-type crystal comprising at least five of the following peaks as diffraction angles 2θ in powder X-ray diffraction. The following diffraction angles (2θ values) may be diffraction angles (2θ values) of the hydrate crystal preserved at a temperature of 25°C and a relative humidity of 10% for 1 hour.
7.4°, 8.8°, 9.5°, 10.8°, 12.7°, 16.0°, 16.8°, 17.3°, 17.9°, and 19.2° (±0.2°)

In an aspect, the hydrate crystal of the compound of the formula (4) is a 5A-type crystal comprising at least seven of the following peaks as diffraction angles 2θ in powder X-ray diffraction. The following diffraction angles (2θ values) may be diffraction angles (2θ values) of the hydrate crystal preserved at a temperature of 25°C and a relative humidity of 10% for 1 hour.
7.4°, 8.8°, 9.5°, 10.8°, 12.7°, 16.0°, 16.8°, 17.3°, 17.9°, and 19.2° (±0.2°)

In an aspect, the hydrate crystal of the compound of the formula (4) is a 5A-type crystal comprising the following peaks as diffraction angles 2θ in powder X-ray diffraction. The following diffraction angles (2θ values) may be diffraction angles (2θ values) of the hydrate crystal preserved at a temperature of 25°C and a relative humidity of 10% for 1 hour.
7.4°, 8.8°, 9.5°, 10.8°, 12.7°, 16.0°, 16.8°, 17.3°, 17.9°, and 19.2° (±0.2°)

In an aspect, the hydrate crystal of the compound of the formula (4) is a 5A-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 20% for 1 hour.
7.4°, 8.8°, 9.5°, 10.5°, 12.2°, 15.6°, 16.6°, 17.1°, 17.3°, and 17.5° (±0.2°)

In an aspect, the hydrate crystal of the compound of the formula (4) is a 5A-type crystal comprising at least three of the following peaks as diffraction angles 2θ in powder X-ray diffraction. The following diffraction angles (2θ values) may be diffraction angles (2θ values) of the hydrate crystal preserved at a temperature of 25°C and a relative humidity of 20% for 1 hour.
7.4°, 8.8°, 9.5°, 10.5°, 12.2°, 15.6°, 16.6°, 17.1°, 17.3°, and 17.5° (±0.2°)

In an aspect, the hydrate crystal of the compound of the formula (4) is a 5A-type crystal comprising at least five of the following peaks as diffraction angles 2θ in powder X-ray diffraction. The following diffraction angles (2θ values) may be diffraction angles (2θ values) of the hydrate crystal preserved at a temperature of 25°C and a relative humidity of 20% for 1 hour.
7.4°, 8.8°, 9.5°, 10.5°, 12.2°, 15.6°, 16.6°, 17.1°, 17.3°, and 17.5° (±0.2°)

In an aspect, the hydrate crystal of the compound of the formula (4) is a 5A-type crystal comprising at least seven of the following peaks as diffraction angles 2θ in powder X-ray diffraction. The following diffraction angles (2θ values) may be diffraction angles (2θ values) of the hydrate crystal preserved at a temperature of 25°C and a relative humidity of 20% for 1 hour.
7.4°, 8.8°, 9.5°, 10.5°, 12.2°, 15.6°, 16.6°, 17.1°, 17.3°, and 17.5° (±0.2°)

In an aspect, the hydrate crystal of the compound of the formula (4) is a 5A-type crystal comprising the following peaks as diffraction angles 2θ in powder X-ray diffraction. The following diffraction angles (2θ values) may be diffraction angles (2θ values) of the hydrate crystal preserved at a temperature of 25°C and a relative humidity of 20% for 1 hour.
7.4°, 8.8°, 9.5°, 10.5°, 12.2°, 15.6°, 16.6°, 17.1°, 17.3°, and 17.5° (±0.2°)

In an aspect, the hydrate crystal of the compound of the formula (4) is a 5A-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.4°, 8.8°, 9.5°, 10.5°, 12.2°, 15.5°, 16.4°, 16.6°, 17.1°, and 17.6° (±0.2°)

In an aspect, the hydrate crystal of the compound of the formula (4) is a 5A-type crystal comprising at least three of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.4°, 8.8°, 9.5°, 10.5°, 12.2°, 15.5°, 16.4°, 16.6°, 17.1°, and 17.6° (±0.2°)

In an aspect, the hydrate crystal of the compound of the formula (4) is a 5A-type crystal comprising at least five of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.4°, 8.8°, 9.5°, 10.5°, 12.2°, 15.5°, 16.4°, 16.6°, 17.1°, and 17.6° (±0.2°)

In an aspect, the hydrate crystal of the compound of the formula (4) is a 5A-type crystal comprising at least seven of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.4°, 8.8°, 9.5°, 10.5°, 12.2°, 15.5°, 16.4°, 16.6°, 17.1°, and 17.6° (±0.2°)

In an aspect, the hydrate crystal of the compound of the formula (4) is a 5A-type crystal comprising the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.4°, 8.8°, 9.5°, 10.5°, 12.2°, 15.5°, 16.4°, 16.6°, 17.1°, and 17.6° (±0.2°)

In an aspect, the hydrate crystal of the compound of the formula (4) is a 5A-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 60% for 1 hour.
7.4°, 8.8°, 9.5°, 10.3°, 12.2°, 15.4°, 16.3°, 16.6°, 17.0°, and 17.6° (±0.2°)

In an aspect, the hydrate crystal of the compound of the formula (4) is a 5A-type crystal comprising at least three of the following peaks as diffraction angles 2θ in powder X-ray diffraction. The following diffraction angles (2θ values) may be diffraction angles (2θ values) of the hydrate crystal preserved at a temperature of 25°C and a relative humidity of 60% for 1 hour.
7.4°, 8.8°, 9.5°, 10.3°, 12.2°, 15.4°, 16.3°, 16.6°, 17.0°, and 17.6° (±0.2°)

In an aspect, the hydrate crystal of the compound of the formula (4) is a 5A-type crystal comprising at least five of the following peaks as diffraction angles 2θ in powder X-ray diffraction. The following diffraction angles (2θ values) may be diffraction angles (2θ values) of the hydrate crystal preserved at a temperature of 25°C and a relative humidity of 60% for 1 hour.
7.4°, 8.8°, 9.5°, 10.3°, 12.2°, 15.4°, 16.3°, 16.6°, 17.0°, and 17.6° (±0.2°)

In an aspect, the hydrate crystal of the compound of the formula (4) is a 5A-type crystal comprising at least seven of the following peaks as diffraction angles 2θ in powder X-ray diffraction. The following diffraction angles (2θ values) may be diffraction angles (2θ values) of the hydrate crystal preserved at a temperature of 25°C and a relative humidity of 60% for 1 hour.
7.4°, 8.8°, 9.5°, 10.3°, 12.2°, 15.4°, 16.3°, 16.6°, 17.0°, and 17.6° (±0.2°)

In an aspect, the hydrate crystal of the compound of the formula (4) is a 5A-type crystal comprising the following peaks as diffraction angles 2θ in powder X-ray diffraction. The following diffraction angles (2θ values) may be diffraction angles (2θ values) of the hydrate crystal preserved at a temperature of 25°C and a relative humidity of 60% for 1 hour.
7.4°, 8.8°, 9.5°, 10.3°, 12.2°, 15.4°, 16.3°, 16.6°, 17.0°, and 17.6° (±0.2°)

In an aspect, the hydrate crystal of the compound of the formula (4) is a 5E-type crystal comprising at least one of the following peaks as diffraction angles 2θ in powder X-ray diffraction.
9.2°, 10.6°, 11.9°, 13.7°, 15.0°, 15.8°, 16.6°, 17.1°, 18.5°, and 19.2° (±0.2°)

In an aspect, the hydrate crystal of the compound of the formula (4) is a 5E-type crystal comprising at least three of the following peaks as diffraction angles 2θ in powder X-ray diffraction.
9.2°, 10.6°, 11.9°, 13.7°, 15.0°, 15.8°, 16.6°, 17.1°, 18.5°, and 19.2° (±0.2°)

In an aspect, the hydrate crystal of the compound of the formula (4) is a 5E-type crystal comprising at least five of the following peaks as diffraction angles 2θ in powder X-ray diffraction.
9.2°, 10.6°, 11.9°, 13.7°, 15.0°, 15.8°, 16.6°, 17.1°, 18.5°, and 19.2° (±0.2°)

In an aspect, the hydrate crystal of the compound of the formula (4) is a 5E-type crystal comprising at least seven of the following peaks as diffraction angles 2θ in powder X-ray diffraction.
9.2°, 10.6°, 11.9°, 13.7°, 15.0°, 15.8°, 16.6°, 17.1°, 18.5°, and 19.2° (±0.2°)

In an aspect, the hydrate crystal of the compound of the formula (4) is a 5E-type crystal comprising the following peaks as diffraction angles 2θ in powder X-ray diffraction.
9.2°, 10.6°, 11.9°, 13.7°, 15.0°, 15.8°, 16.6°, 17.1°, 18.5°, and 19.2° (±0.2°)

In an aspect, the hydrate crystal of the compound of the formula (4) is a 5J-type crystal comprising at least one of the following peaks as diffraction angles 2θ in powder X-ray diffraction.
7.3°, 9.0°, 9.6°, 10.8°, 12.2°, 15.7°, 16.6°, and 17.3° (±0.2°)

In an aspect, the hydrate crystal of the compound of the formula (4) is a 5J-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.3°, 9.0°, 9.6°, 10.9°, 12.3°, 15.9°, 16.6°, 17.1°, and 17.5° (±0.2°)

In an aspect, the hydrate crystal of the compound of the formula (4) is a 5J-type crystal comprising at least three of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.3°, 9.0°, 9.6°, 10.9°, 12.3°, 15.9°, 16.6°, 17.1°, and 17.5° (±0.2°)

In an aspect, the hydrate crystal of the compound of the formula (4) is a 5J-type crystal comprising at least five of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.3°, 9.0°, 9.6°, 10.9°, 12.3°, 15.9°, 16.6°, 17.1°, and 17.5° (±0.2°)

In an aspect, the hydrate crystal of the compound of the formula (4) is a 5J-type crystal comprising at least seven of the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.3°, 9.0°, 9.6°, 10.9°, 12.3°, 15.9°, 16.6°, 17.1°, and 17.5° (±0.2°)

In an aspect, the hydrate crystal of the compound of the formula (4) is a 5J-type crystal comprising the following peaks as diffraction angles 2θ in powder X-ray diffraction. The following diffraction angles (2θ values) may be diffraction angles (2θ values) of the hydrate crystal preserved at a temperature of 25°C and a relative humidity of 30% for 1 hour.
7.3°, 9.0°, 9.6°, 10.9°, 12.3°, 15.9°, 16.6°, 17.1°, and 17.5° (±0.2°)

In an aspect, the hydrate crystal of the compound of the formula (4) is a 5J-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 40% for 1 hour.
7.3°, 8.9°, 9.6°, 10.8°, 12.2°, 15.7°, 16.3°, 16.6°, 17.0°, and 17.3° (±0.2°)

In an aspect, the hydrate crystal of the compound of the formula (4) is a 5J-type crystal comprising at least three of the following peaks as diffraction angles 2θ in powder X-ray diffraction. The following diffraction angles (2θ values) are preferably diffraction angles (2θ values) of the hydrate crystal preserved at a temperature of 25°C and a relative humidity of 40% for 1 hour.
7.3°, 8.9°, 9.6°, 10.8°, 12.2°, 15.7°, 16.3°, 16.6°, 17.0°, and 17.3° (±0.2°)

In an aspect, the hydrate crystal of the compound of the formula (4) is a 5J-type crystal comprising at least five of the following peaks as diffraction angles 2θ in powder X-ray diffraction. The following diffraction angles (2θ values) are preferably diffraction angles (2θ values) of the hydrate crystal preserved at a temperature of 25°C and a relative humidity of 40% for 1 hour.
7.3°, 8.9°, 9.6°, 10.8°, 12.2°, 15.7°, 16.3°, 16.6°, 17.0°, and 17.3° (±0.2°)

In an aspect, the hydrate crystal of the compound of the formula (4) is a 5J-type crystal comprising at least seven of the following peaks as diffraction angles 2θ in powder X-ray diffraction. The following diffraction angles (2θ values) are preferably diffraction angles (2θ values) of the hydrate crystal preserved at a temperature of 25°C and a relative humidity of 40% for 1 hour.
7.3°, 8.9°, 9.6°, 10.8°, 12.2°, 15.7°, 16.3°, 16.6°, 17.0°, and 17.3° (±0.2°)

In an aspect, the hydrate crystal of the compound of the formula (4) is a 5J-type crystal comprising the following peaks as diffraction angles 2θ in powder X-ray diffraction. The following diffraction angles (2θ values) are preferably diffraction angles (2θ values) of the hydrate crystal preserved at a temperature of 25°C and a relative humidity of 40% for 1 hour.
7.3°, 8.9°, 9.6°, 10.8°, 12.2°, 15.7°, 16.3°, 16.6°, 17.0°, and 17.3° (±0.2°)

In an aspect, the hydrate crystal of the compound of the formula (4) is a 5J-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 50 to 60% for 1 hour.
7.3°, 8.9°, 9.6°, 10.7°, 12.1°, 15.6°, 16.2°, 16.6°, 16.9°, and 17.2° (±0.2°)

In an aspect, the hydrate crystal of the compound of the formula (4) is a 5J-type crystal comprising at least three of the following peaks as diffraction angles 2θ in powder X-ray diffraction. The following diffraction angles (2θ values) may be diffraction angles (2θ values) of the hydrate crystal preserved at a temperature of 25°C and a relative humidity of 50 to 60% for 1 hour.
7.3°, 8.9°, 9.6°, 10.7°, 12.1°, 15.6°, 16.2°, 16.6°, 16.9°, and 17.2° (±0.2°)

In an aspect, the hydrate crystal of the compound of the formula (4) is a 5J-type crystal comprising at least five of the following peaks as diffraction angles 2θ in powder X-ray diffraction. The following diffraction angles (2θ values) may be diffraction angles (2θ values) of the hydrate crystal preserved at a temperature of 25°C and a relative humidity of 50 to 60% for 1 hour.
7.3°, 8.9°, 9.6°, 10.7°, 12.1°, 15.6°, 16.2°, 16.6°, 16.9°, and 17.2° (±0.2°)

In an aspect, the hydrate crystal of the compound of the formula (4) is a 5J-type crystal comprising at least seven of the following peaks as diffraction angles 2θ in powder X-ray diffraction. The following diffraction angles (2θ values) may be diffraction angles (2θ values) of the hydrate crystal preserved at a temperature of 25°C and a relative humidity of 50 to 60% for 1 hour.
7.3°, 8.9°, 9.6°, 10.7°, 12.1°, 15.6°, 16.2°, 16.6°, 16.9°, and 17.2° (±0.2°)

In an aspect, the hydrate crystal of the compound of the formula (4) is a 5J-type crystal comprising the following peaks as diffraction angles 2θ in powder X-ray diffraction. The following diffraction angles (2θ values) may be diffraction angles (2θ values) of the hydrate crystal preserved at a temperature of 25°C and a relative humidity of 50 to 60% for 1 hour.
7.3°, 8.9°, 9.6°, 10.7°, 12.1°, 15.6°, 16.2°, 16.6°, 16.9°, and 17.2° (±0.2°)

In an aspect, the hydrate crystal of the compound of the formula (4) is a 5J-type crystal comprising at least one of the following peaks as diffraction angles 2θ in powder X-ray diffraction. The following diffraction angles (2θ values) may be diffraction angles (2θ values) of the hydrate crystal preserved at a temperature of 25°C and a relative humidity of 70% or higher for 1 hour.
7.2°, 8.9°, 9.5°, 10.6°, 12.0°, 15.5°, 16.1°, 16.6°, and 17.1° (±0.2°)

In an aspect, the hydrate crystal of the compound of the formula (4) is a 5J-type crystal comprising at least three of the following peaks as diffraction angles 2θ in powder X-ray diffraction. The following diffraction angles (2θ values) may be diffraction angles (2θ values) of the hydrate crystal preserved at a temperature of 25°C and a relative humidity of 70% or higher for 1 hour.
7.2°, 8.9°, 9.5°, 10.6°, 12.0°, 15.5°, 16.1°, 16.6°, and 17.1° (±0.2°)

In an aspect, the hydrate crystal of the compound of the formula (4) is a 5J-type crystal comprising at least five of the following peaks as diffraction angles 2θ in powder X-ray diffraction. The following diffraction angles (2θ values) may be diffraction angles (2θ values) of the hydrate crystal preserved at a temperature of 25°C and a relative humidity of 70% or higher for 1 hour.
7.2°, 8.9°, 9.5°, 10.6°, 12.0°, 15.5°, 16.1°, 16.6°, and 17.1° (±0.2°)

In an aspect, the hydrate crystal of the compound of the formula (4) is a 5J-type crystal comprising at least seven of the following peaks as diffraction angles 2θ in powder X-ray diffraction. The following diffraction angles (2θ values) may be diffraction angles (2θ values) of the hydrate crystal preserved at a temperature of 25°C and a relative humidity of 70% or higher for 1 hour.
7.2°, 8.9°, 9.5°, 10.6°, 12.0°, 15.5°, 16.1°, 16.6°, and 17.1° (±0.2°)

In an aspect, the hydrate crystal of the compound of the formula (4) is a 5J-type crystal comprising the following peaks as diffraction angles 2θ in powder X-ray diffraction. The following diffraction angles (2θ values) may be diffraction angles (2θ values) of the hydrate crystal preserved at a temperature of 25°C and a relative humidity of 70% or higher for 1 hour.
7.2°, 8.9°, 9.5°, 10.6°, 12.0°, 15.5°, 16.1°, 16.6°, and 17.1° (±0.2°)

In an aspect, the crystal of the compound of the formula (4) is a 5C-type crystal comprising at least one of the following peaks as diffraction angles 2θ in powder X-ray diffraction.
7.3°, 9.7°, 12.1°, 14.8°, 15.2°, 15.7°, 16.6°, and 17.7° (±0.2°)

In an aspect, the crystal of the compound of the formula (4) is a 5C-type crystal comprising at least three of the following peaks as diffraction angles 2θ in powder X-ray diffraction.
7.3°, 9.7°, 12.1°, 14.8°, 15.2°, 15.7°, 16.6°, and 17.7° (±0.2°)

In an aspect, the crystal of the compound of the formula (4) is a 5C-type crystal comprising at least five of the following peaks as diffraction angles 2θ in powder X-ray diffraction.
7.3°, 9.7°, 12.1°, 14.8°, 15.2°, 15.7°, 16.6°, and 17.7° (±0.2°)

In an aspect, the crystal of the compound of the formula (4) is a 5C-type crystal comprising at least seven of the following peaks as diffraction angles 2θ in powder X-ray diffraction.
7.3°, 9.7°, 12.1°, 14.8°, 15.2°, 15.7°, 16.6°, and 17.7° (±0.2°)

In an aspect, the crystal of the compound of the formula (4) is a 5C-type crystal comprising the following peaks as diffraction angles 2θ in powder X-ray diffraction.
7.3°, 9.7°, 12.1°, 14.8°, 15.2°, 15.7°, 16.6°, and 17.7° (±0.2°)

In an aspect, the non-solvate crystal of the compound of the formula (4) is a 5D-type crystal comprising at least one of the following peaks as diffraction angles 2θ in powder X-ray diffraction.
11.3°, 13.1°, 13.8°, 14.9°, 15.9°, 16.1°, 16.7°, 17.5°, 18.1°, and 18.5° (±0.2°)

In an aspect, the non-solvate crystal of the compound of the formula (4) is a 5D-type crystal comprising at least three of the following peaks as diffraction angles 2θ in powder X-ray diffraction.
11.3°, 13.1°, 13.8°, 14.9°, 15.9°, 16.1°, 16.7°, 17.5°, 18.1°, and 18.5° (±0.2°)

In an aspect, the non-solvate crystal of the compound of the formula (4) is a 5D-type crystal comprising at least five of the following peaks as diffraction angles 2θ in powder X-ray diffraction.
11.3°, 13.1°, 13.8°, 14.9°, 15.9°, 16.1°, 16.7°, 17.5°, 18.1°, and 18.5° (±0.2°)

In an aspect, the non-solvate crystal of the compound of the formula (4) is a 5D-type crystal comprising at least seven of the following peaks as diffraction angles 2θ in powder X-ray diffraction.
11.3°, 13.1°, 13.8°, 14.9°, 15.9°, 16.1°, 16.7°, 17.5°, 18.1°, and 18.5° (±0.2°)

In an aspect, the non-solvate crystal of the compound of the formula (4) is a 5D-type crystal comprising the following peaks as diffraction angles 2θ in powder X-ray diffraction.
11.3°, 13.1°, 13.8°, 14.9°, 15.9°, 16.1°, 16.7°, 17.5°, 18.1°, and 18.5° (±0.2°)

In an aspect, the crystal of the compound of the formula (4) is a 5G-type crystal comprising at least one of the following peaks as diffraction angles 2θ in powder X-ray diffraction.
9.4°, 11.0°, 13.4°, 15.0°, 16.1°, 17.8°, 18.1°, 19.7°, 20.8°, and 22.0° (±0.2°)

In an aspect, the crystal of the compound of the formula (4) is a 5G-type crystal comprising at least three of the following peaks as diffraction angles 2θ in powder X-ray diffraction.
9.4°, 11.0°, 13.4°, 15.0°, 16.1°, 17.8°, 18.1°, 19.7°, 20.8°, and 22.0° (±0.2°)

In an aspect, the crystal of the compound of the formula (4) is a 5G-type crystal comprising at least five of the following peaks as diffraction angles 2θ in powder X-ray diffraction.
9.4°, 11.0°, 13.4°, 15.0°, 16.1°, 17.8°, 18.1°, 19.7°, 20.8°, and 22.0° (±0.2°)

In an aspect, the crystal of the compound of the formula (4) is a 5G-type crystal comprising at least seven of the following peaks as diffraction angles 2θ in powder X-ray diffraction.
9.4°, 11.0°, 13.4°, 15.0°, 16.1°, 17.8°, 18.1°, 19.7°, 20.8°, and 22.0° (±0.2°)

In an aspect, the crystal of the compound of the formula (4) is a 5G-type crystal comprising the following peaks as diffraction angles 2θ in powder X-ray diffraction.
9.4°, 11.0°, 13.4°, 15.0°, 16.1°, 17.8°, 18.1°, 19.7°, 20.8°, and 22.0° (±0.2°)

In an aspect, the hydrate crystal of the compound of the formula (5) is a 4B-type crystal comprising at least one of the following peaks as diffraction angles 2θ in powder X-ray diffraction.
7.7°, 13.3°, 14.0°, 15.4°, 16.2°, 17.9°, 18.2°, 18.5°, 19.1°, and 20.6° (±0.2°)

In an aspect, the hydrate crystal of the compound of the formula (5) is a 4B-type crystal comprising at least three of the following peaks as diffraction angles 2θ in powder X-ray diffraction.
7.7°, 13.3°, 14.0°, 15.4°, 16.2°, 17.9°, 18.2°, 18.5°, 19.1°, and 20.6° (±0.2°)

In an aspect, the hydrate crystal of the compound of the formula (5) is a 4B-type crystal comprising at least five of the following peaks as diffraction angles 2θ in powder X-ray diffraction.
7.7°, 13.3°, 14.0°, 15.4°, 16.2°, 17.9°, 18.2°, 18.5°, 19.1°, and 20.6° (±0.2°)

In an aspect, the hydrate crystal of the compound of the formula (5) is a 4B-type crystal comprising at least seven of the following peaks as diffraction angles 2θ in powder X-ray diffraction.
7.7°, 13.3°, 14.0°, 15.4°, 16.2°, 17.9°, 18.2°, 18.5°, 19.1°, and 20.6° (±0.2°)

In an aspect, the hydrate crystal of the compound of the formula (5) is a 4B-type crystal comprising the following peaks as diffraction angles 2θ in powder X-ray diffraction.
7.7°, 13.3°, 14.0°, 15.4°, 16.2°, 17.9°, 18.2°, 18.5°, 19.1°, and 20.6° (±0.2°)

In an aspect, the crystal of the compound of the formula (5) is a 4A-type crystal comprising at least one of the following peaks as diffraction angles 2θ in powder X-ray diffraction.
11.5°, 11.8°, 15.7°, 17.2°, 17.5°, 18.1°, 19.5°, 20.3°, 20.5°, and 21.2° (±0.2°)

In an aspect, the crystal of the compound of the formula (5) is a 4A-type crystal comprising at least three of the following peaks as diffraction angles 2θ in powder X-ray diffraction.
11.5°, 11.8°, 15.7°, 17.2°, 17.5°, 18.1°, 19.5°, 20.3°, 20.5°, and 21.2° (±0.2°)

In an aspect, the crystal of the compound of the formula (5) is a 4A-type crystal comprising at least five of the following peaks as diffraction angles 2θ in powder X-ray diffraction.
11.5°, 11.8°, 15.7°, 17.2°, 17.5°, 18.1°, 19.5°, 20.3°, 20.5°, and 21.2° (±0.2°)

In an aspect, the crystal of the compound of the formula (5) is a 4A-type crystal comprising at least seven of the following peaks as diffraction angles 2θ in powder X-ray diffraction.
11.5°, 11.8°, 15.7°, 17.2°, 17.5°, 18.1°, 19.5°, 20.3°, 20.5°, and 21.2° (±0.2°)

In an aspect, the crystal of the compound of the formula (5) is a 4A-type crystal comprising the following peaks as diffraction angles 2θ in powder X-ray diffraction.
11.5°, 11.8°, 15.7°, 17.2°, 17.5°, 18.1°, 19.5°, 20.3°, 20.5°, and 21.2° (±0.2°)

In the present specification, the term "to" which indicates a numeric range includes values described at both ends thereof. For example, the term "A to B" means a numeric range of A or more and B or less.

In the present specification, the term "approximately", when used in combination with a numeric value, means a range of values of +10% and -10% of the numeric value.

In the present specification, the term "and/or" is meant to include every combination of the terms "and" and "or" appropriately combined. Specifically, for example, the term "A, B, and/or C" includes the following seven variations: (i) A, (ii) B, (iii) C, (iv) A and B, (v) A and C, (vi) B and C, and (vii) A, B, and C.

The compounds represented by the formulas (1) to (5) may be synthesized by various methods and may be synthesized by methods well known to those skilled in the art. The compounds represented by the formulas (1) to (5) can be synthesized by, for example, methods described in Example 1 mentioned later.

### <Production method>

The crystal of the compound of the present invention or a salt thereof, or a solvate thereof can be produced by, for example, the following method.

A solvent suitable for crystallization is added to the compound, then seed crystals are arbitrarily added thereto, and the mixture can be stirred, if necessary, to obtain crystals of the compound or a salt thereof, or a solvate thereof. The solvent to be added for crystallization is not particularly limited as long as the solvent enables the compound to form crystals. The solvent is preferably a solvent that permits an operation of reducing the solubility of the compound as to a solution containing the compound dissolved therein. When the crystals can be formed by reducing the solubility of the compound, for example, by the addition of a poor solvent or the cooling of a solution, examples thereof include solvents that permit such an operation. When the crystals of the compound can be obtained by keeping a suspended state for an arbitrary time under a suspension of crude crystals of the compound, a solvent that permits such an operation can be used in crystallization. Specific examples of the solvent to be added for crystallization include acetone, water, dimethyl sulfoxide (DMSO), dimethylformamide (DMF), acetonitrile, tetrahydrofuran, methyl ethyl ketone, methyl isobutyl ketone, ethyl acetate, heptane, polyethylene glycol (PEG), 2-propanol, methanol, or ethanol, and mixed solvents thereof.

### <Pharmaceutical composition>

The present invention provides a pharmaceutical composition containing the crystal of the compound of the present invention or a salt thereof, or a solvate thereof.

The pharmaceutical composition of the present invention may be formulated in accordance with a method known in the art by introducing a pharmaceutically acceptable carrier in addition to the crystal of the compound of the present invention or a salt thereof, or a solvate thereof. For the formulation, an excipient, a binder, a lubricant, a colorant, a corrigent, and optionally a stabilizer, an emulsifier, an absorption promoter, a surfactant, a pH adjuster, an antiseptic, an antioxidant, and the like which are usually used can be used. The formulation is performed in accordance with a routine method by blending components that are generally used as starting materials for pharmaceutical preparations.

For the formulation, the active ingredient for use in a medicament can be processed into the optimum shape or nature for a use method or a use purpose, i.e., a dosage form, by a method known in the art. Examples of the dosage form which is usually used include, but are not limited to, liquid pharmaceutical preparations (liquid agents) such as injections, suspensions, emulsions, and eye drops, and solid pharmaceutical preparations (solid preparations) such as tablets, powders, fine granules, granules, coated tablets, capsules, dry syrups, troches, and suppositories.

In order to produce, for example, a liquid agent, formulation is performed by appropriately adding pharmaceutically acceptable carriers or media, specifically, pharmaceutically acceptable additives which are usually used in the pharmaceutical preparation field such as sterile water or physiological saline, a plant oil, an emulsifier, a suspending agent, a surfactant, a stabilizer, a flavor, an excipient, a vehicle, an antiseptic, and a binder, in combination to the crystal of the compound of the present invention or a salt thereof, or a solvate thereof, followed by mixing in a generally accepted unit dosage form required for pharmaceutical practice. Alternatively, a solid preparation prepared for liquid agents may be dissolved, when necessary, by the addition of an appropriate solvent, for example, sterile water or physiological saline, before administration and then subjected to administration.

Such a liquid agent may be parenterally used, for example, in the form of an injection of an antiseptic solution or suspension with water or any of other pharmaceutically acceptable liquids. Formulation can be performed, for example, by appropriately combining with pharmaceutically acceptable carriers or media, specifically, sterile water or physiological saline, a plant oil, an emulsifier, a suspending agent, a surfactant, a stabilizer, a flavor, an excipient, a vehicle, an antiseptic, a binder, and the like, followed by mixing in a generally accepted unit dosage form required for pharmaceutical practice. Specifically, examples of the carrier can include light anhydrous silicic acid, lactose, crystalline cellulose, mannitol, starch, carmellose calcium, carmellose sodium, hydroxypropylcellulose, hydroxypropylmethylcellulose, polyvinylacetal diethylaminoacetate, polyvinylpyrrolidone, gelatin, medium-chain fatty acid triglyceride, polyoxyethylene hydrogenated castor oil 60, saccharose, carboxymethylcellulose, corn starch, and inorganic salts. The amount of the active ingredient in such a preparation is set so as to obtain an appropriate volume in a prescribed range.

An aseptic composition for injection can be formulated in accordance with usual pharmaceutical practice using a vehicle such as injectable distilled water.

Examples of the aqueous solution for injection include physiological saline and other isotonic liquids containing an adjuvant, for example, D-sorbitol, D-mannose, D-mannitol, and sodium chloride, which may be used in combination with an appropriate solubilizer (e.g., an alcohol, specifically ethanol, and a polyalcohol, for example, propylene glycol and polyethylene glycol) and/or a nonionic surfactant (e.g., polysorbate 80(R) and HCO-50).

Examples of the oily liquid include sesame oil and soybean oil, which may be used in combination with benzyl benzoate or benzyl alcohol as a solubilizer. Such a liquid may be blended with a buffer (e.g., phosphate buffer solutions and sodium acetate buffer solutions), a soothing agent (e.g., procaine hydrochloride), a stabilizer (e.g., benzyl alcohol and phenol), and an antioxidant. An appropriate ampule is usually packed with the prepared injection.

In order to produce, for example, a solid preparation, an excipient and optionally pharmaceutically acceptable additives which are usually used in the pharmaceutical preparation field such as a binder, a disintegrant, a lubricant, a colorant, and a corrigent are appropriately added in combination to the crystal of the compound of the present invention or a salt thereof, or a solvate thereof, and then prepared into tablets, powders, fine granules, granules, coated tablets, capsules, dry syrups, troches, suppositories, or the like by a routine method.

Examples of the pharmaceutically acceptable additive for use in such solid preparations include: animal or plant oils such as soybean oil, beef tallow, and synthetic glyceride; hydrocarbon such as liquid paraffin, squalane, and solid paraffin; ester oils such as octyl dodecyl myristate and isopropyl myristate; higher alcohols such as cetostearyl alcohol and behenyl alcohol; silicone resins ; silicone oils; surfactants such as polyoxyethylene fatty acid ester, sorbitan fatty acid ester, glycerin fatty acid ester, polyoxyethylene sorbitan fatty acid ester, polyoxyethylene hydrogenated castor oil, and polyoxyethylene/polyoxypropylene block copolymers; water-soluble polymers such as hydroxyethylcellulose, polyacrylic acid, carboxyvinyl polymers, polyethylene glycol, polyvinylpyrrolidone, and methylcellulose; lower alcohols such as ethanol and isopropanol; polyhydric alcohols such as glycerin, propylene glycol, dipropylene glycol, and sorbitol; sugars such as lactose, lactose hydrate, fructose, and sucrose; inorganic powders such as silicic anhydride, aluminum magnesium silicate, and aluminum silicate; and purified water.

Examples of the excipient include sugars (e.g., lactose, lactose hydrate, fructose, and sucrose), sugar alcohols (e.g., mannitol), starch (corn starch, potato starch, wheat starch, rice starch, pregelatinized starch, gelatinized starch, etc.), cellulose (e.g., crystalline cellulose), and inorganic salts (e.g., calcium silicate, anhydrous calcium hydrogen phosphate, and precipitated calcium carbonate).

Examples of the binder include polyvinyl alcohol, polyvinyl ether, methylcellulose, ethylcellulose, gum arabic, tragacanth, gelatin, shellac, hydroxypropylmethylcellulose, hydroxypropylcellulose, polyvinylpyrrolidone, and polypropylene glycol/polyoxyethylene block copolymers.

Examples of the disintegrant include croscarmellose sodium, carmellose sodium, hydroxypropylcellulose, carmellose, carmellose calcium, methylcellulose, crystalline cellulose, sodium lauryl sulfate, povidone, and polysorbate.

Examples of the lubricant include magnesium stearate, calcium stearate, talc, sucrose fatty acid ester, sodium stearyl fumarate, and hydrogenated oils.

A colorant whose addition to medicaments is accepted is used. Cacao powder, menthol, aromatic powder, peppermint oil, camphol, powdered cinnamon bark, or the like is used as the corrigent.

These tablets or granules may be sugar-coated or appropriately coated in other manners, if necessary. In order to produce a liquid agent such as a syrup or a preparation for injection, formulation is performed in accordance with a routine method by adding a pH adjuster, a dissolving agent, a tonicity agent, and the like and optionally a solubilizer, a stabilizer, and the like to the crystal of the compound of the present invention or a salt thereof, or a solvate thereof.

Administration is preferably parenteral administration, though the administration method is not limited to parenteral administration. Specifically, examples of the parenteral administration include injection dosage forms, transnasal administration dosage forms, transpulmonary administration dosage forms, and percutaneous administration forms. Examples of the injection dosage form include intravenous injection, intramuscular injection, intraperitoneal administration, and subcutaneous administration, which can attain systemic or local administration.

The administration method can be appropriately selected depending on the age and symptoms of a patient. The dose of the pharmaceutical composition containing the crystal of the compound of the present invention or a salt thereof, or a solvate thereof produced by the method of the present invention may be selected, for example, in the range of 0.0001 mg to 1000 mg per kg body weight per dose. Alternatively, the dose can be selected, for example, in the range of 0.001 to 100000 mg/body per patient, though the dose is not necessarily limited to these numeric values. The dose and the administration method vary depending on the body weight, age, symptoms, etc. of a patient and can be appropriately selected by those skilled in the art.

In an aspect, the crystal of the compound of the present invention or a salt thereof, or a solvate thereof can be used for activating Nrf2 or for inhibiting Keap1 and activating Nrf2.

In an aspect, the pharmaceutical composition of the present invention can be used for treating or preventing, for example, a disease in a subject described in Nature Reviews Drug Discovery, 2019, 18, p. 295-317, more specifically, neurodegenerative disease such as Alzheimer's disease, Parkinson's disease, Huntington's disease, Friedreich's ataxia, or amyotrophic lateral sclerosis, lung disease such as idiopathic pulmonary fibrosis, acute respiratory distress syndrome, chronic obstructive pulmonary disease, pulmonary arterial hypertension, or asthma, kidney disease such as chronic kidney disease or acute kidney injury, ophthalmic disease such as uveitis, glaucoma, or age-related macular degeneration, liver disease such as nonalcoholic steatohepatitis, immunological or inflammatory disease such as multiple sclerosis, rheumatoid arthritis, or ulcerative colitis, and cell proliferative disease exemplified by solid cancer such as head and neck cancer (throat cancer, voice box cancer, tongue cancer, etc.), esophageal cancer, stomach cancer, large intestinal cancer (appendix cancer, colon cancer, rectal cancer, etc.), lung cancer (small-cell cancer, non-small cell cancer, etc.), thyroid gland cancer, breast cancer, gallbladder cancer, pancreatic cancer, liver cancer, prostate cancer, ovary cancer, uterine cancer (endometrial cancer, uterine cervical cancer, etc.), testicle cancer, renal cell cancer, urinary bladder cancer, renal pelvis/ureter cancer, malignant melanoma, and skin cancer, or cancer of blood or lymph such as leukemia (acute myeloid leukemia, acute lymphoid leukemia, chronic myeloid leukemia, chronic lymphoid leukemia, etc.), malignant lymphoma (Hodgkin's disease, non-Hodgkin lymphoma, etc.), multiple myeloma, and myelodysplastic syndrome in a subject.

In the present specification, the "subject" includes a mammal. The mammal is preferably a human.

The present invention provides a pharmaceutical composition comprising the crystal of the compound of the present invention or a salt thereof, or a solvate thereof mixed with a pharmacologically acceptable carrier or medium. The present invention also provides a method for producing a pharmaceutical composition containing the crystal of the compound of the present invention or a salt thereof, or a solvate thereof as an active ingredient, comprising the step of mixing the crystal with a pharmacologically acceptable carrier or medium.

The present invention also provides a method for producing a pharmaceutical composition containing the compound of the present invention or a salt thereof, or a solvate thereof as an active ingredient, comprising the step of mixing the compound or the salt thereof or the solvate of the compound or the salt with a pharmacologically acceptable carrier or medium.

In the present specification, the phrase "mixing the crystal of the compound or a salt thereof, or a solvate thereof with a pharmacologically acceptable carrier or medium" includes any aspect of the actions of: (a) sequentially supplementing one of the components: (1) the crystal of the compound or a salt thereof, or a solvate thereof; and (2) a pharmacologically acceptable carrier or medium, with the other component; and (b) adding the components (1) and (2) at the same time. In the present specification, the phrase "mixing the compound or a salt thereof, or a solvate thereof with a pharmacologically acceptable carrier or medium" includes any aspect of the actions of: (a') sequentially supplementing one of the components: (1') the compound or a salt thereof, or a solvate thereof; and (2') a pharmacologically acceptable carrier or medium, with the other component; and (b') adding the components (1') and (2') at the same time. In this context, the "mixing" does not require that a homogeneous mixture should be obtained when the components (1) and (2) or (1') and (2') are mixed. The "mixing" includes, for example, "dissolution", "suspension", or "emulsification".

### Examples

The contents of the present invention will be further described with reference to Examples and Reference Examples given below. All starting materials and reagents were obtained from commercial suppliers or synthesized by use of methods known in the art. Room temperature (rt) refers to 5 to 35°C. The HPLC purification of compounds was performed using AutoPurification HPLC/MS System (manufactured by Waters Corp.) and Trilution (manufactured by Gilson Inc.). ¹H-NMR spectra were measured with or without Me4Si as an internal standard using MR400 OneNMR probe (400 MHz, Agilent technology), AVANCE III HD 400 SMART-BBFO probe (400 MHz, Bruker), ECP400 (400 MHz, JEOL), AVANCE III HD 400 SMART-BBFO probe (400 MHz, Bruker), MR400 OneNMR probe (400 MHz, Agilent technology), or AVANCE NEO 400 iProbe (400 MHz, Bruker) (s = singlet, brs = broad singlet, d = doublet, t = triplet, q = quartet, dd = double doublet ddd = double double doublet, dt = double triplet, td = triple doublet, m = multiplet). NMR data was indicated by ppm (parts per million, δ). Mass spectral data was obtained using a single quadrupole mass spectrometer (LCMS-2020) with ultrahigh-performance liquid chromatography (Nexera UC or Nexera) manufactured by Shimadzu Corp. or a single quadrupole mass spectrometer (SQD or SQD2) with Acquity ultrahigh-performance liquid chromatography (UPLC or UPLC I-Class) manufactured by Waters Corp. Two retention times described respectively denote the retention times of rotational isomers. Microwave irradiation was performed using Initiator(TM) (manufactured by Biotage Japan Ltd.).

**[Table 1]**

| Analysis conditions | Apparatus | Column used | Column temperature | Mobile phase, gradient | Flow rate (mL/min) | Detection wavelength **(**PDA **t**otal) |
|---|---|---|---|---|---|---|
| A | Nexera/2020 | Ascentis Express C18 2.1mmI.D. x 50mmL, 2.7um | 35°C | A) 0.05%TFA, CH₃CN, B) 0.05% TFA, H₂O | 1 | 210-400nm |
| | | | | A/B = 5/95 to 100/0 (1.5 min) → 100/0 (0.5 min) | | |
| B | Nexera UC/2020 | XSelect CSH C18 2.1mmI.D. x 50mmL, 2.5um | 35°C | A) 0.1%FA, CH₃CN, B) 0.1% FA, H₂O | 1 | 210-400nm |
| | | | | A/B = 5/95 to 100/0 (1.75 min) → 100/0 (1.25 min) | | |
| C | Nexera/2020 | Meteoric Core C18 2.1mmI.D. x 50mmL, 2.7um | 35°C | A) 0.05%TFA, CH₃CN, B) 0.05% TFA, H₂O | 1 | 210-400nm |
| | | | | A/B = 5/95 to 100/0 (1.5 min) → 100/0 (0.5 min) | | |
| D | Acquity SQD/SQD2 | Ascentis Express C18 2.1mmI.D. x 50mmL, 2.7um | 35°C | A) 0.1%FA, CH₃CN, B) 0.1% FA, H₂O | 1 | 210-400nm |
| | | | | A/B = 5/95 to 100/0 (1.0 min) → 100/0 (0.4 min) | | |
| E | Ac quity SQD/SQD2 | Ascentis Express C18 2.1mmI.D. x 50mmL, 5um | 35°C | A) MeOH, B)10 mM AA, H₂O | 0.9 | 210-400nm |
| | | | | A/B = 5/95 to 100/0 (1.0 min) → 100/0 (0.4 min) | | |
| F | Nexera/2020 | Ascentis Express C18 2.1mmI.D. x 50mmL, 2.7um | 35°C | A) 0.1%FA, CH₃CN, B) 0.1% FA, H₂O | 1 | 210-400nm |
| | | | | A/B = 5/95 to 100/0 (1.5 min) → 100/0 (0.5 min) | | |
| G | Nexera/2020 | Meteoric Core C18 2.1mmI.D. x 50mmL, 2.7um | 35°C | A) 0.1%FA, CH₃CN, B) 0.1% FA, H₂O | 1 | 210-400nm |
| | | | | A/B = 5/95 to 100/0 (1.5 min) → 100/0 (0.5 min) | | |
| H | Nexera/2020 | Speed Core C18 2.1mmI.D. x 50mmL, 2.7um | 35°C | A) 0.1%FA, CH₃CN, B) 0.1% FA, H₂O | 1 | 210-400nm |
| | | | | A/B = 5/95 to 100/0 (1.5 min) → 100/0 (0.5 min) | | |
| I | Nexera/2020 | Kinetex 1.7u C18 2.1mmI.D. x 50mmL. 1.7um | 35°C | A) 0.05%TFA, CH₃CN, B) 0.05% TFA, H₂O | 1 | 210-400nm |
| | | | | A/B = 5/95 to 0/100 (1.5 min) → 0/100 (0.5 min) | | |
| J | Nexera/2020 | Ascentis Express C18 2.1mmI.D. x 50mmL, 5um | 35°C | A) 0.1%FA, CH₃CN, B) 0.1% FA, H₂O | 1 | 210-400nm |
| | | | | A/B = 5/95 to 100/0 (4.5 min) → 100/0 (0.5 min) | | |
| K | Shimadzu LCMS-2020 | Shim-pack XR-ODS 3mmx5cm | 40°C | A)0.05%TFA , CH₃CN, | 1 | 220±4nm |
| | | | | B)0.05%TFA, H₂O | | |
| | | | | A/B = 5/95 to 100/0 (2.2 min) →100/0 (1.0 min) | | |
| L | Shimadzu LCMS-2020 | Shim-pack XR-ODS 2.2um 3mmx5cm | 40°C | A)0.05%TFA, CH₃CN, | 1 | 254±4nm |
| | | | | B)0.05%TFA, H₂O | | |
| | | | | A/B = 5/95 to 100/0 (1.2 min) →100/0 (1.0 min) | | |

### [Example 1] Synthesis of compound

### <Example 1-1>

### Compound A

### Methyl 4-[3-(4-bromo-2,6-dichlorobenzoyl)-2,4-dihydro-1,3-benzoxazin-8-yl]-5-fluoro-2-(3-oxa-8-azabicyclo[3.2.1]octan-8-yl)benzoate

### First step

### Compound A1

### tert-Butyl N-[(3-bromo-2-hydroxyphenyl)methyl]carbamate

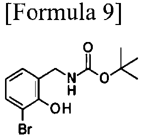

A solution of 3-bromo-2-hydroxybenzaldehyde (5.00 g, 24.9 mmol) and tert-butyl carbamate (8.74 g, 74.6 mmol) in acetonitrile (35.0 mL) was cooled to 0°C. Triethylsilane (11.9 mL, 74.6 mmol) and trifluoroacetic acid (3.81 mL, 49.7 mmol) were added to the reaction solution. The mixture was stirred at 35°C for 5 hours and then cooled to room temperature. After stirring at room temperature for 15 hours, water was added to the reaction solution, and the mixture was stirred for 1 hour. Then, water was further added thereto, and the mixture was stirred for 30 minutes. The reaction solution was filtered and washed with acetonitrile/water (1/2). The resultant was further washed with heptane and dried under reduced pressure to obtain the title compound (88%, 6.60 g).
LCMS: m/z 300[M-H]⁻
HPLC retention time: 1.16 min (analysis condition F)

### Second step

### Compound A2

### tert-Butyl 8-bromo-2,4-dihydro-1,3-benzoxazine-3-carboxylate

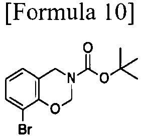

To a solution of tert-butyl N-[(3-bromo-2-hydroxyphenyl)methyl]carbamate (5.00 g, 16.6 mol) in acetonitrile (30.0 mL), a 36% aqueous formaldehyde solution (5.06 mL, 66.2 mmol) and formic acid (5.08 mL, 132 mmol) were added, and the mixture was stirred at 56°C for 7 hours. The reaction solution was cooled to 25°C. Water was added thereto, and the mixture was stirred for 30 minutes. Water was further added thereto, and the mixture was stirred for 30 minutes. The reaction solution was filtered and washed with acetonitrile/water (1/2). The resultant was dried under reduced pressure to obtain the title compound (81%, 4.20 g).
LCMS: m/z 214[M-Boc+H]⁺
HPLC retention time: 1.31 min (analysis condition F)

### Third step

### Compound A3

### 4-Bromo-5-fluoro-2-(3-oxa-8-azabicyclo[3.2.1]octan-8-yl)benzoic acid

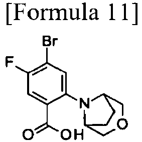

To a suspension of 4-bromo-2,5-difluorobenzoic acid (3.00 g, 12.7 mmol) and 3-oxa-8-azabicyclo[3.2.1]octane hydrochloride (2.46 g, 16.5 mmol) in tetrahydrofuran (6.00 mL), a solution of 1 M lithium(bistrimethylsilyl)amide in tetrahydrofuran (50.6 mL, 50.6 mmol) was added at room temperature over 12 minutes. The reaction solution was stirred at room temperature for 5.5 hours and then left standing at room temperature for 15 hours. 2 M hydrochloric acid was added to the reaction solution, followed by extraction with ethyl acetate. The aqueous layer was subjected to extraction with ethyl acetate again, and two organic layers were combined and washed with a 20% aqueous ammonium chloride solution and a 15% aqueous sodium chloride solution. The washed organic layer was concentrated to obtain the title compound as a crude product.
LCMS: m/z 330[M+H]⁺
HPLC retention time: 0.61 min (analysis condition D)

### Fourth step

### Compound A4

### Methyl 4-bromo-5-fluoro-2-(3-oxa-8-azabicyclo[3.2.1]octan-8-yl)benzoate

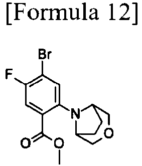

To a solution of 4-bromo-5-fluoro-2-(3-oxa-8-azabicyclo[3.2.1]octan-8-yl)benzoic acid (4.18 g, 12.7 mmol) in N,N-dimethylformamide (21.0 mL), potassium carbonate (2.10 g, 15.2 mmol) and iodomethane (1.58 mL, 25.3 mmol) were added, and the mixture was stirred at room temperature for 30 minutes. A 20% aqueous ammonium chloride solution and water were added to the reaction solution, followed by extraction with ethyl acetate. The organic layer was washed with a 20% aqueous ammonium chloride solution and concentrated. Methanol was added to the obtained crude product, and the mixture was heated to 60°C, then cooled to room temperature, and stirred for 30 minutes. Water was added thereto, and the mixture was stirred for 1 hour. Then, water was further added thereto, and the mixture was stirred for 30 minutes. A solid obtained after filtration was washed with methanol/water (1/1) and dried under reduced pressure to obtain the title compound (55%, 2.38 g).
LCMS: m/z 344[M+H]⁺
HPLC retention time: 1.18 min (analysis condition F)

### Fifth step

### Compound A5

### tert-Butyl 8-[2-fluoro-4-methoxycarbonyl-5-(3-oxa-8-azabicyclo[3.2.1]octan-8-yl)phenyl]-2,4-dihydro-1,3-benzoxazine-3-carboxylate

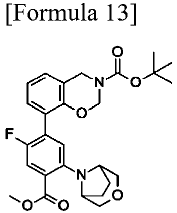

A solution of methyl 4-bromo-5-fluoro-2-(3-oxa-8-azabicyclo[3.2.1]octan-8-yl)benzoate (1.21 g, 3.50 mmol) in tetrahydrofuran (5.30 mL) was cooled to -10°C. A 2 M solution of isopropyl magnesium chloride in tetrahydrofuran (1.91 mL, 3.82 mmol) was added thereto, and the mixture was stirred for 1 hour. A 2 M solution of zinc(II) chloride in 2-methyltetrahydrofuran (0.955 mL, 1.91 mmol) was added to the reaction solution. tert-Butyl 8-bromo-2,4-dihydro-1,3-benzoxazine-3-carboxylate (1.00 g, 3.18 mmol) and SPhos Pd G3 (0.0250 g, 0.0320 mmol) were added to the reaction solution, and the mixture was warmed to 45°C and stirred for 90 minutes. The reaction solution was cooled to room temperature, and ethyl acetate and an aqueous ammonium chloride solution were added thereto, followed by extraction with ethyl acetate. The organic layer was washed with an aqueous N-acetylcysteine solution and an aqueous sodium chloride solution and concentrated to obtain a crude product. Ethanol was added to the obtained crude product, which was dissolved by warming to 80°C. The solution was cooled to room temperature. Heptane was added thereto, and the mixture was cooled to 0°C. A solid obtained by filtration was washed with ethanol/heptane (1/2) and dried under reduced pressure to obtain the title compound (73%, 1.16 g).
LCMS: m/z 499[M+H]⁺
HPLC retention time: 1.40 min (analysis condition F)

### Sixth step

### Compound A6

### Methyl 4-(3,4-dihydro-2H-1,3-benzoxazin-8-yl)-5-fluoro-2-(3-oxa-8-azabicyclo[3.2.1]octan-8-yl)benzoate dihydrochloride

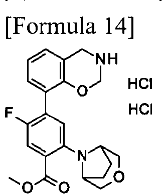

To tert-butyl 8-[2-fluoro-4-methoxycarbonyl-5-(3-oxa-8-azabicyclo[3.2.1]octan-8-yl)phenyl]-2,4-dihydro-1,3-benzoxazine-3-carboxylate (10.0 g, 20.1 mmol) in acetonitrile (100 mL), a 4 M solution of hydrochloric acid in ethyl acetate (25.1 mL, 100 mmol) was added, and the mixture was stirred at room temperature for 2 hours. The reaction solution was filtered to obtain the title compound (76%, 7.16 g) as crystals.
LCMS: m/z 399[M+H]⁺
HPLC retention time: 0.75 min (analysis condition F)

### Seventh step

### Compound A7

### 4-Bromo-2,6-dichlorobenzoyl chloride

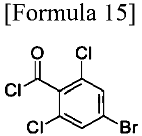

To a solution of 4-bromo-2,6-dichlorobenzoic acid (45.5 g, 169 mmol) in toluene (241 mL), thionyl chloride (24.5 mL, 337 mmol) and N,N-dimethylformamide (0.261 mL, 3.37 mmol) were added, and the mixture was stirred at 70°C for 7 hours. The reaction solution was cooled to room temperature and concentrated, and the obtained residue was purified by silica gel column chromatography (hexane/ethyl acetate) to obtain the title compound (99%, 48.0 g). HPLC retention time: 1.48 min (analysis condition F)
¹H NMR (400 MHz, CDCl₃) δ 7.56 (s, 2H)

### Eighth step

### Compound A

### Methyl 4-[3-(4-bromo-2,6-dichlorobenzoyl)-2,4-dihydro-1,3-benzoxazin-8-yl]-5-fluoro-2-(3-oxa-8-azabicyclo[3.2.1]octan-8-yl)benzoate

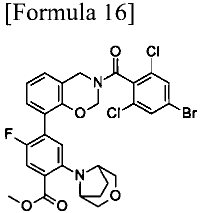

To a solution of methyl 4-(3,4-dihydro-2H-1,3-benzoxazin-8-yl)-5-fluoro-2-(3-oxa-8-azabicyclo[3.2.1]octan-8-yl)benzoate dihydrochloride (9.22 g, 19.6 mmol) in toluene (92.0 mL), 4-bromo-2,6-dichlorobenzoyl chloride (7.33 g, 25.4 mmol) and pyridine (11.1 mL, 137 mmol) were added, and the mixture was stirred at 70°C for 12 hours. Ethyl acetate and 1 M hydrochloric acid were added to the reaction solution for extraction. The organic layer was washed with a 50% saturated aqueous solution of sodium bicarbonate and a 50% saturated aqueous solution of sodium chloride and dried over anhydrous magnesium sulfate. After concentration, the obtained residue was triturated with hexane to obtain the title compound (48%, 6.06 g).
LCMS: m/z 649[M+H]⁺
HPLC retention time: 1.42 min (analysis condition F)

### <Example 1-2>

### Compound B

### Methyl 4-[3-(4-bromo-2,6-dichlorobenzoyl)-2,4-dihydro-1,3-benzoxazin-8-yl]-5-fluoro-2-morpholin-4-ylbenzoate

### First step

### Compound B1

### 4- Bromo-5- fluoro-2-morpholin-4-ylbenzoic acid

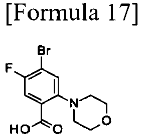

To 4-bromo-2,5-difluorobenzoic acid (950 mg, 4.01 mmol), morpholine (6.98 g, 80.0 mmol) was added, and the mixture was stirred at 100°C for 24 hours. Ethyl acetate and hydrochloric acid were added to the reaction solution for extraction. Then, the extract was washed with saturated saline. The organic layer was concentrated to obtain the title compound (98%, 1.20 g).
LCMS: m/z 304[M+H]⁺
HPLC retention time: 0.77 min (analysis condition H)

### Second step

### Compound B2

### Methyl 4-bromo-5-fluoro-2-morpholin-4-ylbenzoate

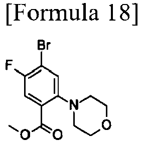

To a solution of 4-bromo-5-fluoro-2-morpholin-4-ylbenzoic acid (400 mg, 1.32 mmol) in dichloromethane (5.00 mL)-methanol (1.00 mL), a 2 M solution of trimethylsilyldiazomethane in hexane (1.32 mL, 2.63 mmol) was added, and the mixture was stirred at room temperature for 30 minutes. Acetic acid was added to the reaction solution, and the reaction solution was concentrated. Then, the reaction mixture was purified by silica gel chromatography (hexane/ethyl acetate) to obtain the title compound (81%, 341 mg).
LCMS: m/z 318[M+H]⁺
HPLC retention time: 1.10 min (analysis condition H)

### Third step

### Compound B3

### tert-Butyl 8-(2-fluoro-4-methoxycarbonyl-5-morpholin-4-ylphenyl)-2,4-dihydro-1,3-benzoxazine-3-carboxylate

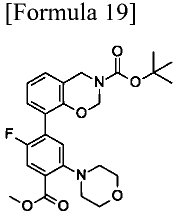

A solution of methyl 4-bromo-5-fluoro-2-morpholin-4-ylbenzoate (2.95 g, 9.28 mmol), bis(pinacolato)diboron (2.78 g, 11.0 mmol), potassium acetate (2.48 g, 25.3 mmol), and a 1,1'-bis(diphenylphosphino)ferrocene-palladium(II) dichloride dichloromethane complex (0.344 g, 0.422 mmol) in 1,4-dioxane (16.9 mL) was stirred at 90°C for 5 hours. The reaction solution was cooled to room temperature. tert-Butyl 8-bromo-2,4-dihydro-1,3-benzoxazine-3-carboxylate (2.65 g, 8.43 mmol), a 1,1'-bis(diphenylphosphino)ferrocene-palladium(II) dichloride dichloromethane complex (0.344 g, 0.422 mmol), potassium carbonate (3.50 g, 25.3 mmol), and water (4.22 mL) were added thereto, and the mixture was stirred at 90°C for 3 hours. The reaction solution was cooled to room temperature. Water and ethyl acetate were added thereto, and the mixture was filtered through celite. The organic layer was washed with saline, dried, and concentrated. The obtained residue was purified by silica gel chromatography (hexane/ethyl acetate) to obtain the title compound (91%, 3.63 g).
LCMS: m/z 473[M+H]⁺
HPLC retention time: 1.34 min (analysis condition F)

### Fourth step

### Compound B4

### Methyl 4-(3,4-dihydro-2H-1,3-benzoxazin-8-yl)-5-fluoro-2-morpholin-4-ylbenzoate dihydrochloride

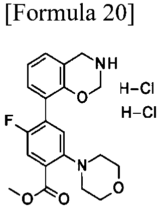

To a solution of tert-butyl 8-(2-fluoro-4-methoxycarbonyl-5-morpholin-4-ylphenyl)-2,4-dihydro-1,3-benzoxazine-3-carboxylate (100 mg, 0.212 mmol) in dichloromethane (1.058 mL), a 4 M solution of hydrochloric acid in 1,4-dioxane (0.794 mL, 3.17 mmol) was added, and the mixture was stirred at room temperature for 2 hours. The reaction solution was concentrated to obtain the title compound as a crude product.
LCMS: m/z 373[M+H]⁺
HPLC retention time: 0.49 min (analysis condition D)

### Fifth step

### Compound B

### Methyl 4-[3-(4-bromo-2,6-dichlorobenzoyl)-2,4-dihydro-1,3-benzoxazin-8-yl]-5-fluoro-2-morpholin-4-ylbenzoate

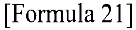

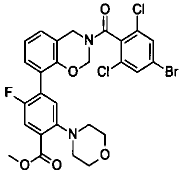

A solution of methyl 4-(3,4-dihydro-2H-1,3-benzoxazin-8-yl)-5-fluoro-2-morpholin-4-ylbenzoate dihydrochloride (141 mg, 0.317 mmol) and 4-bromo-2,6-dichlorobenzoyl chloride (137 mg, 0.476 mmol) in dichloromethane (1.585 mL) was cooled to 0°C. N,N-Diisopropylethylamine (0.166 mL, 0.951 mmol) was added thereto, and the mixture was stirred at room temperature for 3 hours. Ethanolamine was added to the reaction solution, and the mixture was purified by reverse phase column chromatography (acetonitrile/water, 0.1% formic acid) to obtain the title compound (56%, 110 mg).
LCMS: m/z 623[M+H]⁺
HPLC retention time: 1.37 min (analysis condition F)

### <Example 1-3>

### Compound 1

### 4-[3-[2,6-Dichloro-4-[(2R)-2,4-dimethylpiperazin-1-yl]benzoyl]-2,4-dihydro-1,3-benzoxazin-8-yl]-5-fluoro-2-(3-oxa-8-azabicyclo[3.2.1]octan-8-yl)benzoic acid

### First step

### Compound 1-1

### Methyl 4-[3-[2,6-dichloro-4-[(2R)-2,4-dimethylpiperazin-1-yl]benzoyl]-2,4-dihydro-1,3-benzoxazin-8-yl]-5-fluoro-2-(3-oxa-8-azabicyclo[3.2.1]octan-8-yl)benzoate

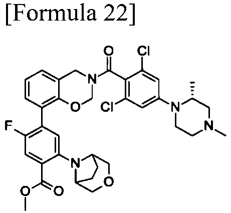

A solution of compound A (20.2 g, 31.1 mmol), (3R)-1,3-dimethylpiperazine (4.61 g, 40.4 mmol), rac-BINAP Pd G4 (938 mg, 0.932 mmol) and cesium carbonate (30.4 g, 93.0 mmol) in 1,4-dioxane (202 mL) was stirred at 80°C for 7.5 hours. (3R)-1,3-Dimethylpiperazine (2.48 g, 21.7 mmol) was further added thereto, and the reaction solution was stirred at 100°C for 49 hours. The reaction solution was filtered through celite and washed with ethyl acetate. The filtrate was washed with a 15% aqueous sodium chloride solution. The organic layer was concentrated, and the obtained residue was purified by silica gel column chromatography (hexane/ethyl acetate/methanol) to obtain the title compound (75%, 15.9 g).
LCMS: m/z 683[M+H]⁺
HPLC retention time: 0.89 min (analysis condition F)

### Second step

### Compound 1

### 4-[3-[2,6-Dichloro-4-[(2R)-2,4-dimethylpiperazin-1-yl]benzoyl]-2,4-dihydro-1,3-benzoxazin-8-yl]-5-fluoro-2-(3-oxa-8-azabicyclo[3.2.1]octan-8-yl)benzoic acid

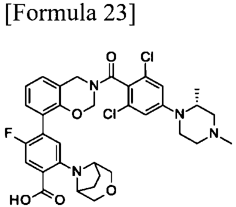

To a solution of methyl 4-[3-[2,6-dichloro-4-[(2R)-2,4-dimethylpiperazin-1-yl]benzoyl]-2,4-dihydro-1,3-benzoxazin-8-yl]-5-fluoro-2-(3-oxa-8-azabicyclo[3.2.1]octan-8-yl)benzoate (18.6 g, 27.2 mmol) in 1-methylpyrrolidin-2-one (186 mL), an 8 M aqueous potassium hydroxide solution (11.9 mL, 95.0 mmol) was added, and the mixture was stirred at 60°C for 2 hours. An aqueous formic acid solution was added to the reaction solution, and the mixture was purified by reverse phase column chromatography (acetonitrile/water, 0.1% formic acid) to obtain the title compound (85%, 15.4 g).
LCMS: m/z 669[M+H]⁺
HPLC retention time: 0.99 min and 1.04 min (analysis condition B)

### <Example 1-4>

### Compound 2

### 4-[3-[2,6-Dichloro-4-[4-(2-methoxyethyl)piperazin-1-yl]benzoyl]-2,4-dihydro-1,3-benzoxazin-8-yl]-5-fluoro-2-morpholin-4-ylbenzoic acid

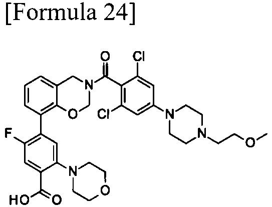

A solution of compound B (1.72 g, 2.76 mmol), 1-(2-methoxyethyl)piperazine (0.818 ml, 5.51 mmol), [2,2'-bis(diphenylphosphino)-1,1'-binaphthyl]dichloropalladium(II) (65.0 mg, 0.0810 mmol), and cesium carbonate (2.69 g, 8.27 mmol) in toluene (15.0 mL) was heated at 95°C for 2 hours. The reaction solution was cooled to room temperature, and the solvent was concentrated. Dimethyl sulfoxide (5.00 mL), 1,4-dioxane (5.00 mL) and a 5 M aqueous sodium hydroxide solution (2.76 mL, 13.8 mmol) were added to the residue, and the mixture was stirred at 90°C for 1 hour. The reaction solution was concentrated to remove 1,4-dioxane. Formic acid was added to the residue, and the reaction mixture was purified by reverse phase column chromatography (acetonitrile/water, 0.1% formic acid) to obtain the title compound (67%, 1.24 g).
LCMS: m/z 673[M+H]⁺
HPLC retention time: 0.77 min and 0.82 min (analysis condition A)

### <Example 1-5>

### Compound 3

### 4-[3-[2,6-Dichloro-4-(7,7-dimethyl-5,9-dioxa-2-azaspiro[3.5]nonan-2-yl)benzoyl]-2,4-dihydro-1,3-benzoxazin-8-yl]-5-fluoro-2-(3-oxa-8-azabicyclo[3.2.1]octan-8-yl)benzoic acid First step

### Compound 3-1

### Benzyl 7,7-dimethyl-5,9-dioxa-2-azaspiro[3.5]nonane-2-carboxylate

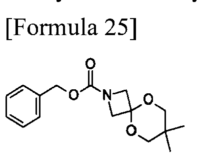

To a solution of benzyl 3-oxoazetidine-1-carboxylate (15.0 g, 73.1 mmol) in acetonitrile (300 mL), 2,2-dimethyl-1,3-propanediol (14.5 ml, 146 mmol) and trimethylsilyl trifluoromethanesulfonate (6.60 ml, 36.5 mmol) were added, and the mixture was stirred at 70°C for 7 hours. The reaction solution was cooled to room temperature and concentrated. The obtained residue was dissolved in ethyl acetate and washed with a 5% aqueous sodium bicarbonate solution and a 15% aqueous sodium chloride solution, and the organic layer was then dried over magnesium sulfate and concentrated. The obtained residue was purified by silica gel column chromatography (hexane/ethyl acetate) to obtain the title compound (84%, 17.9 g).
LCMS: m/z 292[M+H]⁺
HPLC retention time: 1.15 min (analysis condition F)

### Second step

### Compound 3-2

### 7,7-Dimethyl-5,9-dioxa-2-azaspiro[3.5]nonane

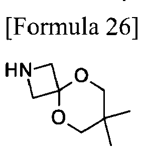

To a solution of benzyl 7,7-dimethyl-5,9-dioxa-2-azaspiro[3.5]nonane-2-carboxylate (3.63 g, 12.5 mmol) in 1,4-dioxane (36.3 mL), 20% palladium hydroxide-active carbon (containing water) (306 mg, 0.436 mmol) was added, and the mixture was stirred for 14 hours in a hydrogen atmosphere. The reaction solution was filtered through celite, washed with 1,4-dioxane, and concentrated to obtain the title compound as a crude product in a 1,4-dioxane solution.

### Third step

### Compound 3-3

### Methyl 4-[3-[2,6-dichloro-4-(7,7-dimethyl-5,9-dioxa-2-azaspiro[3.5]nonan-2-yl)benzoyl]-2,4-dihydro-1,3-benzoxazin-8-yl]-5-fluoro-2-(3-oxa-8-azabicyclo[3.2.1]octan-8-yl)benzoate

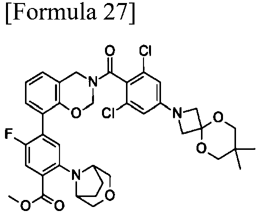

A solution of compound A (4.40 g, 6.77 mmol), 6.58 wt% 7,7-dimethyl-5,9-dioxa-2-azaspiro[3.5]nonane in a 1,4-dioxane solution (21.0 g, 8.80 mmol), cesium carbonate (6.61 g, 20.3 mmol) and rac-BINAP Pd G4 (204 mg, 0.203 mmol) in 1,4-dioxane (30.8 mL) was stirred at 60°C for 4.5 hours. The reaction solution was filtered through celite and washed with ethyl acetate. The filtrate was washed with a 15% aqueous sodium chloride solution, followed by extraction. The organic layer was dried over sodium sulfate and concentrated. Ethyl acetate and hexane were added to the obtained residue, and the mixture was triturated to quantitatively obtain the title compound (4.96 g).
LCMS: m/z 726[M+H]⁺
HPLC retention time: 1.48 min (analysis condition F)

### Fourth step

### Compound 3

### 4-[3-[2,6-Dichloro-4-(7,7-dimethyl-5,9-dioxa-2-azaspiro[3.5]nonan-2-yl)benzoyl]-2,4-dihydro-1,3-benzoxazin-8-yl]-5-fluoro-2-(3-oxa-8-azabicyclo[3.2.1]octan-8-yl)benzoic acid

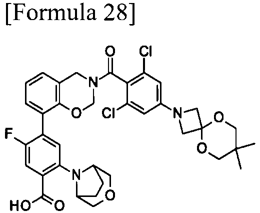

To a solution of methyl 4-[3-[2,6-dichloro-4-(7,7-dimethyl-5,9-dioxa-2-azaspiro[3.5]nonan-2-yl)benzoyl]-2,4-dihydro-1,3-benzoxazin-8-yl]-5-fluoro-2-(3-oxa-8-azabicyclo[3.2.1]octan-8-yl)benzoate (12.8 g, 17.6 mmol) in tetrahydrofuran (32.0 mL) and methanol (32.0 mL), an 8 M aqueous potassium hydroxide solution (6.61 mL, 52.8 mmol) was added, and the mixture was stirred at 50°C for 3 hours. The reaction solution was cooled to room temperature. 1 M hydrochloric acid was added thereto, and water was added, followed by extraction with ethyl acetate. The organic layer was washed with a 3% aqueous N-acetylcysteine solution and a 15% aqueous sodium chloride solution, dried over sodium sulfate, and concentrated. Ethanol was added to the residue, and the mixture was heated at 60°C. Then, water was added thereto, and the mixture was cooled to room temperature. Water was further added thereto, and the obtained solid was collected by filtration and washed with ethanol/water (1/6) to obtain the title compound (88%, 11.0 g).
LCMS: m/z 712[M+H]⁺
HPLC retention time: 1.68 min and 1.72 min (analysis condition B)

### <Example 1-6>

### Compound 4

### 4-[3-[2,6-Dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoic acid Synthesis method A

### First step

### Compound 4-1

### 2,6-Dichloro-4-(6-methoxy-2-azaspiro[3.3]heptan-2-yl)benzoic acid

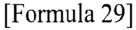

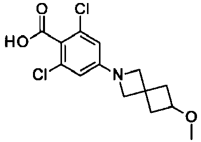

To a solution of 4-bromo-2,6-dichlorobenzoic acid (1.28 g, 4.74 mmol), 6-methoxy-2-azaspiro[3.3]heptane hydrochloride (1.55 g, 9.48 mmol), Xantphos (137 mg, 0.237 mmol), allylpalladium(II) chloride (dimer) (87.0 mg, 0.237 mmol), and trans,trans-1,5-diphenyl-1,4-pentadien-3-one (56.0 mg, 0.237 mmol) in 1,3-dimethyl-2-imidazolidinone (23.7 mL), a 1 M solution of sodium bis(trimethylsilyl)amide in tetrahydrofuran (19.0 mL, 19.0 mmol) was added dropwise. The reaction mixture was stirred at room temperature for 17 hours. Then, an aqueous formic acid solution and dimethyl sulfoxide were added thereto, and the mixture was purified by reverse phase column chromatography (acetonitrile/water, 0.1% formic acid) to obtain the title compound (84%, 1.26 g).
LCMS: m/z 316[M+H]⁺
HPLC retention time: 1.01 min (analysis condition F)

### Second step

### Compound 4-2

### Methyl 4-[3-[2,6-dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoate

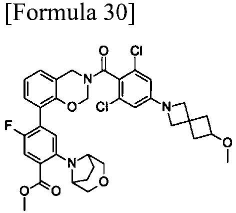

To a solution of 2,6-dichloro-4-(6-methoxy-2-azaspiro[3.3]heptan-2-yl)benzoic acid (698 mg, 2.21 mmol) in ethyl acetate (14.0 mL), a Ghosez reagent (0.350 mL, 2.65 mmol) was added. The mixture was stirred at room temperature for 30 minutes. 4-Methylmorpholine (1.54 mL, 14.1 mmol) and methyl 4-(3,4-dihydro-2H-1,3-benzoxazin-8-yl)-5-fluoro-2-(3-oxa-8-azabicyclo[3.2.1]octan-8-yl)benzoate dihydrochloride (944 mg, 2.01 mmol) were added to the reaction mixture. The reaction mixture was stirred overnight at 50°C. Then, the solution was cooled to room temperature, and 0.5 M sulfuric acid was added thereto, followed by extraction with ethyl acetate. The organic layer was washed with a saturated aqueous solution of sodium bicarbonate and concentrated to obtain a crude product of the title compound.
LCMS: m/z 696[M+H]⁺
HPLC retention time: 3.22 min (analysis condition J)

### Third step

### Compound 4

### 4-[3-[2,6-Dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoic acid

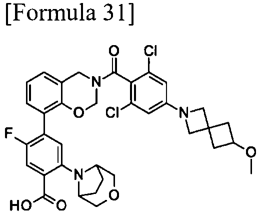

A solution of methyl 4-[3-[2,6-dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoate (196 mg, 0.281 mmol) and an 8 M aqueous potassium hydroxide solution (0.352 mL, 2.81 mmol) in tetrahydrofuran (0.471 mL) and ethanol (1.57 mL) was stirred at 60°C for 1 hour. The reaction mixture was cooled to room temperature. Then, an aqueous formic acid solution and dimethyl sulfoxide were added thereto, and the mixture was purified by reverse phase column chromatography (acetonitrile/water, 0.1% formic acid) to obtain the title compound (95%, 183 mg).
LCMS: m/z 682[M+H]⁺
HPLC retention time: 1.58 min and 1.61 min (analysis condition B)

### <Example 1-7>

### Compound 4

### 4-[3-[2,6-Dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoic acid Synthesis method B

### First step

### Compound 4-2

### Methyl 4-[3-[2,6-dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoate

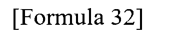

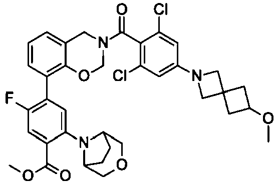

A solution of compound A (12.0 g, 18.5 mmol), 6-methoxy-2-azaspiro[3.3]heptane hydrochloride (4.53 g, 27.7 mmol), rac-BINAP (1.15 g, 1.85 mmol), tris(dibenzylideneacetone)dipalladium(0) (846 mg, 0.923 mmol) and cesium carbonate (30.1 g, 92.0 mmol) in 1-methylpyrrolidin-2-one (84.0 mL) and water (8.39 mL) was stirred at 100°C for 3 hours. The reaction solution was cooled to room temperature, followed by extraction by the addition of ethyl acetate and water. The organic layer was washed with an aqueous N-acetylcysteine solution and an aqueous sodium chloride solution, dried over magnesium sulfate, and then concentrated, and the obtained residue was purified by silica gel column chromatography (hexane/ethyl acetate) to obtain the title compound (12.6 g) as a mixture with ethyl acetate.
LCMS: m/z 696[M+H]⁺
HPLC retention time: 3.22 min (analysis condition J)

### Second step

### Compound 4

### 4-[3-[2,6-Dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoic acid

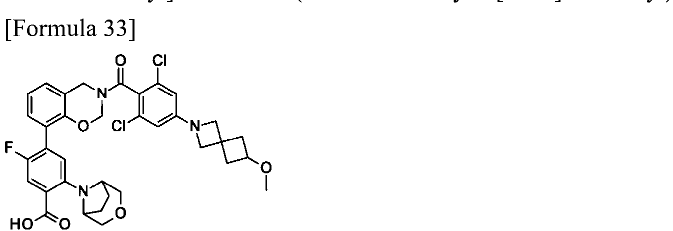

To a solution of methyl 4-[3-[2,6-dichloro-4-(6-methoxy-2-azaspiro[3.3]heptan-2-yl)benzoyl]-2,4-dihydro-1,3-benzoxazin-8-yl]-5-fluoro-2-(3-oxa-8-azabicyclo[3.2.1]octan-8-yl)benzoate (12.3 g, 17.6 mmol) in tetrahydrofuran (22.0 mL)/ethanol(22.0 mL), an 8 M aqueous potassium hydroxide solution (11.0 mL, 88.0 mmol) was added, and the mixture was stirred at 60°C for 2.5 hours. The reaction solution was cooled to room temperature. 0.5 M hydrochloric acid and water were added thereto, and the mixture was stirred. The obtained solid was collected by filtration, washed with water, and then dried under reduced pressure. An ethanol/water (1/9) solution was added to the obtained solid, and the solution was frozen in a freezer of -80°C and then freeze-dried to obtain the title compound (91%, 10.9 g).
LCMS: m/z 682[M+H]⁺
HPLC retention time: 1.58 min and 1.61 min (analysis condition B)

### <Example 1-8>

### Compound 5

### 4-[3-[2-Chloro-4-[(2R,5R)-2,4,5-trimethylpiperazin-1-yl]benzoyl]-2,4-dihydro-1,3-benzoxazin-8-yl]-5-fluoro-2-(3-oxa-8-azabicyclo[3.2.1]octan-8-yl)benzoic acid

### First step

### Compound 5-1

### (2R,5R)-1,2,5-Trimethylpiperazine

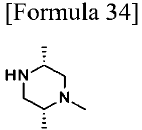

To a solution of (2R,5R)-1,2,5-trimethylpiperazine dihydrochloride (10.3 g, 51.0 mmol) in tetrahydrofuran (49.6 mL), a 50% aqueous potassium hydroxide solution (11.4 g, 102 mmol) was added, and the mixture was stirred at room temperature, then filtered, and washed with tetrahydrofuran. The filtrate was concentrated to obtain the title compound in a tetrahydrofuran solution.
¹H-NMR (400 MHz, DMSO-D6) δ: 5.75 (1H, s), 2.78-2.68 (2H, m), 2.56 (1H, dd, J = 12.0, 4.6 Hz), 2.39-2.36 (1H, m), 2.19 (1H, dd, J = 11.0, 3.4 Hz), 2.13-2.09 (4H, m), 0.99 (3H, d, J = 6.4 Hz), 0.91 (3H, d, J = 6.6 Hz).

### Second step

### Compound 5-2

### 2-Chloro-4-[(2R,5R)-2,4,5-trimethylpiperazin-1-yl]benzoic acid

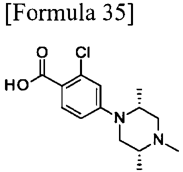

To a solution of (2R,5R)-1,2,5-trimethylpiperazine (1.63 g, 12.7 mmol) in tetrahydrofuran (18.4 mL), sodium tert-butoxide (2.78 g, 28.9 mmol) was added. A solution of 4-bromo-2-chlorobenzoic acid (2.00 g, 8.49 mmol) and rac-BINAP (0.264 g, 0.425 mmol) in 4-methyltetrahydropyran (6.00 mL) was added thereto. In another container, 4-methyltetrahydropyran (4.00 mL) was added to allylpalladium(II) chloride (dimer) (0.0780 g, 0.212 mmol) and rac-BINAP (0.264 g, 0.425 mmol), and the mixture was stirred for 30 minutes. The solution thus prepared was added to the reaction solution, and the mixture was stirred at 90°C for 2 hours. The reaction solution was cooled to room temperature. Then, an aqueous N-acetylcysteine solution was added thereto, and the mixture was stirred at 45°C for 3 hours. 6 M hydrochloric acid and 4-methyltetrahydropyran were added thereto for back-extraction. Ethyl acetate was added to the aqueous layer, and back-extraction was performed by the addition of an 8 M aqueous sodium hydroxide solution. Ammonium sulfate was added to the obtained aqueous layer, followed by extraction with tetrahydrofuran. The organic layer was concentrated. Trifluoroethanol was added to the residue, and the mixture was heated to 75°C. Acetonitrile was added thereto, and the mixture was stirred at 75°C. The reaction mixture was cooled to 25°C, and the obtained solid was collected by filtration, washed with acetonitrile, and then dried under reduced pressure to obtain the title compound (60%, 1.22 g).
LCMS: m/z 283[M+H]⁺
HPLC retention time: 0.33 min (analysis condition E)

### Third step

### Compound 5-3

### Methyl 4-(3,4-dihydro-2H-1,3-benzoxazin-8-yl)-5-fluoro-2-(3-oxa-8-azabicyclo[3.2.1]octan-8-yl)benzoate methanesulfonate

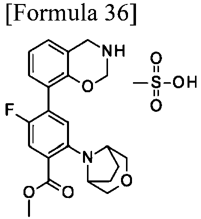

To a solution of tert-butyl 8-[2-fluoro-4-methoxycarbonyl-5-(3-oxa-8-azabicyclo[3.2.1]octan-8-yl)phenyl]-2,4-dihydro-1,3-benzoxazine-3-carboxylate (7.04 g, 14.1 mmol) in acetonitrile (28.1 mL), methanesulfonic acid (4.58 mL, 70.6 mmol) was added, and the mixture was stirred at room temperature for 2 hours to obtain the title compound in an acetonitrile solution.

### Fourth step

### Compound 5-4

### 2-Chloro-4-[(2R,5R)-2,4,5-trimethylpiperazin-1-yl]benzoyl chloride

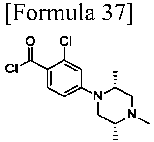

To a mixture of 2-chloro-4-[(2R,5R)-2,4,5-trimethylpiperazin-1-yl]benzoic acid (4.99 g, 16.9 mmol) and acetonitrile (42.2 mL), a Ghosez reagent (3.97 mL, 28.8 mmol) was added, and the mixture was stirred at room temperature for 1 hour. Methanesulfonic acid (1.19 mL, 18.3 mmol) was added thereto. A solution of the title compound in acetonitrile was obtained.

### Fifth step

### Compound 5-5

### Methyl 4-[3-[2-chloro-4-[(2R,5R)-2,4,5-trimethylpiperazin-1-yl]benzoyl]-2,4-dihydro-1,3-benzoxazin-8-yl]-5-fluoro-2-(3-oxa-8-azabicyclo[3.2.1]octan-8-yl)benzoate

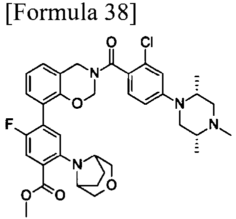

To the solution of 2-chloro-4-[(2R,5R)-2,4,5-trimethylpiperazin-1-yl]benzoyl chloride in acetonitrile obtained in the fourth step, the solution of methyl 4-(3,4-dihydro-2H-1,3-benzoxazin-8-yl)-5-fluoro-2-(3-oxa-8-azabicyclo[3.2.1]octan-8-yl)benzoate methanesulfonate in acetonitrile obtained in the third step was added. N,N-Diisopropylethylamine (16.0 mL, 92.0 mmol) was added thereto, and the mixture was stirred at 25°C for 1 hour. Ethyl acetate and water were added to the reaction solution, and water, a 15% aqueous sodium chloride solution, an 8 M aqueous sodium hydroxide solution, and 1 M hydrochloric acid were then added for extraction. The obtained organic layer was concentrated. 1 M hydrochloric acid and ethyl acetate were added to the obtained residue for back-extraction. An 8 M aqueous sodium hydroxide solution and 2 M hydrochloric acid were added to the obtained aqueous layer, followed by extraction with 4-methyltetrahydropyran. The organic layer was washed with a 15% aqueous sodium chloride solution, concentrated, and dried under reduced pressure to obtain the title compound (69%, 8.99 g).
LCMS: m/z 663[M+H]⁺
HPLC retention time: 0.86 min (analysis condition F)

### Sixth step

### Compound 5

### 4-[3-[2-Chloro-4-[(2R,5R)-2,4,5-trimethylpiperazin-1-yl]benzoyl]-2,4-dihydro-1,3-benzoxazin-8-yl]-5-fluoro-2-(3-oxa-8-azabicyclo[3.2.1]octan-8-yl)benzoic acid

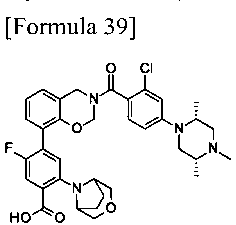

To methyl 4-[3-[2-chloro-4-[(2R,5R)-2,4,5-trimethylpiperazin-1-yl]benzoyl]-2,4-dihydro-1,3-benzoxazin-8-yl]-5-fluoro-2-(3-oxa-8-azabicyclo[3.2.1]octan-8-yl)benzoate (5.74 g, 8.66 mmol), tetrahydrofuran (29.3 mL), 4-methyltetrahydropyran (1.38 mL) and methanol (8.61 mL) were added, then a 50% aqueous potassium hydroxide solution (1.93 mL, 26.0 mmol) was added, and the mixture was stirred at 25°C for 3 hours. Water and cyclopentyl methyl ether were added to the reaction solution for back-extraction. 6 M hydrochloric acid was added to the obtained aqueous layer, and the mixture was washed with ethyl acetate. 2-Butanone and 8 M sodium hydroxide were added to the aqueous layer, and sodium chloride was added. The reaction mixture was subjected to extraction with 2-butanone. The obtained organic layer was concentrated, filtered, and washed with 2-butanone. The obtained filtrate and wash were concentrated, and acetone was added to the residue. Water was added to the obtained solution, and the mixture was stirred at 25°C. The obtained solid was collected by filtration and washed with water. The solid was dried to obtain the title compound (83%, 4.67 g).
LCMS: m/z 649[M+H]⁺
HPLC retention time: 1.13 min (analysis condition B)

### <Example 1-9>

### Compound 6

### 4-[3-[4-Chloro-6-(7,7-dimethyl-5,9-dioxa-2-azaspiro[3.5]nonan-2-yl)-2-methylpyridine-3-carbonyl]-2,4-dihydro-1,3-benzoxazin-8-yl]-5-fluoro-2-(3-oxa-8-azabicyclo[3.2.1]octan-8-yl)benzoic acid

### First step

### Compound 6-1

### 4,6-Dichloro-2-methylpyridine-3-carbonyl chloride

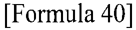

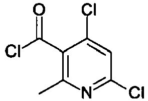

To a suspension of 4,6-dichloro-2-methylnicotinic acid (10.0 g, 48.5 mmol) in dichloromethane (194 mL), oxalyl chloride (5.00 mL, 58.2 mmol) and N,N-dimethylformamide (0.0380 mL, 0.485 mmol) were added, and the mixture was stirred at room temperature for 17 hours. Oxalyl chloride (5.00 mL, 58.2 mmol) was further added to the reaction solution, and the mixture was stirred at room temperature for 3 hours. The reaction solution was concentrated to obtain the title compound as a crude product.

### Second step

### Compound 6-2

### Methyl 4-[3-(4,6-dichloro-2-methylpyridine-3-carbonyl)-2,4-dihydro-1,3-benzoxazin-8-yl]-5-fluoro-2-(3-oxa-8-azabicyclo[3.2.1]octan-8-yl)benzoate

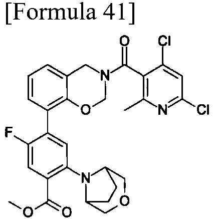

The title compound was obtained by the same operation as in the fifth step of compound B using methyl 4-(3,4-dihydro-2H-1,3-benzoxazin-8-yl)-5-fluoro-2-(3-oxa-8-azabicyclo[3.2.1]octan-8-yl)benzoate dihydrochloride and 4,6-dichloro-2-methylpyridine-3-carbonyl chloride. However, 4-methylmorpholine was used instead of N,N-diisopropylethylamine.
LCMS: m/z 586[M+H]⁺
HPLC retention time: 1.30 min (analysis condition F)

### Third step

### Compound 6-3

### Methyl 4-[3-[4-chloro-6-(7,7-dimethyl-5,9-dioxa-2-azaspiro[3.5]nonan-2-yl)-2-methylpyridine-3-carbonyl]-2,4-dihydro-1,3-benzoxazin-8-yl]-5-fluoro-2-(3-oxa-8-azabicyclo[3.2.1]octan-8-yl)benzoate

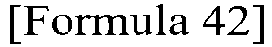

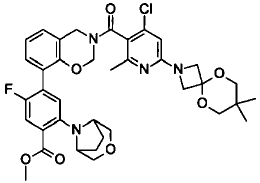

A suspension of methyl 4-[3-(4,6-dichloro-2-methylpyridine-3-carbonyl)-2,4-dihydro-1,3-benzoxazin-8-yl]-5-fluoro-2-(3-oxa-8-azabicyclo[3.2.1]octan-8-yl)benzoate (5.00 g, 7.93 mmol), 7,7-dimethyl-5,9-dioxa-2-azaspiro[3.5]nonane (1.31 g, 8.33 mmol), XantPhos Pd G3 (0.376 g, 0.396 mmol) and cesium carbonate (10.3 g, 31.7 mmol) in 1,4-dioxane (52.9 mL) was stirred overnight at 60°C. The reaction solution was cooled to room temperature. XantPhos Pd G3 (0.376 g, 0.396 mmol) was added thereto, and the mixture was stirred at 60°C for 8 hours. Water was added to the reaction solution, followed by extraction using ethyl acetate. The organic layer was washed with a saturated aqueous solution of sodium chloride, dried over magnesium sulfate, and filtered. The obtained residue was purified by silica gel column chromatography (hexane/ethyl acetate) to obtain the title compound (36%, 2.01 g).
LCMS: m/z 707[M+H]⁺
HPLC retention time: 1.39 min (analysis condition F)

### Fourth step

### Compound 6

### 4-[3-[4-Chloro-6-(7,7-dimethyl-5,9-dioxa-2-azaspiro[3.5]nonan-2-yl)-2-methylpyridine-3-carbonyl]-2,4-dihydro-1,3-benzoxazin-8-yl]-5-fluoro-2-(3-oxa-8-azabicyclo[3.2.1]octan-8-yl)benzoic acid

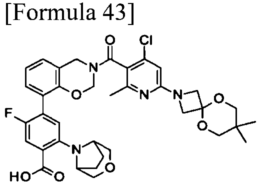

To a solution of methyl 4-[3-[4-chloro-6-(7,7-dimethyl-5,9-dioxa-2-azaspiro[3.5]nonan-2-yl)-2-methylpyridine-3-carbonyl]-2,4-dihydro-1,3-benzoxazin-8-yl]-5-fluoro-2-(3-oxa-8-azabicyclo[3.2.1]octan-8-yl)benzoate (5.97 g, 8.44 mmol) in tetrahydrofuran (16.9 mL) and methanol (16.9 mL), an 8 M aqueous potassium hydroxide solution (3.17 mL, 25.3 mmol) was added thereto, and the mixture was stirred at 50°C for 1.5 hours. An aqueous formic acid solution was added to the reaction solution, and the mixture was then purified by reverse phase column chromatography (acetonitrile/water, 0.1% formic acid) to obtain the title compound (91%, 5.33 g).
LCMS: m/z 693[M+H]⁺
HPLC retention time: 1.62 min and 1.65 min (analysis condition B)

### <Example 1-10>

### Compound 7

### (2S,3R)-3-[2-[2,6-Dichloro-4-(2-methoxyethoxy)benzoyl]-3,4-dihydro-1H-isoquinolin-5-yl]-2-methylbutanoic acid

### First step

### Compound 7-1

### Ethyl (E)-3-(3,4-dihydroisoquinolin-5-yl)but-2-enoate

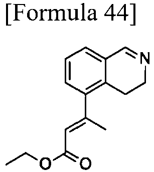

A solution of 5-bromo-3,4-dihydroisoquinoline (500 mg, 2.38 mmol), ethyl (E)-but-2-enoate (678 mg, 5.94 mmol), dichlorobis(triphenylphosphine)palladium(II) (175 mg, 0.250 mmol) and potassium carbonate (1.15 g, 8.32 mmol) in N,N-dimethylformamide (5.00 mL) was stirred at 120°C for 16 hours. The reaction solution was diluted with water, followed by extraction with ethyl acetate. The organic layer was dried over sodium sulfate and concentrated, and the obtained residue was purified by reverse phase column chromatography (acetonitrile/water, 0.1% formic acid) to obtain the title compound (35%, 200 mg).
LCMS: m/z 244[M+H]⁺
HPLC retention time: 1.22 min (analysis condition L)

### Second step

### Compound 7-2

### Ethyl 3-(1,2,3,4-tetrahydroisoquinolin-5-yl)butanoate

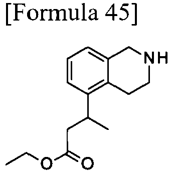

A solution of ethyl (E)-3-(3,4-dihydroisoquinolin-5-yl)but-2-enoate (100 mg, 0.410 mmol) and 10% palladium carbon (20.0 mg) in ethanol (10.0 mL) was stirred for 10 days in a hydrogen atmosphere. The reaction solution was filtered, and the filtrate was concentrated to obtain the title compound as a crude product.
LCMS: m/z 248[M+H]⁺
HPLC retention time: 1.35 min (analysis condition L)

### Third step

### Compound 7-3

### Ethyl 3-(1,2,3,4-tetrahydroisoquinolin-5-yl)butanoate hydrochloride

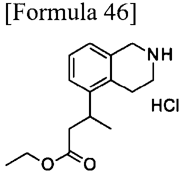

To a solution of ethyl 3-(1,2,3,4-tetrahydroisoquinolin-5-yl)butanoate (500 mg, 2.02 mmol) in ethyl acetate (20.0 mL), hydrochloric acid gas was bubbled at room temperature for 30 minutes. The obtained solid was collected by filtration to obtain the title compound as a crude product.
LCMS: m/z 248[M+H]⁺
HPLC retention time: 1.34 min (analysis condition K)

### Fourth step

### Compound 7-4

### Ethyl (3R)-3-(1,2,3,4-tetrahydroisoquinolin-5-yl)butanoate

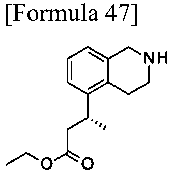

Ethyl 3-(1,2,3,4-tetrahydroisoquinolin-5-yl)butanoate hydrochloride (300 mg) was resolved into both stereoisomers by SFC (Chiralpak IBZ × 25 cm, 0.005 mm chiral-PCIB, hexane (0.2% isopropanol)/ethanol (0.2% isopropanol)) to obtain the title compound (47.9 mg) and its isomer (121.2 mg).
LCMS: 248[M+H]⁺
HPLC retention time: 1.36 min (analysis condition K)
SFC retention time: 3.19 min (isomer retention time: 4.90 min) (analysis condition: CHIRALPAK IB-3, 0.46 × 15 cm, 3 µm, hexane (0.2% isopropanol):ethanol = 70:30, 9.0 min), 25°C, 220 nm)

### Fifth step

### Compound 7-5

### tert-Butyl 5-[(2R)-4-ethoxy-4-oxobutan-2-yl]-3,4-dihydro-1H-isoquinoline-2-carboxylate

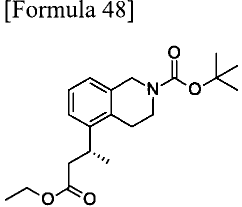

Ethyl (3R)-3-(1,2,3,4-tetrahydroisoquinolin-5-yl)butanoate (29.0 mg, 0.117 mmol), di-tert-butyl dicarbonate (30.7 mg, 0.141 mmol) and triethylamine (0.0245 mL, 0.176 mmol) in dichloromethane (0.391 mL) were stirred at room temperature for 3 hours. The reaction solution was poured to 1 M hydrochloric acid, followed by extraction with ethyl acetate. The organic layer was washed with a saturated aqueous solution of sodium bicarbonate, followed by passing through a phase separator. Then, a residue obtained by concentration was purified by silica gel column chromatography (hexane/ethyl acetate) to obtain the title compound (74%, 30.2 mg).
LCMS: m/z 248[M+H-tBu]⁺
HPLC retention time: 1.40 min (analysis condition I)

### Sixth step

### Compound 7-6

### (R)-3-(2-(tert-Butoxycarbonyl)-1,2,3,4-tetrahydroisoquinolin-5-yl)butanoic acid

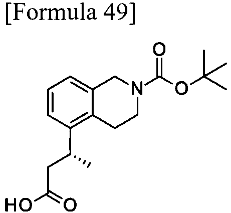

To a solution of tert-butyl 5-[(2R)-4-ethoxy-4-oxobutan-2-yl]-3,4-dihydro-1H-isoquinoline-2-carboxylate (416 mg, 1.20 mmol) in tetrahydrofuran (2.99 mL) and methanol (2.99 mL), a 5 M aqueous sodium hydroxide solution (0.718 mL, 1.13 mmol) was added, and the mixture was stirred at room temperature for 2 hours. An aqueous hydrochloric acid solution was added to the reaction solution, followed by extraction with ethyl acetate. The organic layer was washed with a saturated aqueous solution of sodium hydroxide, followed by passing through a phase separator. Then, the organic layer was concentrated to obtain the title compound as a crude product.
LCMS: m/z 342[M+Na]⁺
HPLC retention time: 1.17 min (analysis condition I)

### Seventh step

### Compound 7-7

### tert-Butyl 5-((R)-4-((S)-4-isopropyl-2-oxooxazolidin-3-yl)-4-oxobutan-2-yl)-3,4-dihydroisoquinoline-2(1H)-carboxylate

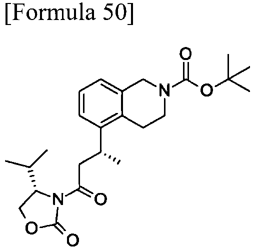

A solution of the crude product of (R)-3-(2-(tert-butoxycarbonyl)-1,2,3,4-tetrahydroisoquinolin-5-yl)butanoic acid and triethylamine (0.499 mL, 3.59 mmol) in tetrahydrofuran (4.79 mL) was cooled to 0°C. Pivaloyl chloride (0.162 mL, 1.32 mmol) was added thereto, and the mixture was stirred at 0°C for 1 hour. (S)-4-Isopropyl-2-oxazolidinone (170 mg, 1.32 mmol) and lithium chloride (66.0 mg, 1.56 mmol) were added thereto at 0°C, and the mixture was stirred for 1 hour while warmed to room temperature. 1 M hydrochloric acid and methanol were added to the reaction solution at 0°C, and the mixture was concentrated and then purified by reverse-phase column chromatography (acetonitrile/water, 0.1% formic acid) to obtain the title compound (95%, 490 mg).
LCMS: m/z 453[M+Na]⁺
HPLC retention time: 1.41 min (analysis condition I)

### Eighth step

### Compound 7-8

### tert-Butyl 5-((2R,3S)-4-((S)-4-isopropyl-2-oxooxazolidin-3-yl)-3-methyl-4-oxobutan-2-yl)-3,4-dihydroisoquinoline-2(1H)-carboxylate

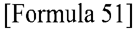

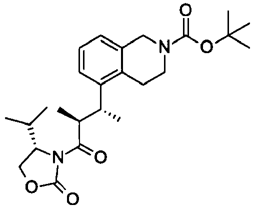

A solution of tert-butyl 5-((R)-4-((S)-4-isopropyl-2-oxooxazolidin-3-yl)-4-oxobutan-2-yl)-3,4-dihydroisoquinoline-2(1H)-carboxylate (365 mg, 0.848 mmol) in tetrahydrofuran (4.24 mL) was cooled to -78°C. 1 M sodium bis(trimethylsilyl)amide (1.10 mL, 1.10 mmol) was added dropwise thereto, and the mixture was stirred for 1 hour. Iodomethane (0.158 mL, 2.54 mmol) was added thereto, and the mixture was stirred for 1 hour and then further stirred at -40°C for 2 hours. A saturated aqueous solution of ammonium chloride was added to the reaction solution, followed by extraction with ethyl acetate. The organic layer was washed with a saturated aqueous solution of sodium bicarbonate and saturated saline, followed by passing through a phase separator. The organic layer was concentrated and then purified by reverse-phase column chromatography (acetonitrile/water, 0.1% formic acid) to obtain the title compound (70%, 263 mg).
LCMS: m/z 467[M+Na]⁺
HPLC retention time: 1.51 min (analysis condition G)

### Nineth step

### Compound 7-9

### (4S)-3-[(2S,3R)-2-Methyl-3-(1,2,3,4-tetrahydroisoquinolin-5-yl)butanoyl]-4-propan-2-yl-1,3-oxazolidin-2-one hydrochloride

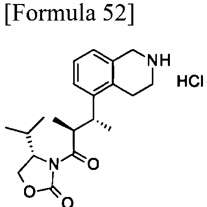

To tert-butyl 5-[(2R)-4-ethoxy-4-oxobutan-2-yl]-3,4-dihydro-1H-isoquinoline-2-carboxylate (228 mg, 0.513 mmol), a 4 M solution of hydrochloric acid in 1,4-dioxane (5.00 ml, 20.0 mmol) was added, and the mixture was stirred at room temperature for 1 hour. The reaction solution was concentrated to obtain the title compound as a crude product.
LCMS: m/z 345[M+H]⁺
HPLC retention time: 0.73 min (analysis condition G)

### Tenth step

### Compound 7-10

### (4S)-3-[(2S,3R)-3-[2-[2,6-Dichloro-4-(2-methoxyethoxy)benzoyl]-3,4-dihydro-1H-isoquinolin-5-yl]-2-methylbutanoyl]-4-propan-2-yl-1,3-oxazolidin-2-one

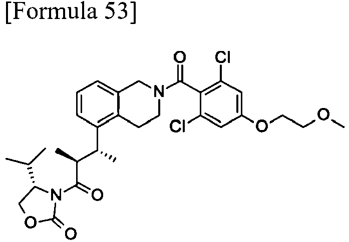

To a solution of 2,6-dichloro-4-(2-methoxyethoxy)benzoic acid (119 mg, 0.448 mmol) in dichloromethane (2.00 ml), oxalyl chloride (0.0710 ml, 0.814 mmol) and N,N-dimethylformamide (0.00315 ml, 0.0410 mmol) were added, and the mixture was stirred at room temperature for 1 hour. The reaction solution was concentrated and azeotroped with toluene to obtain 2,6-dichloro-4-(2-methoxyethoxy)benzoyl chloride as a crude product. A solution of (4S)-3-[(2S,3R)-2-methyl-3-(1,2,3,4-tetrahydroisoquinolin-5-yl)butanoyl]-4-propan-2-yl-1,3-oxazolidin-2-one hydrochloride (155 mg, 0.407 mmol) in dichloromethane (2.00 ml) was cooled to 0°C. 2,6-Dichloro-4-(2-methoxyethoxy)benzoyl chloride and triethylamine (0.170 ml, 1.22 mmol) were added thereto, and the mixture was stirred at room temperature for 30 minutes. The reaction solution was poured to 1 M hydrochloric acid, followed by extraction with ethyl acetate. The organic layer was washed with a saturated aqueous solution of sodium bicarbonate, followed by passing through a phase separator. Then, the organic layer was concentrated to obtain the title compound as a crude product.
LCMS: m/z 591[M+H]⁺
HPLC retention time: 1.39 min (analysis condition G)

### Eleventh step

### Compound 7

### (2S,3R)-3-[2-[2,6-Dichloro-4-(2-methoxyethoxy)benzoyl]-3,4-dihydro-1H-isoquinolin-5-yl]-2-methylbutanoic acid

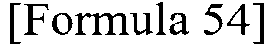

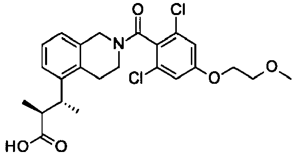

A solution of (4S)-3-[(2S,3R)-3-[2-[2,6-dichloro-4-(2-methoxyethoxy)benzoyl]-3,4-dihydro-1H-isoquinolin-5-yl]-2-methylbutanoyl]-4-propan-2-yl-1,3-oxazolidin-2-one (241 mg, 0.407 mmol) in tetrahydrofuran (2.17 ml) and water (0.543 ml) was cooled to 0°C. 34.5% hydrogen peroxide water (0.281 ml, 2.85 mmol) and lithium hydroxide monohydrate (51.2 mg, 1.22 mmol) were added thereto, and the mixture was stirred at room temperature for 3 hours. Methanol and formic acid were added to the reaction solution, and the mixture was purified by reverse phase column chromatography (acetonitrile/water, 0.1% formic acid) to obtain the title compound (96%, 187 mg).
LCMS: m/z 480[M+H]⁺
HPLC retention time: 1.07 min and 1.09 min (analysis condition C)

### [Example 2] Method for producing crystals

### (Example 2-1-1)

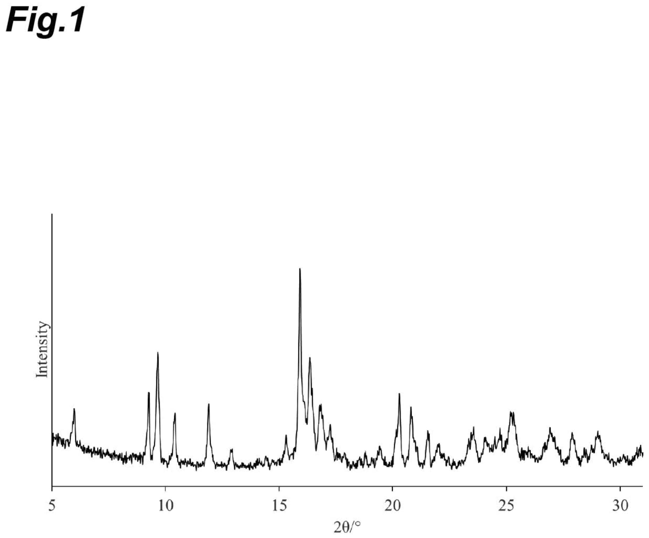

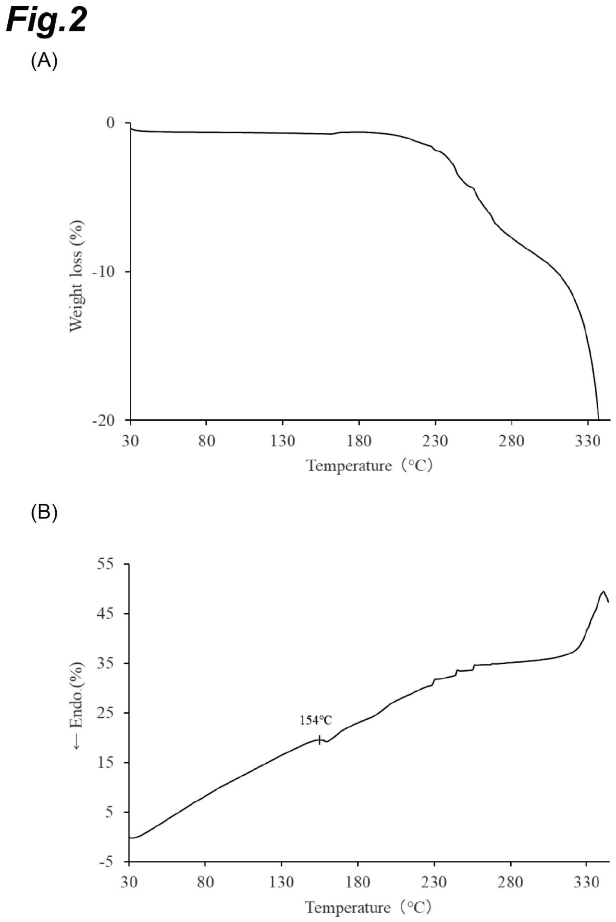

To compound 1 (28.9 mg), 120 µL of tetrahydrofuran was added, and the compound was dissolved at room temperature. To the solution, a mixed solution of methanol/water = 9/1 (v/v) was added (480 µL), and the mixture was stirred for 20 minutes. Further, a mixed solution of methanol/water = 9/1 (v/v) was added (600 µL) thereto, and the mixture was stirred for 4 hours to obtain a suspension. The suspension was filtered and vacuum-dried for 24 hours to obtain 18.8 mg of a hydrate 1A-type crystal powder. Results of powder X-ray diffractometry of this powder are shown in Figure 1, and typical peaks are shown in Table 2 below. Results of TG-DTA measurement are shown in Figure 2.

**[Table 2]**

| 2 *θ* (° ) |
|---|
| 6.0 |
| 9.3 |
| 9.7 |
| 10.4 |
| 11.9 |
| 12.9 |
| 15.3 |
| 15.9 |
| 16.4 |
| 16.8 |

### (Example 2-1-2)

To compound 1 (10.8 mg), a mixed solution of ethanol/water = 1/3 (v/v) was added (50 µL), and the mixture was shaken at 25°C for 13 days to obtain crystals. The obtained crystals were confirmed to be hydrate 1G-type crystals by powder X-ray diffraction.

### (Example 2-1-3)

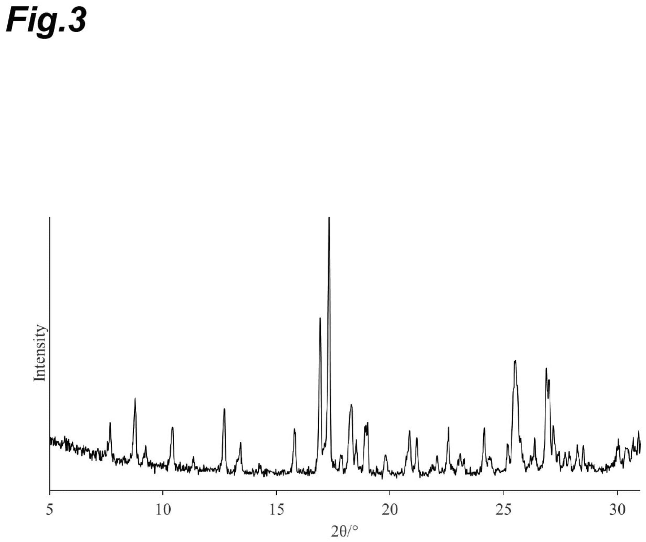

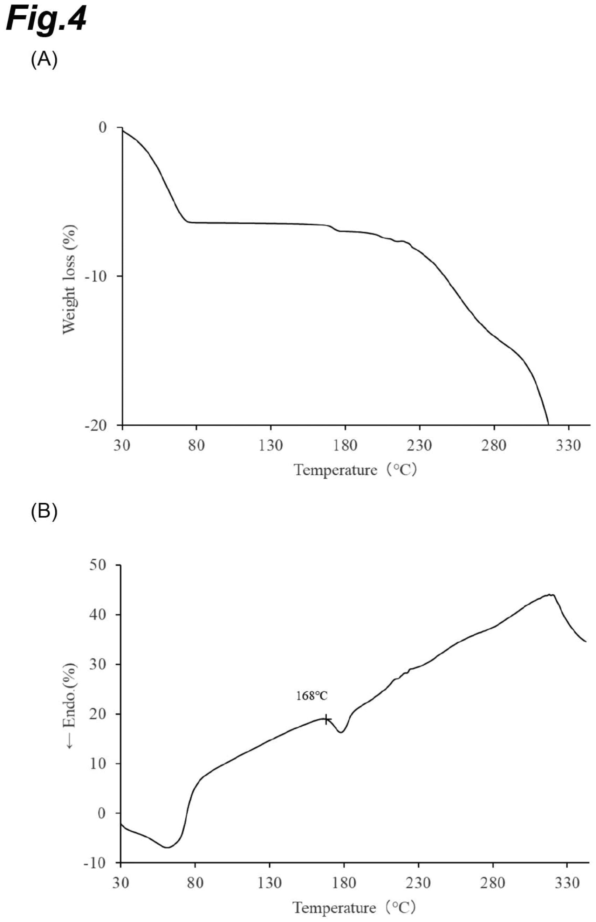

To compound 1 (30.3 mg), 120 µL of tetrahydrofuran was added, and the compound was dissolved at room temperature. To the solution, 120 µL of water was added, and approximately 0.1 mg of the crystals obtained in Example 2-1-2 was added as seed crystals. Further, 60 µL of water was added thereto 30 minutes later. Further, 60 µL of water was added thereto 130 minutes later. Further, 120 µL of water was added thereto 50 minutes later. Further, 120 µL of water was added thereto 150 minutes later. Further, 240 µL of water was added thereto 80 minutes later. The solution was filtered 20 hours later and dried at room temperature for 24 hours to obtain 27.8 mg of a hydrate 1G-type crystal powder. Results of powder X-ray diffractometry of this powder are shown in Figure 3, and typical peaks are shown in Table 3 below. Results of TG-DTA measurement are shown in Figure 4.

**[Table 3]**

| 2 *θ* (° ) |
|---|
| 8.8 |
| 9.2 |
| 10.4 |
| 12.7 |
| 13.4 |
| 15.8 |
| 16.9 |
| 17.3 |
| 18.3 |
| 18.9 |

### (Example 2-1-4)

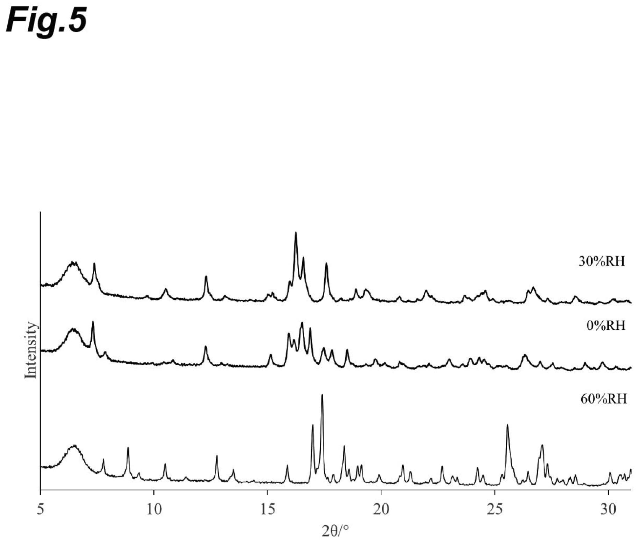

1G-type crystals were preserved at 34°C at a relative humidity of 60% for approximately 5 minutes and subjected to powder X-ray diffractometry. Then, the relative humidity was changed to 0%, and the crystals were preserved for approximately 60 minutes and subjected to powder X-ray diffractometry. From the obtained results, it was confirmed that the 1G-type crystals were transformed to non-solvate 1H-type crystals by changing the relative humidity from 60% to 0%. The relative humidity was further changed to 30%, and the crystals were preserved for approximately 15 minutes and subjected to powder X-ray diffractometry. From the obtained results, it was confirmed that the 1H-type crystals were further transformed to hydrate 1J-type crystals by changing the relative humidity from 0% to 30%. These results of powder X-ray diffractometry are shown in Figure 5, and typical peaks at each humidity are shown in Table 4 below.

**[Table 4]**

| 2 *θ* (° ) | | |
|---|---|---|
| Relative humidity 0% | Relative humidity 30% | Relative humidity 60% |
| 12.3 | 9.7 | 8.9 |
| 15.1 | 10.5 | 9.3 |
| 15.9 | 13.1 | 10.5 |
| 16.2 | 15.0 | 12.8 |
| 16.5 | 16.2 | 13.5 |
| 16.9 | 16.6 | 15.9 |
| 17.5 | 17.6 | 17.0 |
| 17.8 | 18.9 | 17.4 |
| 18.5 | 19.3 | 18.4 |
| 19.7 | 20.8 | 19.0 |

### (Example 2-1-5)

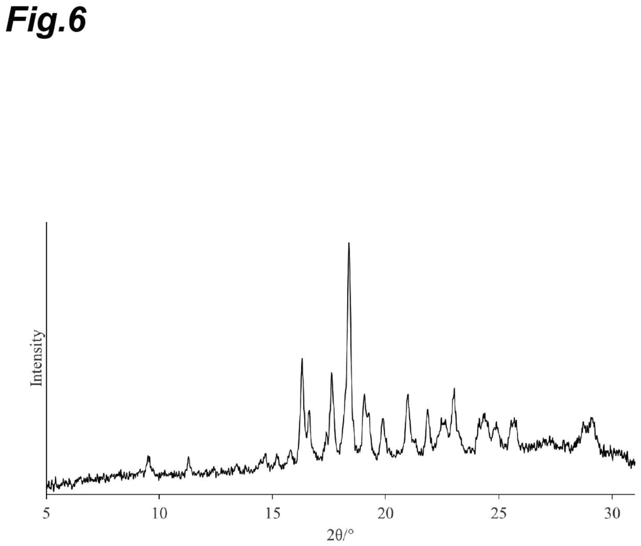

To compound 1 (5.6 mg), acetone (15 µL) was added, and the mixture was stirred. Acetone was added (35 µL) thereto 5 minutes later. The suspension was filtered to obtain 1K-type crystals. Results of powder X-ray diffraction are shown in Figure 6, and typical peaks are shown in Table 5 below.

**[Table 5]**

| 2 *θ* (° ) |
|---|
| 9.5 |
| 11.3 |
| 16.3 |
| 16.6 |
| 17.6 |
| 18.4 |
| 19.1 |
| 19.9 |
| 21.0 |
| 21.9 |

### (Example 2-1-6)

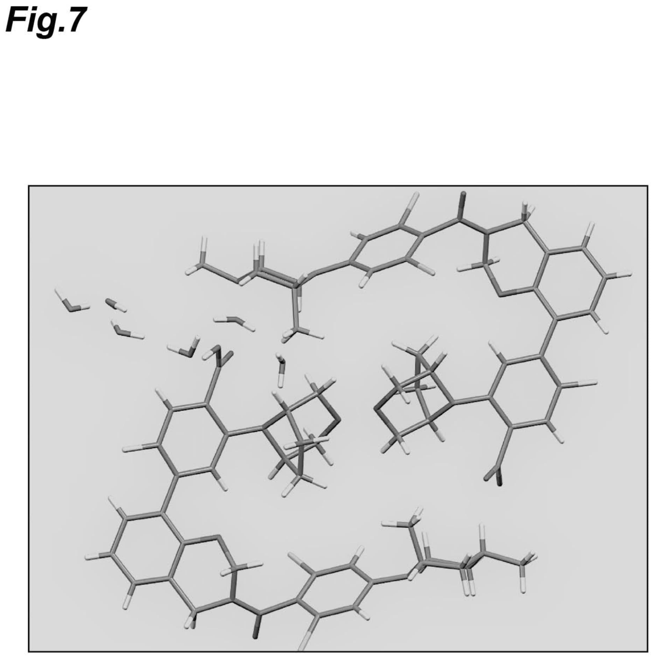

To compound 1 (10.5 mg), dimethylformamide (40 µL) was added, and the compound was dissolved at 80°C. To the solution, water (20 µL) was added, then 1G-type crystals (0.1 mg) were added as seed crystals, and the mixture was cooled to room temperature. Then, after heating to 80°C, water (10 µL) was added thereto, and the mixture was cooled to room temperature. Then, the mixture was further heated to 80°C and cooled to room temperature. After further heating to 80°C, water (5 µL) was added thereto, and the mixture was cooled to room temperature. Then, the mixture was further heated to 80°C and cooled to room temperature. The obtained crystals were subjected to X-ray crystal structure analysis. The obtained crystal structure of the 1G-type crystals is shown in Figure 7. Parameters characterizing the crystals are shown in Table 6 below.

**[Table 6]**

| | |
|---|---|
| Crystal system | Triclinic |
| Space group | *P*1 |
| a [Å] | 10.5287 |
| b [Å] | 12.1052 |
| c [Å] | 14.7418 |
| *α* [° ] | 72.949 |
| *β* [° ] | 69.845 |
| *γ* [° ] | 80.445 |

### (Example 2-2-1)

To compound 4 (3 mg), 2-propanol was added (15 µL), and the mixture was shaken for 6 days to obtain crystals. The obtained crystals were confirmed to be non-solvate 2A-type crystals by powder X-ray diffraction.

### (Example 2-2-2)

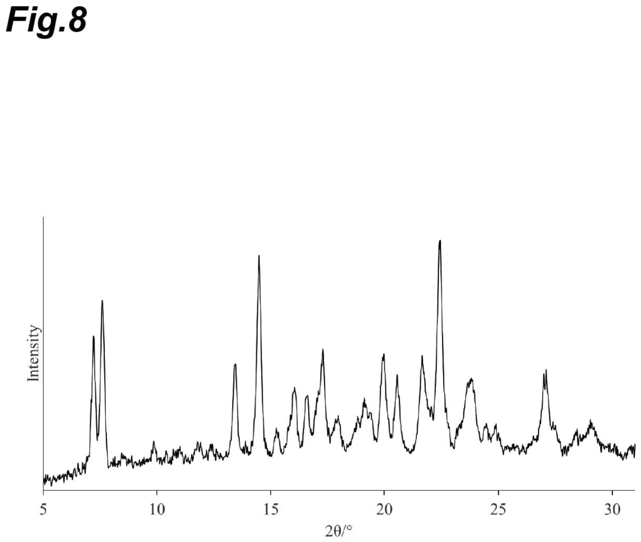

To compound 4 (3 mg), ethanol was added (15 µL) and the mixture was shaken for 6 days to obtain non-solvate 2B-type crystals. Results of powder X-ray diffraction are shown in Figure 8, and typical peaks are shown in Table 7 below.

**[Table 7]**

| 2 *θ* (° ) |
|---|
| 7.2 |
| 7.6 |
| 13.4 |
| 14.5 |
| 16.0 |
| 16.6 |
| 17.3 |
| 19.9 |
| 20.6 |
| 22.4 |

### (Example 2-2-3)

To compound 4 (approximately 5 mg), acetonitrile (100 µL) was added, and the compound was dissolved under a condition of heating to reflux. Then, the solution was cooled to room temperature to obtain crystals. The obtained crystals were confirmed to be non-solvate 2F-type crystals by powder X-ray diffraction.

### (Example 2-2-4)

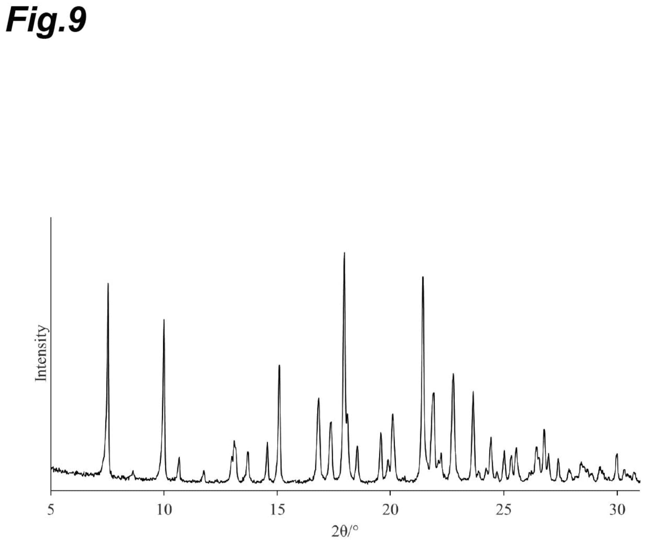

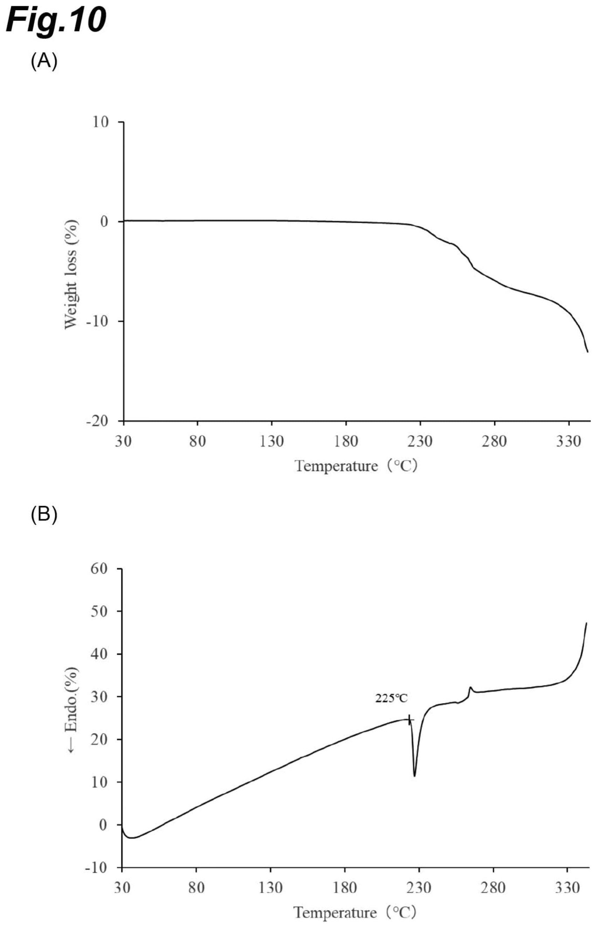

To compound 4 (146.8 mg), DMSO (1 mL) was added, and the compound was dissolved at 120°C. The solution was cooled to room temperature. To the solution, 2-propanol (5 mL) was added, and the crystals (approximately 0.1 mg) obtained in Example 2-2-1 were further added as seed crystals. Further, 2-propanol (10 mL) was added thereto, and the mixture was stirred for 18 hours and then filtered. The obtained solid was washed with 2-propanol (2 mL). The solid was vacuum-dried for 1 day to obtain 125.3 mg of non-solvate 2A-type crystals. Results of powder X-ray diffractometry are shown in Figure 9, and typical peaks are shown in Table 8 below. Results of TG-DTA measurement are shown in Figure 10.

**[Table 8]**

| 2 *θ* (° ) |
|---|
| 7.5 |
| 10.0 |
| 10.7 |
| 13.7 |
| 14.6 |
| 15.1 |
| 16.8 |
| 17.4 |
| 18.0 |
| 18.5 |

### (Example 2-2-5)

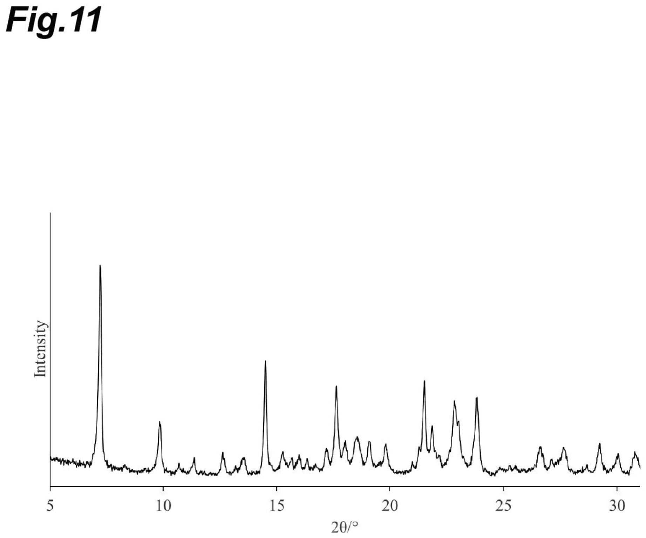

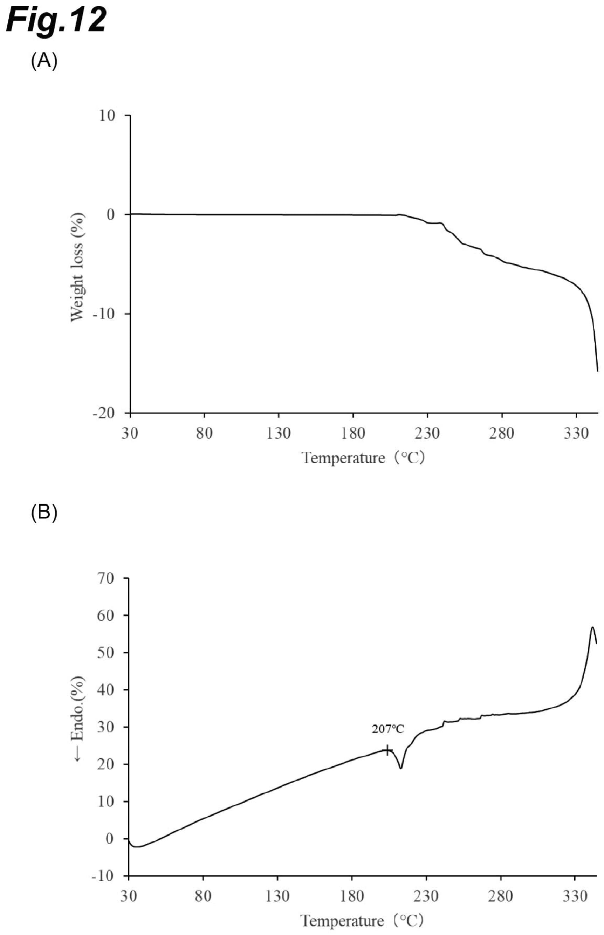

To compound 4 (105.8 mg), acetonitrile (5 mL) was added, and the compound was dissolved by heating to reflux. The solution was cooled to room temperature. This solution was added dropwise to water (15 mL), and the mixture was stirred overnight to obtain 92.2 mg of non-solvate 2C-type crystals. Results of powder X-ray diffractometry are shown in Figure 11, and typical peaks are shown in Table 9 below. Results of TG-DTA measurement are shown in Figure 12.

**[Table 9]**

| 2 *θ* (° ) |
|---|
| 7.2 |
| 9.9 |
| 14.5 |
| 15.3 |
| 15.7 |
| 17.2 |
| 17.6 |
| 18.0 |
| 19.1 |
| 19.8 |

### (Example 2-2-6)

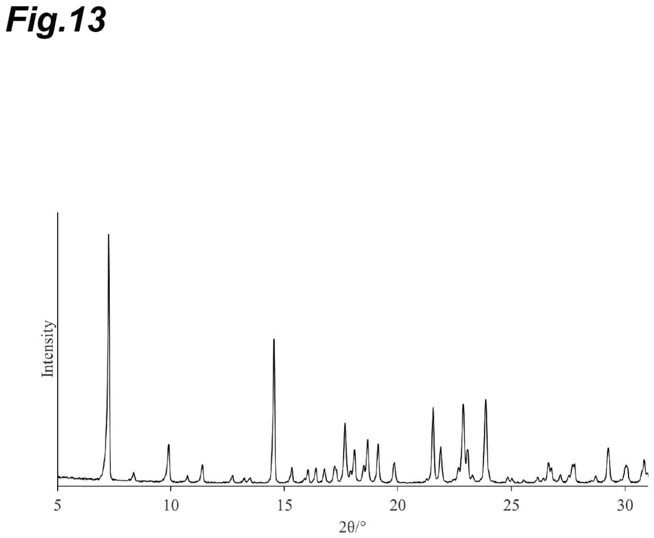

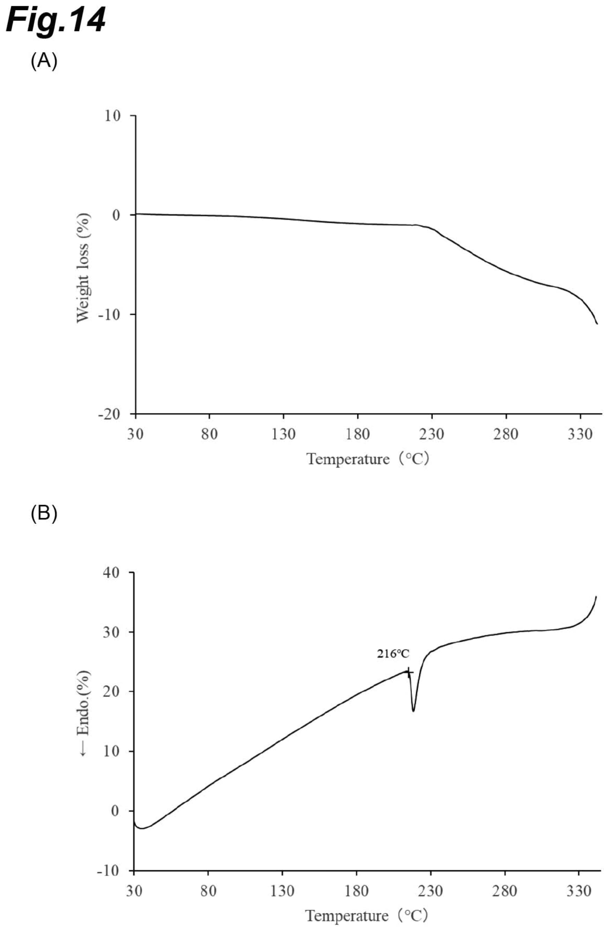

To compound 4 (29.4 mg), acetonitrile (900 µL) was added, and the compound was dissolved under a condition of heating to reflux. Then, the solution was cooled to room temperature. The crystals (approximately 0.1 mg) obtained in Example 2-2-3 were added thereto as seed crystals, and the mixture was stirred for 3 hours. After filtration, the obtained powder was vacuum-dried to obtain 18.9 mg of 2F-type crystals. Results of powder X-ray diffractometry are shown in Figure 13, and typical peaks are shown in Table 10 below. Results of TG-DTA measurement are shown in Figure 14.

**[Table 10]**

| 2 *θ* (° ) |
|---|
| 7.3 |
| 9.9 |
| 11.4 |
| 14.5 |
| 15.3 |
| 16.4 |
| 16.8 |
| 17.2 |
| 17.7 |
| 18.1 |

### (Example 2-3-1)

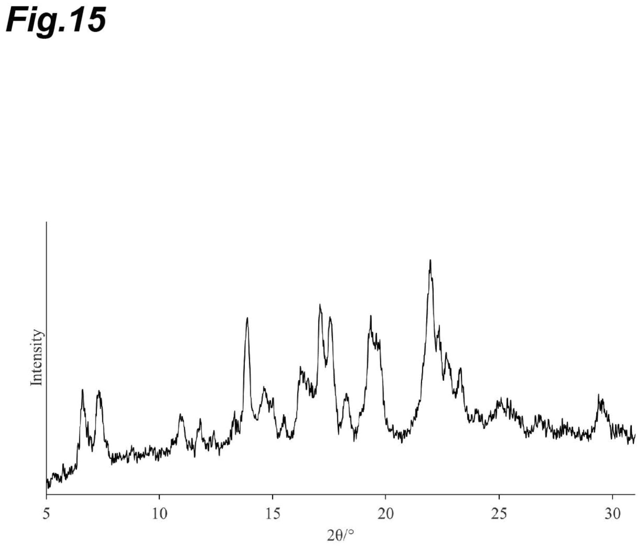

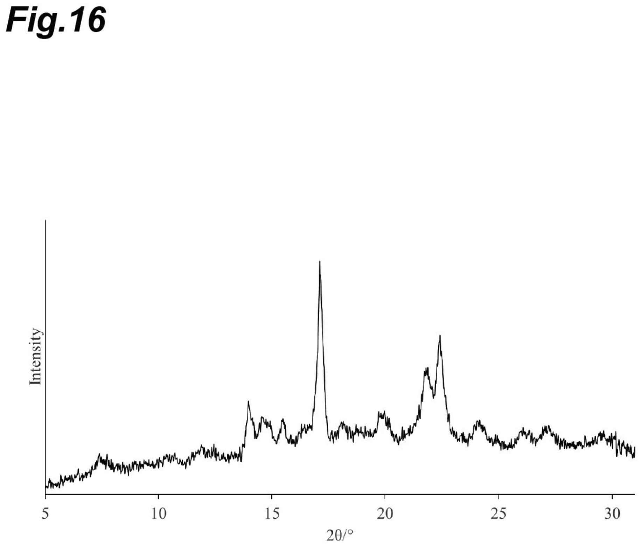

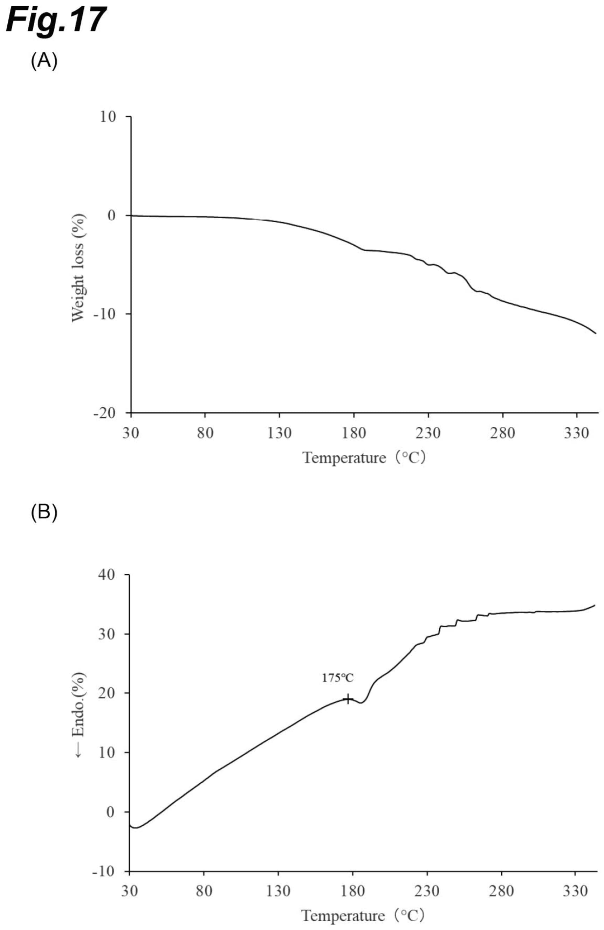

To compound 3 (approximately 30 mg), ethyl acetate (300 µL) was added. A portion of the suspension was filtered 30 minutes later to obtain 3D-type crystals. Results of powder X-ray diffractometry are shown in Figure 15. The remaining suspension was further stirred for 15 minutes and then filtered, and the obtained solid was vacuum-dried overnight to obtain 3B-type crystals. Results of powder X-ray diffractometry are shown in Figure 16. Results of TG-DTA measurement are shown in Figure 17. Typical peaks at each run of powder X-ray diffractometry are shown in Table 11 below.

**[Table 11]**

| 2 θ (° ) | |
|---|---|
| 3D-type crystals | 3B-type crystals |
| 6.6 | 7.4 |
| 7.3 | 14.0 |
| 10.9 | 14.6 |
| 11.8 | 15.5 |
| 13.9 | 17.1 |
| 17.1 | 18.1 |
| 17.5 | 19.8 |
| 18.3 | 21.8 |
| 19.3 | 22.4 |
| 22.0 | 24.1 |

### (Example 2-3-2)

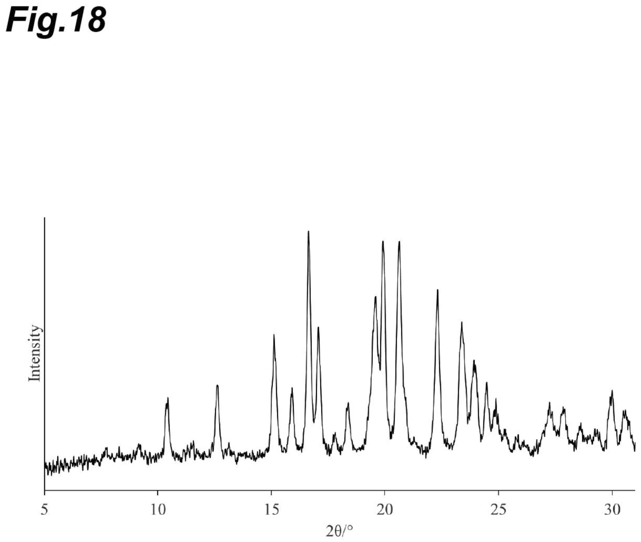

To compound 3 (29.4 mg), ethanol (300 µL) was added, and the compound was dissolved under heating to reflux. The solution was cooled to room temperature and stirred for 16 hours. Then, the suspension was filtered and subjected to powder X-ray diffractometry. The obtained powder was vacuum-dried to obtain non-solvate 3A-type crystals. Results of powder X-ray diffractometry are shown in Figure 18, and typical peaks are shown in Table 12 below.

**[Table 12]**

| 2 *θ* (° ) |
|---|
| 10.4 |
| 12.6 |
| 15.1 |
| 15.9 |
| 16.6 |
| 17.1 |
| 18.4 |
| 19.6 |
| 19.9 |
| 20.6 |

### (Example 2-3-3)

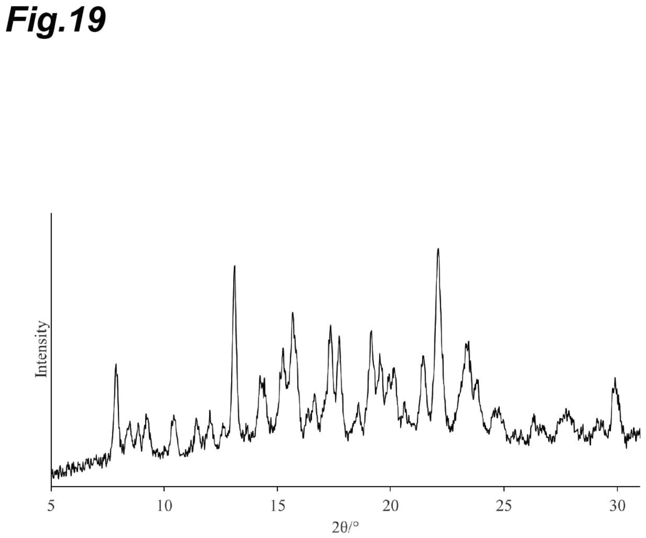

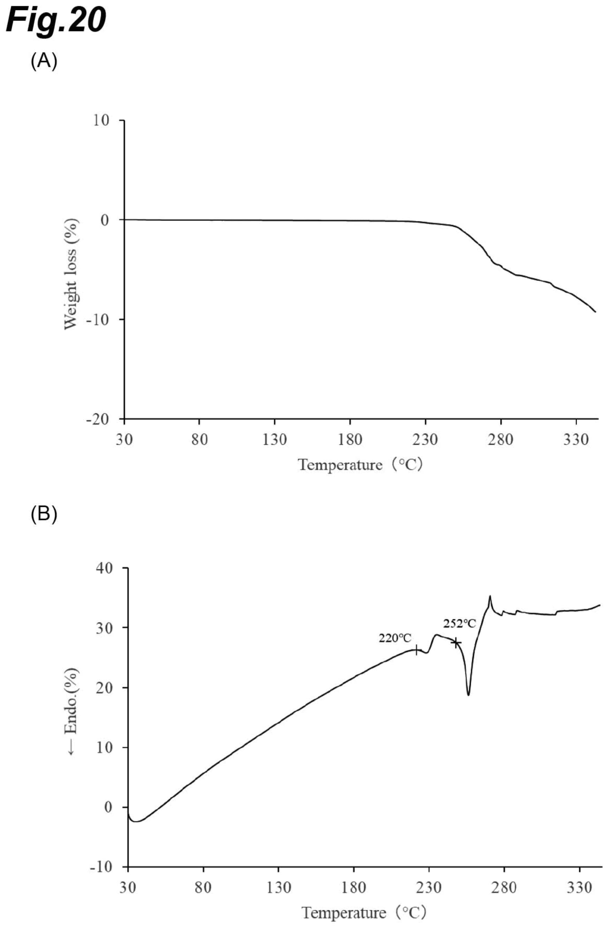

To compound 3 (approximately 30 mg), methanol (300 µL) was added, and the mixture was stirred at room temperature for approximately 30 minutes. A portion of the obtained suspension was filtered and subjected to powder X-ray diffractometry. The remaining portion was also filtered to isolate a solid, which was then vacuum-dried for 1 day to obtain non-solvate 3C-type crystals. Results of powder X-ray diffractometry are shown in Figure 19, and typical peaks are shown in Table 13 below. Results of TG-DTA measurement are shown in Figure 20.

**[Table 13]**

| 2 *θ* (° ) |
|---|
| 7.9 |
| 8.5 |
| 8.8 |
| 9.2 |
| 10.4 |
| 11.4 |
| 12.0 |
| 13.1 |
| 15.2 |
| 15.7 |

### (Example 2-4-1)

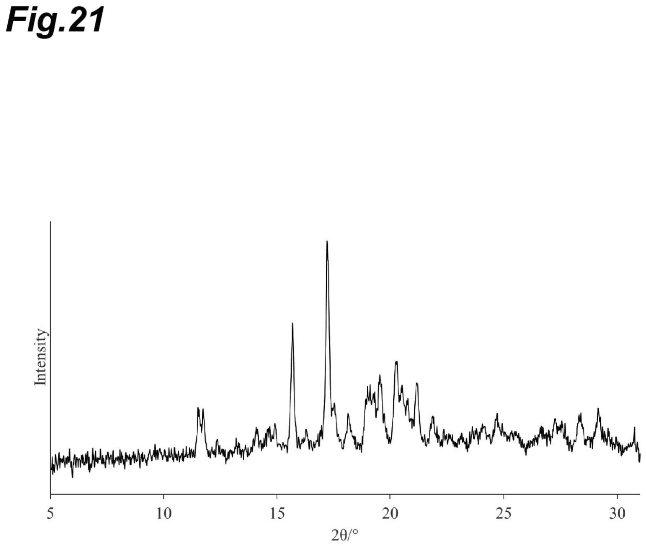

To compound 6 (3 mg), methyl isobutyl ketone (15 µL) was added, and the mixture was shaken for 3 days. Heptane (15 µL) was further added thereto, and the mixture was shaken for 5 days to obtain 4A-type crystals. Results of powder X-ray diffractometry are shown in Figure 21, and typical peaks are shown in Table 14 below.

**[Table 14]**

| 2 *θ* (° ) |
|---|
| 11.5 |
| 11.8 |
| 15.7 |
| 17.2 |
| 17.5 |
| 18.1 |
| 19.5 |
| 20.3 |
| 20.5 |
| 21.2 |

### (Example 2-4-2)

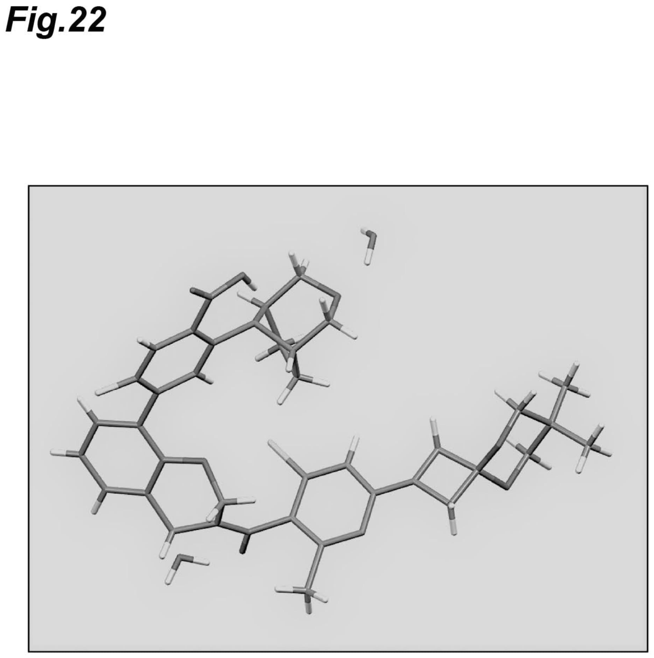

To compound 6 (3 mg), water/acetonitrile = 3/1 (v/v) (15 µL) was added, and the mixture was shaken for 8 days to obtain hydrate 4B-type crystals. Results of single-crystal X-ray crystal structure analysis are shown in Figure 22. Parameters characterizing the crystals are shown in Table 15 below.

**[Table 15]**

| | |
|---|---|
| Crystal system | Monoclinic |
| Space group | *P*2₁/*n* |
| a [Å] | 13.2678 |
| b [Å] | 19.2646 |
| c [Å] | 14.3459 |
| α [° ] | 90 |
| β [° ] | 111.028 |
| γ [° ] | 90 |

### (Example 2-4-3)

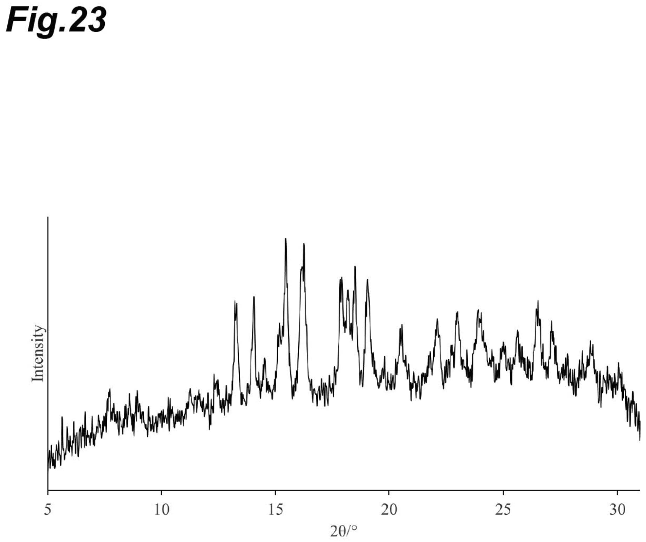

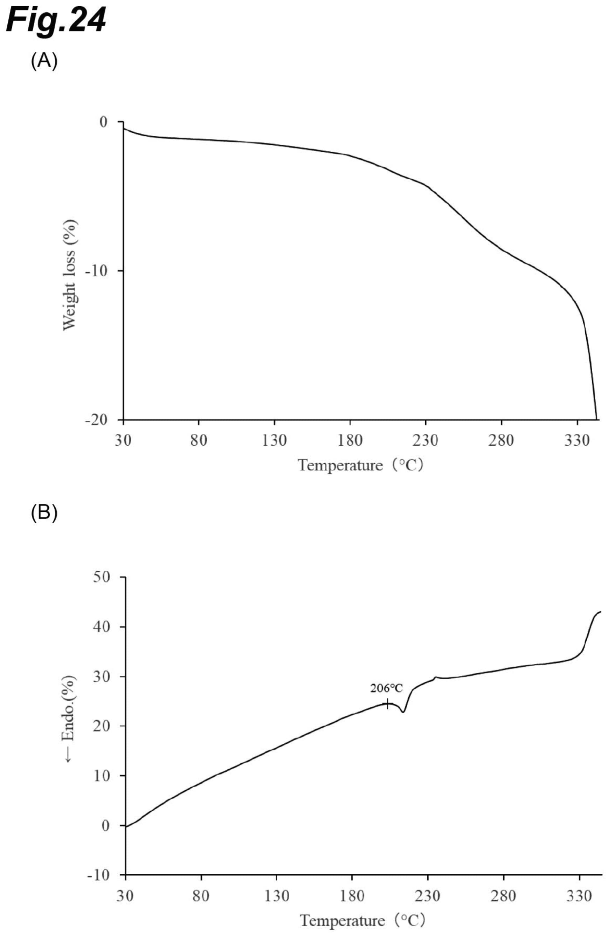

To compound 6 (50.1 mg), acetone (200 µL) was added, and the mixture was heated to 60°C. After cooling to room temperature, water (50 µL) was added thereto. Further, water (100 µL) was added thereto 5 minutes later. Further, water (250 µL) was added thereto 10 minutes later. Further, water (400 µL) was added thereto 5 minutes later. Further, water (1200 µL) was added thereto 5 minutes later. The suspension was filtered 15 minutes later and subjected to powder X-ray diffractometry. The solid was dried at room temperature under ordinary pressure for 15 hours to obtain 44.9 mg of 4B-type crystals. Results of powder X-ray diffractometry are shown in Figure 23, and typical peaks are shown in Table 16 below. Results of TG-DTA measurement are shown in Figure 24.

**[Table 16]**

| 2 *θ* (° ) |
|---|
| 7.7 |
| 13.3 |
| 14.0 |
| 15.4 |
| 16.2 |
| 17.9 |
| 18.2 |
| 18.5 |
| 19.1 |
| 20.6 |

### (Example 2-5-1)

To compound 5 (5.4 mg), PEG400 (15 µL) was added, and the mixture was heated to 80°C. The mixture was stirred overnight, and water (5 µL) was added thereto. The mixture was further cooled to room temperature 7 hours later and stirred for 3 days to obtain crystals. The obtained crystals were confirmed to be hydrate 5A-type crystals by powder X-ray diffraction.

### (Example 2-5-2)

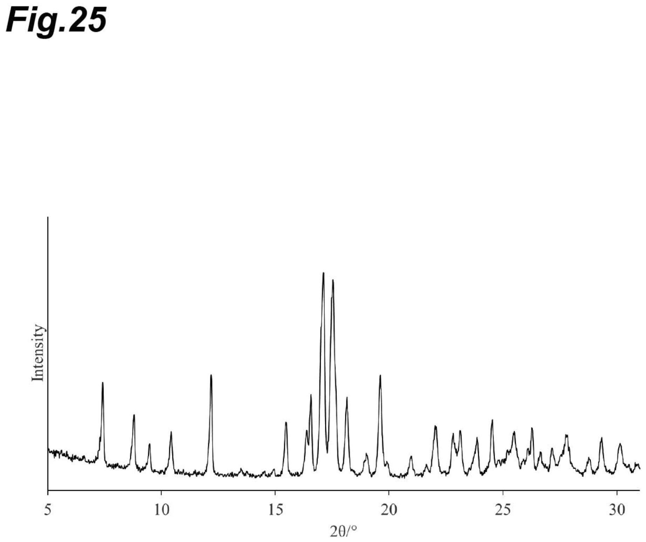

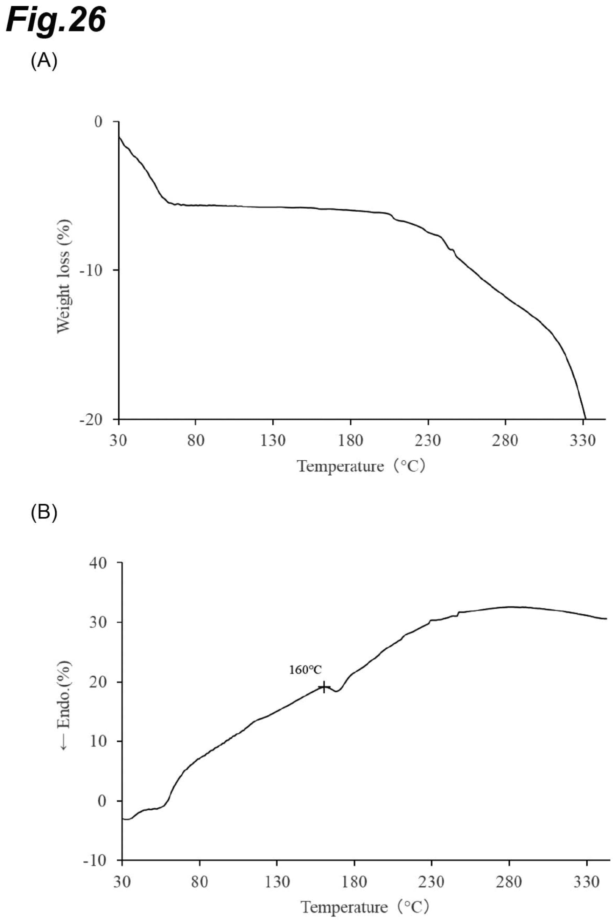

Compound 5 (30.6 mg) was dissolved by the addition of acetone/methyl ethyl ketone = 1/2 (v/v) (120 µL). To the solution, water (120 µL) was added, then the crystals (approximately 0.1 mg) obtained in Example 2-5-1 were further added as seed crystals, and the mixture was stirred for 55 minutes. Water (120 µL) was added thereto, and the mixture was stirred for 210 minutes. Further, water (120 µL) was added thereto, and the mixture was stirred for 70 minutes, followed by the addition of water (240 µL). The mixture was further stirred for 15 hours, and the suspension was then filtered and dried at room temperature under ordinary pressure for 7 hours to obtain 5A-type crystals (26.7 mg). Results of powder X-ray diffractometry are shown in Figure 25, and typical peaks are shown in Table 17 below. Results of TG-DTA measurement are shown in Figure 26.

**[Table 17]**

| 2 θ (° ) |
|---|
| 7.4 |
| 8.8 |
| 9.5 |
| 10.4 |
| 12.2 |
| 15.5 |
| 16.6 |
| 17.1 |
| 17.5 |
| 18.1 |

### (Example 2-5-3)

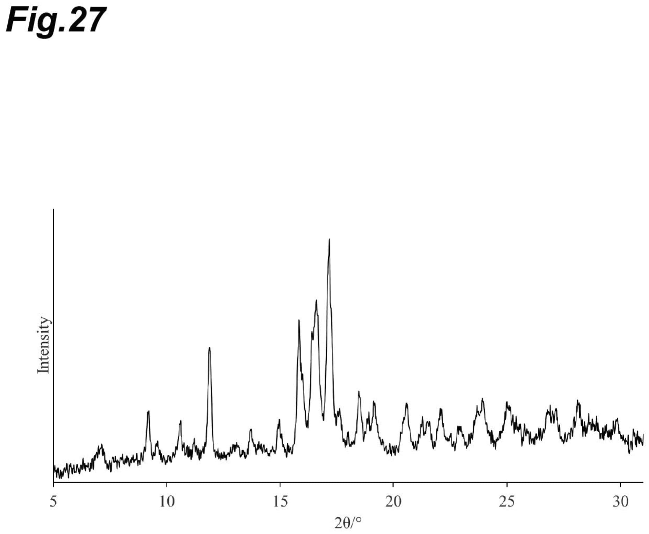

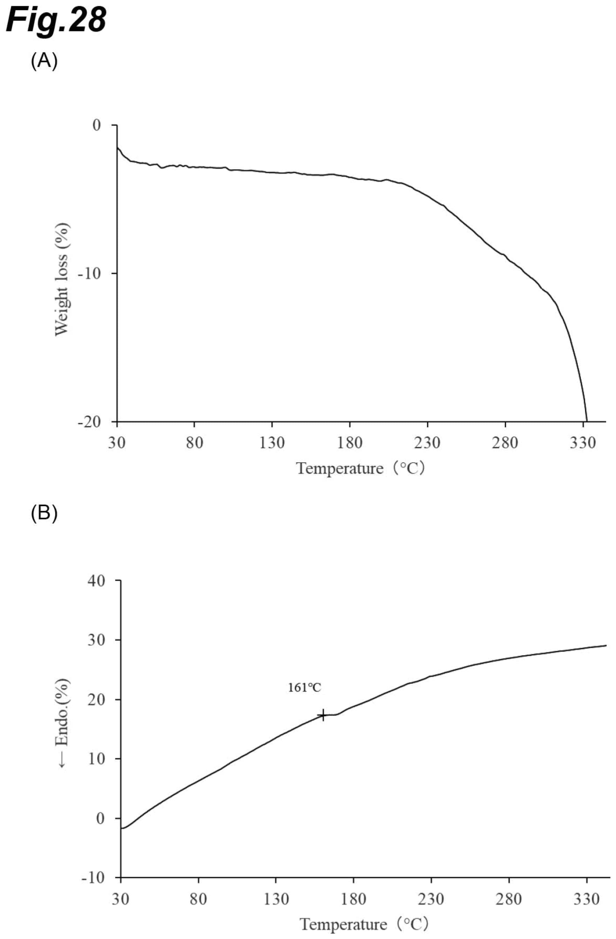

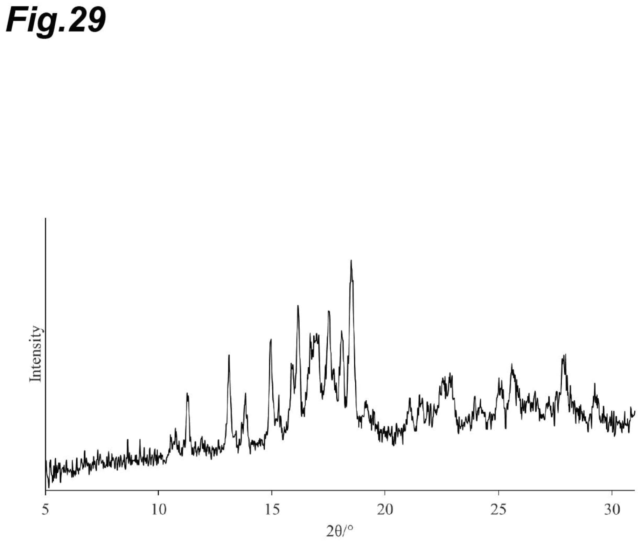

Compound 5 (approximately 25 mg) was heated at 110°C for 1 hour. The solution was cooled to room temperature and further preserved under a relative humidity condition of 100% for 2 hours to obtain hydrate 5E-type crystals. Results of powder X-ray diffractometry are shown in Figure 27. Results of TG-DTA measurement are shown in Figure 28. The obtained 5E-type crystals were further vacuum-dried to obtain non-solvate 5D-type crystals. Results of powder X-ray diffractometry are shown in Figure 29. Typical peaks at each run of powder X-ray diffractometry are shown in Table 18 below.

**[Table 18]**

| 2 *θ* (° ) | |
|---|---|
| 5E-type crystals | 5D-type crystals |
| 9.2 | 11.3 |
| 10.6 | 13.1 |
| 11.9 | 13.8 |
| 13.7 | 14.9 |
| 15.0 | 15.9 |
| 15.8 | 16.1 |
| 16.6 | 16.7 |
| 17.1 | 17.5 |
| 18.5 | 18.1 |
| 19.2 | 18.5 |

### (Example 2-5-4)

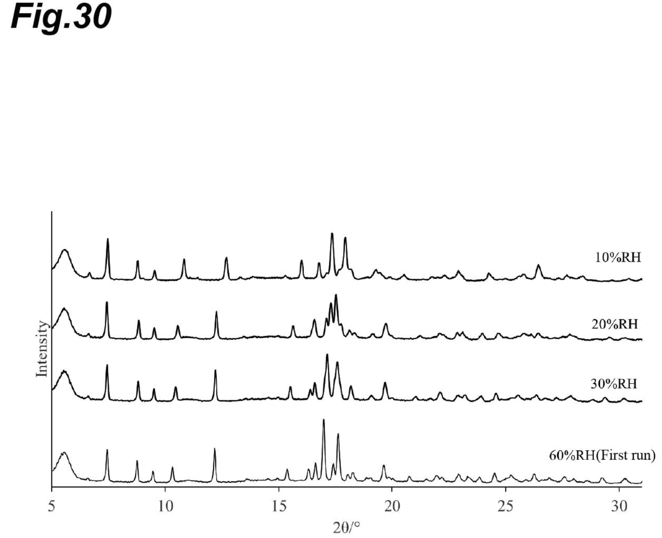

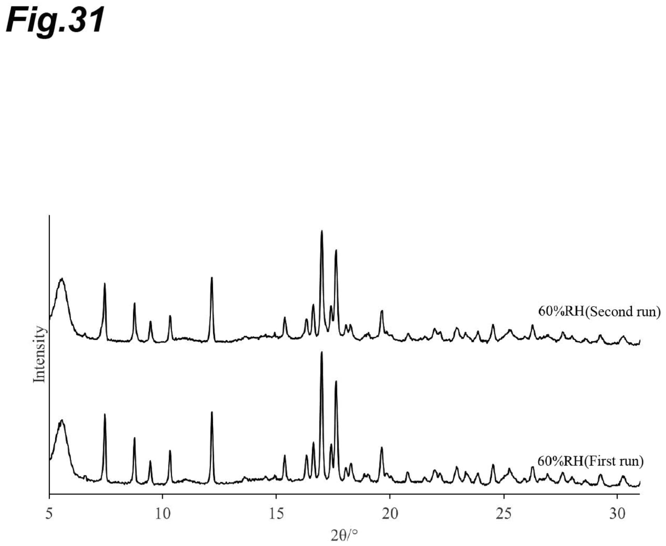

Hydrate 5A-type crystals were preserved at 25°C and a relative humidity of 95% for 1 hour. Then, the crystals were preserved at a relative humidity of 60% for 1 hour and subjected to powder X-ray diffractometry (first run). The crystals were further preserved at a relative humidity of 30% for 1 hour and subjected to powder X-ray diffractometry. The crystals were further preserved at a relative humidity of 20% for 1 hour and subjected to powder X-ray diffractometry. The crystals were further preserved at a relative humidity of 10% for 1 hour and subjected to powder X-ray diffractometry. These results of powder X-ray diffractometry are shown in Figure 30, and typical peaks at each humidity are shown in Table 19 below. Then, the crystals were preserved at a relative humidity of 5% for 1 hour, then further preserved at a relative humidity of 60% for 1 hour, and subjected to powder X-ray diffractometry (second run). As shown in Figure 31, the obtained pattern was consistent with the pattern of the initial measurement at a relative humidity of 60%. This demonstrated that 5A-type crystals had a crystal form whose peak position varied in response to humidities.

**[Table 19]**

| 2 *θ* (° ) | | | |
|---|---|---|---|
| Relative humidity10% | Relative humidity20% | Relative humidity30% | Relative humidity 60% (first run) |
| 7.4 | 7.4 | 7.4 | 7.4 |
| 8.8 | 8.8 | 8.8 | 8.8 |
| 9.5 | 9.5 | 9.5 | 9.5 |
| 10.8 | 10.5 | 10.5 | 10.3 |
| 12.7 | 12.2 | 12.2 | 12.2 |
| 16.0 | 15.6 | 15.5 | 15.4 |
| 16.8 | 16.6 | 16.4 | 16.3 |
| 17.3 | 17.1 | 16.6 | 16.6 |
| 17.9 | 17.3 | 17.1 | 17.0 |
| 19.2 | 17.5 | 17.6 | 17.6 |

### (Example 2-5-5)

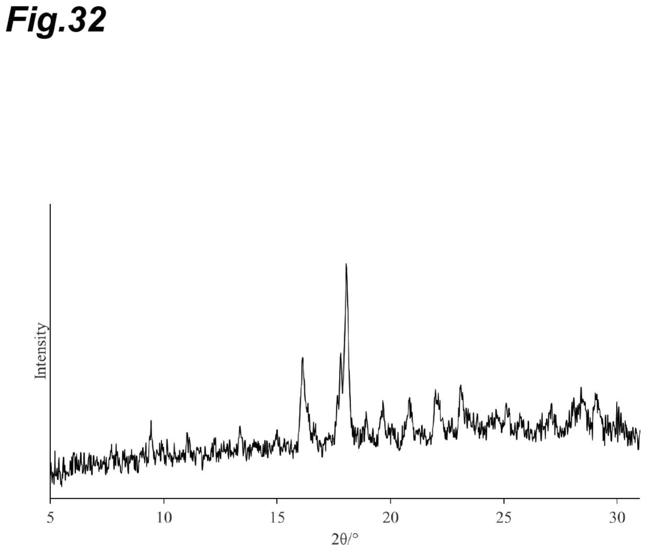

To 5A-type crystals (5.0 mg), acetone (25 µL) was added, then glass beads were added, and the mixture was shaken at 2000 rpm at room temperature for 7 days to obtain 5G-type crystals. Results of powder X-ray diffractometry are shown in Figure 32, and typical peaks are shown in Table 20 below.

**[Table 20]**

| 2 *θ* (° ) |
|---|
| 9.4 |
| 11.0 |
| 13.4 |
| 15.0 |
| 16.1 |
| 17.8 |
| 18.1 |
| 19.7 |
| 20.8 |
| 22.0 |

### (Example 2-5-6)

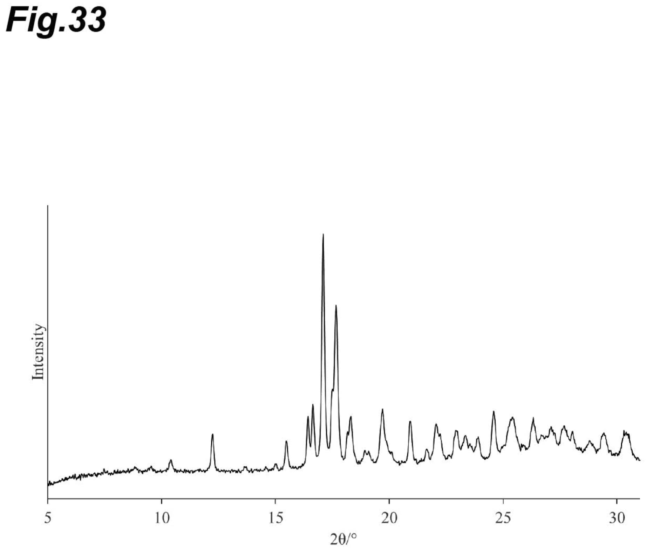

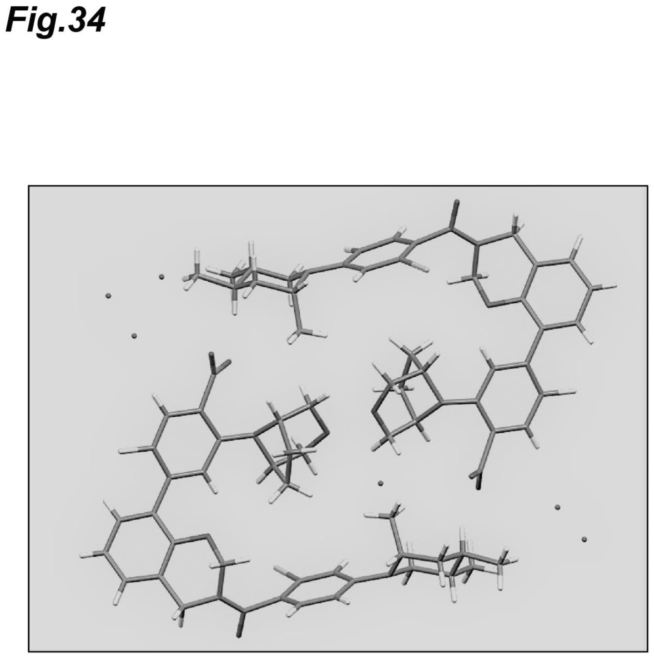

A mixed solution of methyl ethyl ketone/acetone = 2/1 (v/v) was prepared. This mixed solution and water were further mixed at a ratio of 65/35 (v/v). Approximately 3 mg each of this solution (30 µL), 5A-type crystals, and 5G-type crystals was mixed, then glass beads were placed therein, and the mixture was shaken at 2000 rpm for 1 month to obtain a solid. Results of powder X-ray diffractometry are shown in Figure 33. The obtained solid was confirmed to be 5A-type crystals by this measurement. Single-crystal X-ray structural analysis was carried out at 25°C under a relative humidity condition of 30%. The obtained crystal structure is shown in Figure 34. Parameters characterizing the crystals are shown in Table 21 below.

**[Table 21]**

| Crystal system | Triclinic |
|---|---|
| Space group | *P*1 |
| a [Å] | 10.6848 |
| b [Å] | 12.3881 |
| c [Å] | 14.2402 |
| α [° ] | 75.183 |
| β [° ] | 73.343 |
| γ [° ] | 79.590 |

### (Example 2-5-7)

To a solution of compound 5 (850 mg) in methyl ethyl ketone (1.4 v/w), acetone (1.3 v/w, 1.1 mL) was added, and methyl ethyl ketone (1.2 v/w, 1.0 mL) was added. To the mixture, water (1.6 mL) was added, then 5A-type crystals (8.5 mg) were added as seed crystals, and water (1.6 mL) was further added over 30 minutes, and the mixture was stirred for 1 hour. Water (3.2 mL) was added thereto, and the mixture was stirred for 21 hours. Water (3.2 mL) was added thereto over 1 hour, and the mixture was stirred for 3 hours. Further, water (3.2 mL) was added thereto over 1 hour, and the mixture was stirred for 17 hours and 30 minutes. Water (3.2 mL) was added thereto over 1 hour, and the mixture was stirred for 23 hours and 30 minutes. The suspension was filtered and washed with water (6.5 v/w, 5.5 mL) to obtain wet crystals (1.1 g). The wet crystals (297.9 mg) were dried under nitrogen stream for approximately 5 hours to obtain dry crystals (269.4 mg). A 206.2 mg aliquot of the dry crystals was further dried under nitrogen stream for 5 hours and 30 minutes to obtain dry crystals (203.5 mg). A 110.4 mg aliquot thereof was further dried under nitrogen stream for 28 hours to obtain 5A-type crystals (110.3 mg).

### (Example 2-5-8)

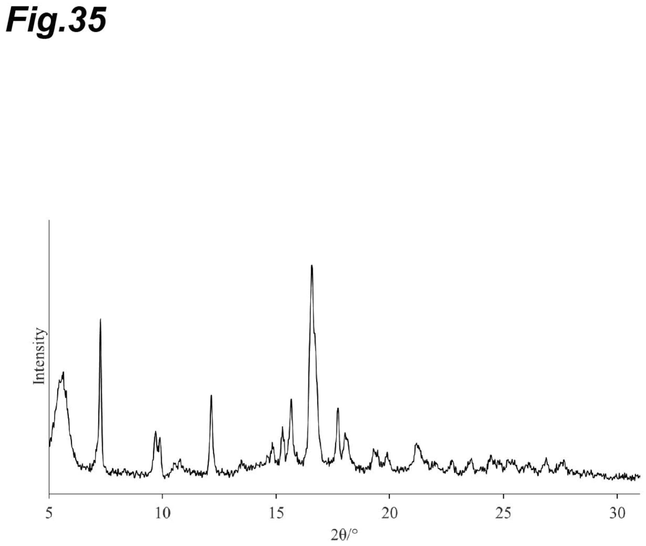

5A-type crystals prepared by the method described in Example 2-5-7 were preserved at 25°C and a relative humidity of 5% or 10% for 1 hour and subjected to powder X-ray diffractometry. From the obtained results, it was confirmed that the hydrate 5A-type crystals were transformed to 5C-type crystals by changing the relative humidity to around 10% or a lower relative humidity. Results of powder X-ray diffractometry are shown in Figure 35, and typical peaks are shown in Table 22 below.

**[Table 22]**

| 2 *θ* (° ) |
|---|
| 7.3 |
| 9.7 |
| 12.1 |
| 14.8 |
| 15.2 |
| 15.7 |
| 16.6 |
| 17.7 |

### (Example 2-5-9)

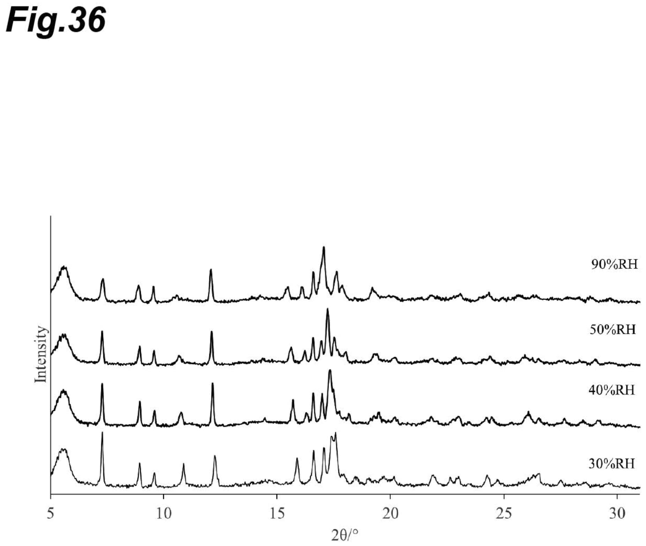

5C-type crystals prepared by the method described in Example 2-5-8 were preserved at 25°C and a relative humidity of around 30% or a higher relative humidity for 1 hour and subjected to powder X-ray diffractometry at each relative humidity. From the obtained results, it was confirmed that the 5C-type crystals were transformed to hydrate 5J-type crystals by changing the relative humidity to around 30% or a higher relative humidity. It was also confirmed that the 5J-type crystals slightly shifted peak positions of a powder X-ray diffraction patten depending on the relative humidity. Results of powder X-ray diffractometry are shown in Figure 36, and typical peaks are shown in Table 23 below.

**[Table 23]**

| 2 θ (° ) | | | |
|---|---|---|---|
| Relative humidity of around 30% | Relative humidity of around 40% | Relative humidity of around 50% to relative humidity of around 60% | Relative humidity of around 70% or higher relative humidity |
| 7.3 | 7.3 | 7.3 | 7.2 |
| 9.0 | 8.9 | 8.9 | 8.9 |
| 9.6 | 9.6 | 9.6 | 9.5 |
| 10.9 | 10.8 | 10.7 | 10.6 |
| 12.3 | 12.2 | 12.1 | 12.0 |
| 15.9 | 15.7 | 15.6 | 15.5 |
| - | 16.3 | 16.2 | 16.1 |
| 16.6 | 16.6 | 16.6 | 16.6 |
| 17.1 | 17.0 | 16.9 | - |
| 17.5 | 17.3 | 17.2 | 17.1 |

### [Evaluation of crystals]

The powder X-ray diffractometry of Example 2 was carried out under any of the following conditions.

### (Measurement method 1)

Measurement apparatus: SmartLab System, D/Tex Ultra detector (manufactured by Rigaku Corp.)
Radiation source: CuKα1
Tube voltage: 45 kV
Tube current: 200 mA
Scanning rate: 5°/min
Sampling width: 0.02°

### (Measurement method 2)

Measurement apparatus: D8 Discover, 2D VÅNTEC-500 solid state detector (manufactured by Bruker)
Radiation source: CuKα
Tube voltage/tube current: 40 kV/40 mA or 50 kV/1000 µA
Measurement range: 5 to 31°
Exposure time: 40 to 600 sec

The powder X-ray diffractometry under temperature and humidity adjustment was carried out under any of the following conditions.

### (Measurement method 1)

Measurement apparatus: X'pert-pro MPD (manufactured by PANalytical)
Radiation source: CuKα
Tube voltage: 45 kV
Tube current: 40 mA
Scanning rate: 0.33°/sec
Sampling width: 0.026°
The number of scans: 3 (measurement was repeated three times under the scanning condition described above, and the obtained results were integrated)

### (Measurement method 2)

Measurement apparatus: SmartLab System, D/Tex Ultra detector, water vapor generator HUM-SL (manufactured by Rigaku Corp.)
Radiation source: CuKα1
Tube voltage: 45 kV
Tube current: 200 mA
Scanning rate: 2.0°/min
Sampling width: 0.02°

The single-crystal X-ray structural analysis was carried out under the following conditions.
Measurement apparatus: XtaLAB Synergy Custom with a VariMax Cu diffractometer or Rigaku R-AXIS RAPID-II with a VariMax Cu diffractometer (manufactured by Rigaku Corp.) Radiation source: CuKα
Tube voltage/tube current: 40 kV/30 mA
Temperature: -180°C or room temperature

In the case of using any of the apparatuses, the measurement was performed using a strategy and an exposure time considered to produce diffraction spots sufficient for the structural analysis.

For the structural analysis, initial structural determination was performed by the direct method (SHELXT2014/5 or SHELXT2018/2), and structural refinement was performed by the full-matrix least-squares method (SHELXL2017/1).

The thermogravimetry-differential thermal analysis (TG-DTA) measurement of Example 2 was carried out under conditions given below. An onset temperature was determined from an exothermal peak and regarded as a melting point.
Apparatus: STA7200RV (manufactured by Hitachi High-Tech Science Corp.)
Measurement atmosphere: nitrogen
Warming condition: warmed from 30°C to 350°C at 10°C/min

### [Example 3] In vitro Nrf2 transcriptional activity measurement by ARE-dependent reporter assay in HepG2 cell

A human liver cancer-derived cell line HepG2 was transfected with pGL4.37 vector (pARE-Luc, Promega Corp.) and incubated overnight at 37°C in a humidified atmosphere containing 5% CO₂. The cells were dissociated by trypsin treatment, inoculated to a 384-well plate supplemented with a test compound, and incubated at 37°C for 4 hours. Then, Bright-Glo Reagent (Promega Corp.) was added to the plate, and luciferase activity was measured with Envision plate reader.

Luciferase activity obtained through the action of compound 2 or compound 7 at a concentration of 3.75 µM was defined as 100%. The luciferase activity of each well was converted to a response rate (%). The dose-response curve of the compound of each Example was prepared to calculate a 50% effective concentration (EC₅₀). The results are shown in Table 24. These results demonstrated that the compound group of the present invention has a Nrf2-activating effect *in vitro.*

**[Table 24]**

| Compound No. | EC50 (nM) |
|---|---|
| Compound 1 | 1.9 |
| Compound 3 | 1.7 |
| Compound 4 | 0.95 |
| Compound 5 | 2.3 |
| Compound 6 | 1.4 |

### Industrial Applicability

The present invention provides crystals of a low-molecular compound or a salt thereof, or a solvate thereof which has a Nrf2-activating effect. The present invention also provides a medicament for the prevention and/or treatment of various diseases, for example, neurodegenerative disease, lung disease, and kidney disease.

## Claims

1. A crystal of a compound represented by any of the following formulas (1) to (5) or a salt thereof, or a solvate thereof:

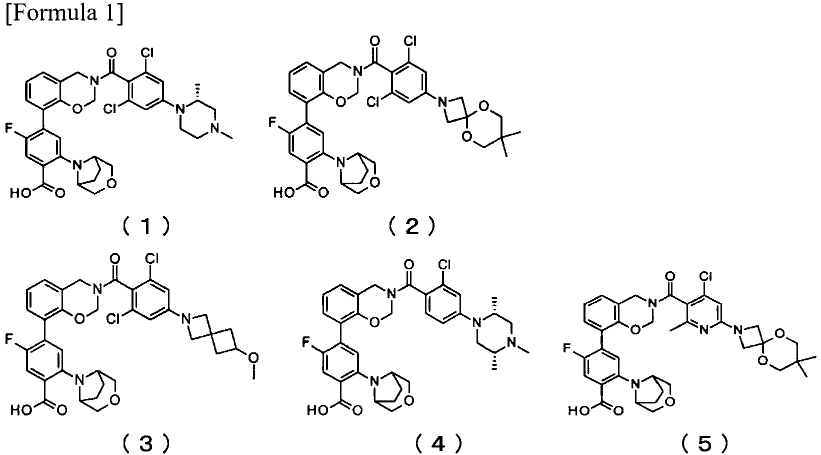

2. The crystal according to claim 1, wherein the crystal is a crystal of the compound represented by the formula (1) or a salt thereof, or a solvate thereof.

3. The crystal according to claim 1, wherein the crystal is a crystal of the compound represented by the formula (2) or a salt thereof, or a solvate thereof.

4. The crystal according to claim 1, wherein the crystal is a crystal of the compound represented by the formula (3) or a salt thereof, or a solvate thereof.

5. The crystal according to claim 1, wherein the crystal is a crystal of the compound represented by the formula (4) or a salt thereof, or a solvate thereof.

6. The crystal according to claim 1, wherein the crystal is a crystal of the compound represented by the formula (5) or a salt thereof, or a solvate thereof.

7. The crystal according to any one of claims 1 to 6, wherein the crystal is a solvate crystal of the compound.

8. The crystal according to claim 7, wherein the solvate crystal is a hydrate crystal.

9. The crystal according to any one of claims 1 to 6, wherein the crystal is a non-solvate crystal of the compound.

10. A pharmaceutical composition comprising a crystal of a compound or a salt thereof, or a solvate thereof according to any one of claims 1 to 9.

11. The pharmaceutical composition according to claim 10 for the prevention and/or treatment of neurodegenerative disease, lung disease, or kidney disease.

12. A method for preventing and/or treating neurodegenerative disease, lung disease, or kidney disease, comprising administering an effective amount of a crystal of a compound or a salt thereof, or a solvate thereof according to any one of claims 1 to 9 to a subject.

13. The crystal of a compound or a salt thereof, or a solvate thereof according to any one of claims 1 to 9 for use in the prevention and/or treatment of neurodegenerative disease, lung disease, or kidney disease.

14. Use of a crystal of a compound or a salt thereof, or a solvate thereof according to any one of claims 1 to 9 for producing a pharmaceutical composition for the prevention and/or treatment of neurodegenerative disease, lung disease, or kidney disease.

15. A method for producing a pharmaceutical composition containing a compound represented by any of the following formulas (1) to (5) or a salt thereof, or a solvate thereof as an active ingredient, comprising the step of
mixing a crystal of a compound or a salt thereof, or a solvate thereof according to any one of claims 1 to 9 with a pharmacologically acceptable carrier or medium:
